# EUROPEAN PATENT APPLICATION

(11) **EP 0 866 131 A2**
(43) Date of publication of application: **23.09.1998**
(21) Application number: 98302113.0
(22) Date of filing: 20.03.1998
(51) Int. Cl.: C12N 15/62, C07K 16/00, C07K 16/28, C12N 15/70, C12N 1/21, A61K 39/395

(54) **Humanized anti-human fas antibody**

(30) Priority: 21.03.1997 JP 67938/97
(71) Applicant: Sankyo Company Limited, Chuo-ku, Tokyo 103-8426 (JP)
(72) Inventor: Serizawa, Nobufusa, c/o Sankyo Company Limited, Shinagawa-ku, Tokyo (JP); Haruyama, Hideyuki, c/o Sankyo Company Limited, Shinagawa-ku, Tokyo (JP); Takahashi, Tohru, c/o Sankyo Company Limited, Shinagawa-ku, Tokyo (JP); Nakahara, Kaori, c/o Sankyo Company Limited, Shinagawa-ku, Tokyo (JP); Yonehara, Shin, Kyoto-shi, Kyoto-fu (JP)
(74) Representative: Gibson, Christian John Robert

(57) **Abstract**

The invention provides humanised anti-human Fas antibodies capable of inducing apoptosis in cells expressing Fas and which are useful in the treatment of autoimmune disease and chronic rheumatoid arthritis. In addition, the invention provides DNA encoding the variable regions of the H and L chain of such antibodies and methods for humanising antibodies.

### Annex to the description

SEQUENCE LISTING

(1) GENERAL INFORMATION:

(i) APPLICANT:
(A) NAME: Sankyo Company, Limited
(B) STREET: 5-1, Nihonbashi Honcho 3-chome, Chuo-ku
(C) CITY: Tokyo
(E) COUNTRY: Japan
(F) POSTAL CODE (ZIP): 103-8426
(G) TELEPHONE: 81-3-5255-7111

(ii) TITLE OF INVENTION: Humanized Anti-Human Fas Antibody

(iii) NUMBER OF SEQUENCES: 189

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: JP Hei 9-67938
(B) FILING DATE: 21-MAR-1997

SEQUENCE LISTING

(2) INFORMATION FOR SEQ ID NO: 1:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 1:

Asp Tyr Asn Met His
1               5

(2) INFORMATION FOR SEQ ID NO: 2:

(i) SEQUENCE CHARACTERISTICS:
(A) LENGTH: 17 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 2:

Tyr Ile Tyr Pro Tyr Asn Gly Gly Thr Gly Tyr Asn Gln Lys Phe Lys
1               5                   10                  15

Ser

(2) INFORMATION FOR SEQ ID NO: 3:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 3:

Ser Tyr Tyr Ala Met Asp Tyr
1               5

(2) INFORMATION FOR SEQ ID NO: 4:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 4:
Arg Ser Ser Lys Ser Leu Val His Ser Asn Gly Asn Thr Tyr Leu His
1               5                   10                  15

(2) INFORMATION FOR SEQ ID NO: 5:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 5:

Lys Val Ser Asn Arg Phe Ser
1               5

(2) INFORMATION FOR SEQ ID NO: 6:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: protein

(v) FRAGMENT TYPE: internal

(xi) SEQUENCE DESCRIPTION: SEQ ID NO: 6:

Ser Gln Ser Thr His Val Pro Pro Ala
1               5

(2) INFORMATION FOR SEQ ID NO: 7:

(i) SEQUENCE CHARACTERISTICS:
(A) LENGTH: 1773 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Mus musculus
(G) CELL TYPE: Hybridoma
(H) CELL LINE: CH11

(ix) FEATURE:
(A) NAME/KEY: CDS
(B) LOCATION:1..1770

(ix) FEATURE:
(A) NAME/KEY: mat_peptide
(B) LOCATION:58..1770

(ix) FEATURE:
(A) NAME/KEY: sig_peptide
(B) LOCATION:1..57

(xi) SEQUENCE DESCRIPTION: SEQ ID NO: 7:

ATG GGA TGG AGC TGG ATC TTT CTC TTC CTC CTG TCA GGA ACT GCA GGC      48
Met Gly Trp Ser Trp Ile Phe Leu Phe Leu Leu Ser Gly Thr Ala Gly
-19             -15                 -10                 -5

GTC CAC TCT GAG GTC CAG CTT CAG CAG TCA GGA CCT GAG CTG GTG AAA      96
Val His Ser Glu Val Gln Leu Gln Gln Ser Gly Pro Glu Leu Val Lys
            1               5                   10

CCT GGG GCC TCA GTG AAG ATA TCC TGC AAG GCT TCT GGA TAC ACA TTC      144
Pro Gly Ala Ser Val Lys Ile Ser Cys Lys Ala Ser Gly Tyr Thr Phe
    15                  20                  25

ACT GAC TAC AAC ATG CAC TGG GTG AAG CAG AGC CAT GGA AAG AGC CTT      192
Thr Asp Tyr Asn Met His Trp Val Lys Gln Ser His Gly Lys Ser Leu
30                  35                  40                  45

GAG TGG ATT GGA TAT ATT TAT CCT TAC AAT GGT GGT ACT GGC TAC AAC 240
Glu Trp Ile Gly Tyr Ile Tyr Pro Tyr Asn Gly Gly Thr Gly Tyr Asn 912
    50                  55                  60

CAG AAG TTC AAG AGC AAG GCC ACA TTG ACT GTT GAC AAT TCC TCC AGC 288
Gln Lys Phe Lys Ser Lys Ala Thr Leu Thr Val Asp Asn Ser Ser Ser 960
65 70 75

ACA GCC TAC ATG GAG CTC CGC AGC CTG ACA TCT GAG GAC TCT GCA GTC 336
Thr Ala Tyr Met Glu Leu Arg Ser Leu Thr Ser Glu Asp Ser Ala Val 1008
80 85 90

TAT TAC TGT GCA AGA AGT TAC TAT GCT ATG GAC TAC TGG GGT CAA GGA 384
Tyr Tyr Cys Ala Arg Ser Tyr Tyr Ala Met Asp Tyr Trp Gly Gln Gly 1056
95 100 105

ACC TCA GTC ACC GTC TCC TCA GAG AGT CAG TCC TTC CCA AAT GTC TTC 432
Thr Ser Val Thr Val Ser Ser Glu Ser Gln Ser Phe Pro Asn Val Phe 1104
110 115 120 125

CCC CTC GTC TCC TGC GAG AGC CCC CTG TCT GAT AAG AAT CTG GTG GCC 480
Pro Leu Val Ser Cys Glu Ser Pro Leu Ser Asp Lys Asn Leu Val Ala 1152
    130                 135                 140

ATG GGC TGC CTA GCC CGG GAC TTC CTG CCC AGC ACC ATT TCC TTC ACC 528
Met Gly Cys Leu Ala Arg Asp Phe Leu Pro Ser Thr Ile Ser Phe Thr 1200
145 150 155

TGG AAC TAC CAG AAC AAC ACT GAA GTC ATC CAG GGT ATC AGA ACC TTC 576
Trp Asn Tyr Gln Asn Asn Thr Glu Val Ile Gln Gly Ile Arg Thr Phe 1248
160 165 170

CCA ACA CTG AGG ACA GGG GGC AAG TAC CTA GCC ACC TCG CAG GTG TTG 624
Pro Thr Leu Arg Thr Gly Gly Lys Tyr Leu Ala Thr Ser Gln Val Leu 1296
175 180 185

CTG TCT CCC AAG AGC ATC CTT GAA GGT TCA GAT GAA TAC CTG GTA TGC 672
Leu Ser Pro Lys Ser Ile Leu Glu Gly Ser Asp Glu Tyr Leu Val Cys 1344
190 195 200 205

AAA ATC CAC TAC GGA GGC AAA AAC AGA GAT CTG CAT GTG CCC ATT CCA 720
Lys Ile His Tyr Gly Gly Lys Asn Arg Asp Leu His Val Pro Ile Pro 1392
    210                 215                 220

GCT GTC GCA GAG ATG AAC CCC AAT GTA AAT GTG TTC GTC CCA CCA CGG 768
Ala Val Ala Glu Met Asn Pro Asn Val Asn Val Phe Val Pro Pro Arg 1440
225 230 235

GAT GGC TTC TCT GGC CCT GCA CCA CGC AAG TCT AAA CTC ATC TGC GAG 816
Asp Gly Phe Ser Gly Pro Ala Pro Arg Lys Ser Lys Leu Ile Cys Glu 1488
240 245 250

GCC ACG AAC TTC ACT CCA AAA CCG ATC ACA GTA TCC TGG CTA AAG GAT 864
Ala Thr Asn Phe Thr Pro Lys Pro Ile Thr Val Ser Trp Leu Lys Asp 1536
255 260 265

GGG AAG CTC GTG GAA TCT GGC TTC ACC ACA GAT CCG GTG ACC ATC GAG 912
Gly Lys Leu Val Glu Ser Gly Phe Thr Thr Asp Pro Val Thr Ile Glu
270 275 280 285

AAC AAA GGA TCC ACA CCC CAA ACC TAC AAG GTC ATA AGC ACA CTT ACC 960
Asn Lys Gly Ser Thr Pro Gln Thr Tyr Lys Val Ile Ser Thr Leu Thr
    290                 295                 300

ATC TCT GAA ATC GAC TGG CTG AAC CTG AAT GTG TAC ACC TGC CGT GTG 1008
Ile Ser Glu Ile Asp Trp Leu Asn Leu Asn Val Tyr Thr Cys Arg Val
305 310 315

GAT CAC AGG GGT CTC ACC TTC TTG AAG AAC GTG TCC TCC ACA TGT GCT 1056
Asp His Arg Gly Leu Thr Phe Leu Lys Asn Val Ser Ser Thr Cys Ala
320 325 330

GCC AGT CCC TCC ACA GAC ATC CTA ACC TTC ACC ATC CCC CCC TCC TTT 1104
Ala Ser Pro Ser Thr Asp Ile Leu Thr Phe Thr Ile Pro Pro Ser Phe
335 340 345

GCC GAC ATC TTC CTC AGC AAG TCC GCT AAC CTG ACC TGT CTG GTC TCA 1152
Ala Asp Ile Phe Leu Ser Lys Ser Ala Asn Leu Thr Cys Leu Val Ser
350 355 360 365

AAC CTG GCA ACC TAT GAA ACC CTG AAT ATC TCC TGG GCT TCT CAA AGT 1200
Asn Leu Ala Thr Tyr Glu Thr Leu Asn Ile Ser Trp Ala Ser Gln Ser
    370                 375                 380

GGT GAA CCA CTG GAA ACC AAA ATT AAA ATC ATG GAA AGC CAT CCC AAT 1248
Gly Glu Pro Leu Glu Thr Lys Ile Lys Ile Met Glu Ser His Pro Asn
385 390 395

GGC ACC TTC AGT GCT AAG GGT GTG GCT AGT GTT TGT GTG GAA GAC TGG 1296
Gly Thr Phe Ser Ala Lys Gly Val Ala Ser Val Cys Val Glu Asp Trp
400 405 410

AAT AAC AGG AAG GAA TTT GTG TGT ACT GTG ACT CAC AGG GAT CTG CCT 1344
Asn Asn Arg Lys Glu Phe Val Cys Thr Val Thr His Arg Asp Leu Pro
415 420 425

TCA CCA CAG AAG AAA TTC ATC TCA AAA CCC AAT GAG GTG CAC AAA CAT 1392
Ser Pro Gln Lys Lys Phe Ile Ser Lys Pro Asn Glu Val His Lys His
430 435 440 445

CCA CCT GCT GTG TAC CTG CTG CCA CCA GCT CGT GAG CAA CTG AAC CTG 1440
Pro Pro Ala Val Tyr Leu Leu Pro Pro Ala Arg Glu Gln Leu Asn Leu
    450                 455                 460

AGG GAG TCA GCC ACA GTC ACC TGC TTG GTG AAG GGC TTC TCT CCT GCA 1488
Arg Glu Ser Ala Thr Val Thr Cys Leu Val Lys Gly Phe Ser Pro Ala
465 470 475

GAC ATC AGT GTG CAG TGG CTT CAG AGA GGG CAA CTC TTG CCC CAA GAG 1536
Asp Ile Ser Val Gln Trp Leu Gln Arg Gly Gln Leu Leu Pro Gln Glu
480 485 490

AAG TAT GTG ACC AGT GCC CCG ATG CCA GAG CCT GGG GCC CCA GGC TTC 1584
Lys Tyr Val Thr Ser Ala Pro Met Pro Glu Pro Gly Ala Pro Gly Phe
495 500 505

TAC TTT ACC CAC AGC ATC CTG ACT GTG ACA GAG GAG GAA TGG AAC TCC 1632
Tyr Phe Thr His Ser Ile Leu Thr Val Thr Glu Glu Glu Trp Asn Ser
510 515 520 525

GGA GAG ACC TAT ACC TGT GTT GTA GGC CAC GAG GCC CTG CCA CAC CTG 1680
Gly Glu Thr Tyr Thr Cys Val Val Gly His Glu Ala Leu Pro His Leu
    530                 535                 540

GTG ACC GAG AGG ACC GTG GAC AAG TCC ACT GGT AAA CCC ACA CTG TAC 1728
Val Thr Glu Arg Thr Val Asp Lys Ser Thr Gly Lys Pro Thr Leu Tyr
545 550 555

AAT GTC TCC CTG ATC ATG TCT GAC ACA GGC GGC ACC TGC TAT 1770
Asn Val Ser Leu Ile Met Ser Asp Thr Gly Gly Thr Cys Tyr
560 565 570

TGA 1773

(2) INFORMATION FOR SEQ ID NO: 8:

(i) SEQUENCE CHARACTERISTICS:
(A) LENGTH: 590 amino acids
(B) TYPE: amino acid
(D) TOPOLOGY: linear

(ii) MOLECULE TYPE: protein
(xi) SEQUENCE DESCRIPTION: SEQ ID NO: 8:

Met Gly Trp Ser Trp Ile Phe Leu Phe Leu Leu Ser Gly Thr Ala Gly
-19 -15 -10 -5

Val His Ser Glu Val Gln Leu Gln Gln Ser Gly Pro Glu Leu Val Lys
1 5 10

Pro Gly Ala Ser Val Lys Ile Ser Cys Lys Ala Ser Gly Tyr Thr Phe
15 20 25

Thr Asp Tyr Asn Met His Trp Val Lys Gln Ser His Gly Lys Ser Leu
30 35 40 45

Glu Trp Ile Gly Tyr Ile Tyr Pro Tyr Asn Gly Gly Thr Gly Tyr Asn
    50                  55                  60

Gln Lys Phe Lys Ser Lys Ala Thr Leu Thr Val Asp Asn Ser Ser Ser
65 70 75

Thr Ala Tyr Met Glu Leu Arg Ser Leu Thr Ser Glu Asp Ser Ala Val
80 85 90

Tyr Tyr Cys Ala Arg Ser Tyr Tyr Ala Met Asp Tyr Trp Gly Gln Gly
95 100 105

Thr Ser Val Thr Val Ser Ser Glu Ser Gln Ser Phe Pro Asn Val Phe
110                 115                 120                 125

Pro Leu Val Ser Cys Glu Ser Pro Leu Ser Asp Lys Asn Leu Val Ala
                130                 135                 140

Met Gly Cys Leu Ala Arg Asp Phe Leu Pro Ser Thr Ile Ser Phe Thr
            145                 150                 155

Trp Asn Tyr Gln Asn Asn Thr Glu Val Ile Gln Gly Ile Arg Thr Phe
        160                 165                 170

Pro Thr Leu Arg Thr Gly Gly Lys Tyr Leu Ala Thr Ser Gln Val Leu
    175                 180                 185

Leu Ser Pro Lys Ser Ile Leu Glu Gly Ser Asp Glu Tyr Leu Val Cys
190                 195                 200                 205

Lys Ile His Tyr Gly Gly Lys Asn Arg Asp Leu His Val Pro Ile Pro
                210                 215                 220

Ala Val Ala Glu Met Asn Pro Asn Val Asn Val Phe Val Pro Pro Arg
            225                 230                 235

Asp Gly Phe Ser Gly Pro Ala Pro Arg Lys Ser Lys Leu Ile Cys Glu
        240                 245                 250

Ala Thr Asn Phe Thr Pro Lys Pro Ile Thr Val Ser Trp Leu Lys Asp
    255                 260                 265

Gly Lys Leu Val Glu Ser Gly Phe Thr Thr Asp Pro Val Thr Ile Glu
270                 275                 280                 285

Asn Lys Gly Ser Thr Pro Gln Thr Tyr Lys Val Ile Ser Thr Leu Thr
                290                 295                 300

Ile Ser Glu Ile Asp Trp Leu Asn Leu Asn Val Tyr Thr Cys Arg Val
            305                 310                 315

Asp His Arg Gly Leu Thr Phe Leu Lys Asn Val Ser Ser Thr Cys Ala
        320                 325                 330

Ala Ser Pro Ser Thr Asp Ile Leu Thr Phe Thr Ile Pro Pro Ser Phe
    335                 340                 345

Ala Asp Ile Phe Leu Ser Lys Ser Ala Asn Leu Thr Cys Leu Val Ser
350                 355                 360                 365

Asn Leu Ala Thr Tyr Glu Thr Leu Asn Ile Ser Trp Ala Ser Gln Ser
                370                 375                 380

Gly Glu Pro Leu Glu Thr Lys Ile Lys Ile Met Glu Ser His Pro Asn
            385                 390                 395

Gly Thr Phe Ser Ala Lys Gly Val Ala Ser Val Cys Val Glu Asp Trp
        400                 405                 410

Asn Asn Arg Lys Glu Phe Val Cys Thr Val Thr His Arg Asp Leu Pro
    415                 420                 425

Ser Pro Gln Lys Lys Phe Ile Ser Lys Pro Asn Glu Val His Lys His
430                 435                 440                 445

Pro Pro Ala Val Tyr Leu Leu Pro Pro Ala Arg Glu Gln Leu Asn Leu
                450                 455                 460

Arg Glu Ser Ala Thr Val Thr Cys Leu Val Lys Gly Phe Ser Pro Ala
            465                 470                 475

Asp Ile Ser Val Gln Trp Leu Gln Arg Gly Gln Leu Leu Pro Gln Glu
        480                 485                 490

Lys Tyr Val Thr Ser Ala Pro Met Pro Glu Pro Gly Ala Pro Gly Phe
    495                 500                 505

Tyr Phe Thr His Ser Ile Leu Thr Val Thr Glu Glu Glu Trp Asn Ser
510                 515                 520                 525

Gly Glu Thr Tyr Thr Cys Val Val Gly His Glu Ala Leu Pro His Leu
                530                 535                 540

Val Thr Glu Arg Thr Val Asp Lys Ser Thr Gly Lys Pro Thr Leu Tyr
            545                 550                 555

Asn Val Ser Leu Ile Met Ser Asp Thr Gly Gly Thr Cys Tyr
        560                 565                 570

(2) INFORMATION FOR SEQ ID NO: 9:

     (i) SEQUENCE CHARACTERISTICS:
          (A) LENGTH: 717 base pairs
          (B) TYPE: nucleic acid
          (C) STRANDEDNESS: double
          (D) TOPOLOGY: linear

    (ii) MOLECULE TYPE: cDNA to mRNA

   (iii) HYPOTHETICAL: NO

    (iv) ANTI-SENSE: NO

    (vi) ORIGINAL SOURCE:
          (A) ORGANISM: Mus musculus
          (G) CELL TYPE: Hybridoma
          (H) CELL LINE: CH11

    (ix) FEATURE:
          (A) NAME/KEY: CDS
          (B) LOCATION:1..714

    (ix) FEATURE:
          (A) NAME/KEY: mat_peptide
          (B) LOCATION:58..714

    (ix) FEATURE:

(A) NAME/KEY: sig_peptide
(B) LOCATION:1..57

(xi) SEQUENCE DESCRIPTION: SEQ ID NO: 9:

ATG AAG TTG CCT GTT AGG CTG TTG GTG CTG ATG TTC TGG ATT CCT GCT 48
Met Lys Leu Pro Val Arg Leu Leu Val Leu Met Phe Trp Ile Pro Ala
-19 -15 -10 -5

TCC AGC AGT GAT GTT GTG ATG ACC CAA AGT CCA CTC TCC CTG CCT GTC 96
Ser Ser Ser Asp Val Val Met Thr Gln Ser Pro Leu Ser Leu Pro Val
1 5 10

AGT CTT GGA GAT CAA GCC TCC ATC TCT TGC AGA TCT AGT AAG AGC CTT 144
Ser Leu Gly Asp Gln Ala Ser Ile Ser Cys Arg Ser Ser Lys Ser Leu
15 20 25

GTA CAC AGT AAT GGA AAC ACC TAT TTA CAT TGG TAC CTG CAG AAG CCA 192
Val His Ser Asn Gly Asn Thr Tyr Leu His Trp Tyr Leu Gln Lys Pro
30 35 40 45

GGC CAG TCT CCA AAG CTC CTG ATC TAC AAA GTT TCC AAC CGA TTT TCT 240
Gly Gln Ser Pro Lys Leu Leu Ile Tyr Lys Val Ser Asn Arg Phe Ser
    50                  55                  60

GGG GTC CCA GAC AGG TTC AGT GGC AGT GGA TCA GGG ACA GAT TTC ACA 288
Gly Val Pro Asp Arg Phe Ser Gly Ser Gly Ser Gly Thr Asp Phe Thr
65 70 75

CTC AAG ATC AGC AGA GTG GAG GCT GAG GAT CTG GGA GTT TAT TTC TGC 336
Leu Lys Ile Ser Arg Val Glu Ala Glu Asp Leu Gly Val Tyr Phe Cys
80 85 90

TCT CAA AGT ACA CAT GTT CCT CCG GCG TTC GGT GGA GGC ACC AAG CTG 384
Ser Gln Ser Thr His Val Pro Pro Ala Phe Gly Gly Gly Thr Lys Leu
95 100 105

GAA ATC AAA CGG GCT GAT GCT GCA CCA ACT GTA TCC ATC TTC CCA CCA 432
Glu Ile Lys Arg Ala Asp Ala Ala Pro Thr Val Ser Ile Phe Pro Pro
110 115 120 125

TCC AGT GAG CAG TTA ACA TCT GGA GGT GCC TCA GTC GTG TGC TTC TTG 480
Ser Ser Glu Gln Leu Thr Ser Gly Gly Ala Ser Val Val Cys Phe Leu
    130                 135                 140

AAC AAC TTC TAC CCC AAA GAC ATC AAT GTC AAG TGG AAG ATT GAT GGC 528
Asn Asn Phe Tyr Pro Lys Asp Ile Asn Val Lys Trp Lys Ile Asp Gly
145 150 155

AGT GAA CGA CAA AAT GGC GTC CTG AAC AGT TGG ACT GAT CAG GAC AGC 576
Ser Glu Arg Gln Asn Gly Val Leu Asn Ser Trp Thr Asp Gln Asp Ser
160 165 170

AAA GAC AGC ACC TAC AGC ATG AGC AGC ACC CTC ACG TTG ACC AAG GAC 624
Lys Asp Ser Thr Tyr Ser Met Ser Ser Thr Leu Thr Leu Thr Lys Asp
175 180 185

GAG TAT GAA CGA CAT AAC AGC TAT ACC TGT GAG GCC ACT CAC AAG ACA 672
Glu Tyr Glu Arg His Asn Ser Tyr Thr Cys Glu Ala Thr His Lys Thr
190 195 200 205

TCA ACT TCA CCC ATT GTC AAG AGC TTC AAC AGG AAT GAG TGT 714
Ser Thr Ser Pro Ile Val Lys Ser Phe Asn Arg Asn Glu Cys
210 215

TAG 717

(2) INFORMATION FOR SEQ ID NO: 10:

(i) SEQUENCE CHARACTERISTICS:
(A) LENGTH: 238 amino acids
(B) TYPE: amino acid
(D) TOPOLOGY: linear

(ii) MOLECULE TYPE: protein
(xi) SEQUENCE DESCRIPTION: SEQ ID NO: 10:

Met Lys Leu Pro Val Arg Leu Leu Val Leu Met Phe Trp Ile Pro Ala
-19 -15 -10 -5

Ser Ser Ser Asp Val Val Met Thr Gln Ser Pro Leu Ser Leu Pro Val
1 5 10

Ser Leu Gly Asp Gln Ala Ser Ile Ser Cys Arg Ser Ser Lys Ser Leu
15 20 25

Val His Ser Asn Gly Asn Thr Tyr Leu His Trp Tyr Leu Gln Lys Pro
30 35 40 45

Gly Gln Ser Pro Lys Leu Leu Ile Tyr Lys Val Ser Asn Arg Phe Ser
    50                  55                  60

Gly Val Pro Asp Arg Phe Ser Gly Ser Gly Ser Gly Thr Asp Phe Thr
65 70 75

Leu Lys Ile Ser Arg Val Glu Ala Glu Asp Leu Gly Val Tyr Phe Cys
80 85 90

Ser Gln Ser Thr His Val Pro Pro Ala Phe Gly Gly Gly Thr Lys Leu
95 100 105

Glu Ile Lys Arg Ala Asp Ala Ala Pro Thr Val Ser Ile Phe Pro Pro
110 115 120 125

Ser Ser Glu Gln Leu Thr Ser Gly Gly Ala Ser Val Val Cys Phe Leu
    130                 135                 140

Asn Asn Phe Tyr Pro Lys Asp Ile Asn Val Lys Trp Lys Ile Asp Gly
            145                 150                 155

Ser Glu Arg Gln Asn Gly Val Leu Asn Ser Trp Thr Asp Gln Asp Ser
        160                 165                 170

Lys Asp Ser Thr Tyr Ser Met Ser Ser Thr Leu Thr Leu Thr Lys Asp
    175                 180                 185

Glu Tyr Glu Arg His Asn Ser Tyr Thr Cys Glu Ala Thr His Lys Thr
190                 195                 200                 205

Ser Thr Ser Pro Ile Val Lys Ser Phe Asn Arg Asn Glu Cys
                210                 215

(2) INFORMATION FOR SEQ ID NO: 11:

     (i) SEQUENCE CHARACTERISTICS:
          (A) LENGTH: 480 base pairs
          (B) TYPE: nucleic acid
          (C) STRANDEDNESS: double
          (D) TOPOLOGY: linear

    (ii) MOLECULE TYPE: cDNA to mRNA

   (iii) HYPOTHETICAL: NO

    (iv) ANTI-SENSE: NO

    (vi) ORIGINAL SOURCE:
          (A) ORGANISM: Mus musculus
          (G) CELL TYPE: Hybridoma
          (H) CELL LINE: CH11

    (ix) FEATURE:
          (A) NAME/KEY: CDS
          (B) LOCATION:1..477

    (ix) FEATURE:
          (A) NAME/KEY: mat_peptide
          (B) LOCATION:67..477

    (ix) FEATURE:
          (A) NAME/KEY: sig_peptide
          (B) LOCATION:1..66


    (xi) SEQUENCE DESCRIPTION: SEQ ID NO: 11:

ATG AAG ACC CAC CTG CTT CTC TGG GGA GTC CTC GCC ATT TTT GTT AAG          48
Met Lys Thr His Leu Leu Leu Trp Gly Val Leu Ala Ile Phe Val Lys
-22     -20                 -15                 -10

GCT GTC CTT GTA ACA GGT GAC GAC GAA GCG ACC ATT CTT GCT GAC AAC          96
Ala Val Leu Val Thr Gly Asp Asp Glu Ala Thr Ile Leu Ala Asp Asn
     -5                   1               5                  10

AAA TGC ATG TGT ACC CGA GTT ACC TCT AGG ATC ATC CCT TCC ACC GAG 144
Lys Cys Met Cys Thr Arg Val Thr Ser Arg Ile Ile Pro Ser Thr Glu
15 20 25

GAT CCT AAT GAG GAC ATT GTG GAG AGA AAT ATC CGA ATT GTT GTC CCT 192
Asp Pro Asn Glu Asp Ile Val Glu Arg Asn Ile Arg Ile Val Val Pro
30 35 40

TTG AAC AAC AGG GAG AAT ATC TCT GAT CCC ACC TCC CCA CTG AGA AGG 240
Leu Asn Asn Arg Glu Asn Ile Ser Asp Pro Thr Ser Pro Leu Arg Arg
45 50 55

AAC TTT GTA TAC CAT TTG TCA GAC GTC TGT AAG AAA TGC GAT CCT GTG 288
Asn Phe Val Tyr His Leu Ser Asp Val Cys Lys Lys Cys Asp Pro Val
60 65 70

GAA GTG GAG CTG GAA GAT CAG GTT GTT ACT GCC ACC CAG AGC AAC ATC 336
Glu Val Glu Leu Glu Asp Gln Val Val Thr Ala Thr Gln Ser Asn Ile
75 80 85 90

TGC AAT GAG GAC GAT GGT GTT CCT GAG ACC TGC TAC ATG TAT GAC AGA 384
Cys Asn Glu Asp Asp Gly Val Pro Glu Thr Cys Tyr Met Tyr Asp Arg
95 100 105

AAC AAG TGC TAT ACC ACT ATG GTC CCA CTT AGG TAT CAT GGT GAG ACC 432
Asn Lys Cys Tyr Thr Thr Met Val Pro Leu Arg Tyr His Gly Glu Thr
110 115 120

AAA ATG GTG CAA GCA GCC TTG ACC CCC GAT TCT TGC TAC CCT GAC 477
Lys Met Val Gln Ala Ala Leu Thr Pro Asp Ser Cys Tyr Pro Asp
125 130 135

TAG 480

(2) INFORMATION FOR SEQ ID NO: 12:

(i) SEQUENCE CHARACTERISTICS:
(A) LENGTH: 159 amino acids
(B) TYPE: amino acid
(D) TOPOLOGY: linear

(ii) MOLECULE TYPE: protein
(xi) SEQUENCE DESCRIPTION: SEQ ID NO: 12:

Met Lys Thr His Leu Leu Leu Trp Gly Val Leu Ala Ile Phe Val Lys
-22 -20 -15 -10

Ala Val Leu Val Thr Gly Asp Asp Glu Ala Thr Ile Leu Ala Asp Asn
-5 1 5 10

Lys Cys Met Cys Thr Arg Val Thr Ser Arg Ile Ile Pro Ser Thr Glu
15 20 25

Asp Pro Asn Glu Asp Ile Val Glu Arg Asn Ile Arg Ile Val Val Pro
30 35 40

Leu Asn Asn Arg Glu Asn Ile Ser Asp Pro Thr Ser Pro Leu Arg Arg
        45                  50                  55

Asn Phe Val Tyr His Leu Ser Asp Val Cys Lys Lys Cys Asp Pro Val
    60                  65                  70

Glu Val Glu Leu Glu Asp Gln Val Val Thr Ala Thr Gln Ser Asn Ile
75                  80                  85                  90

Cys Asn Glu Asp Asp Gly Val Pro Glu Thr Cys Tyr Met Tyr Asp Arg
                95                  100                 105

Asn Lys Cys Tyr Thr Thr Met Val Pro Leu Arg Tyr His Gly Glu Thr
            110                 115                 120

Lys Met Val Gln Ala Ala Leu Thr Pro Asp Ser Cys Tyr Pro Asp
        125                 130                 135

(2) INFORMATION FOR SEQ ID NO: 13:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: protein

    (v) FRAGMENT TYPE: N-terminal

    (xi) SEQUENCE DESCRIPTION: SEQ ID NO: 13:

    Glu Val Gln Leu Gln Gln Ser Gly Pro Glu Leu Val Lys Pro Gly
    1               5                   10                  15

(2) INFORMATION FOR SEQ ID NO: 14:

    (i) SEQUENCE CHARACTERISTICS:
        (A) LENGTH: 21 amino acids
        (B) TYPE: amino acid
        (C) STRANDEDNESS: single
        (D) TOPOLOGY: linear

    (ii) MOLECULE TYPE: protein

    (v) FRAGMENT TYPE: N-terminal

    (xi) SEQUENCE DESCRIPTION: SEQ ID NO: 14:

    Asp Val Val Met Thr Gln Ser Pro Leu Ser Leu Pro Val Ser Leu Gly
    1               5                   10                  15

Asp Gln Ala Ser Ile
            20

(2) INFORMATION FOR SEQ ID NO: 15:

    (i) SEQUENCE CHARACTERISTICS:
        (A) LENGTH: 391 base pairs
        (B) TYPE: nucleic acid
        (C) STRANDEDNESS: double
        (D) TOPOLOGY: linear

    (ii) MOLECULE TYPE: cDNA to mRNA

    (iii) HYPOTHETICAL: NO

    (iv) ANTI-SENSE: NO

    (vi) ORIGINAL SOURCE:
        (A) ORGANISM: Mus musculus
        (G) CELL TYPE: Hybridoma
        (H) CELL LINE: CH11

    (ix) FEATURE:
        (A) NAME/KEY: CDS
        (B) LOCATION:2..391

    (ix) FEATURE:
        (A) NAME/KEY: mat_peptide
        (B) LOCATION:32..391

    (ix) FEATURE:
        (A) NAME/KEY: sig_peptide
        (B) LOCATION:2..31

    (xi) SEQUENCE DESCRIPTION: SEQ ID NO: 15:

C CTC CTG TCA GGA ACT GCA GGC GTC CAC TCT GAG GTC CAG CTT CAG      46
  Leu Leu Ser Gly Thr Ala Gly Val His Ser Glu Val Gln Leu Gln
  -10                 -5                  1               5

CAG TCA GGA CCT GAG CTG GTG AAA CCT GGG GCC TCA GTG AAG ATA TCC      94
Gln Ser Gly Pro Glu Leu Val Lys Pro Gly Ala Ser Val Lys Ile Ser
                10                  15                  20

TGC AAG GCT TCT GGA TAC ACA TTC ACT GAC TAC AAC ATG CAC TGG GTG      142
Cys Lys Ala Ser Gly Tyr Thr Phe Thr Asp Tyr Asn Met His Trp Val
            25                  30                  35

AAG CAG AGC CAT GGA AAG AGC CTT GAG TGG ATT GGA TAT ATT TAT CCT      190
Lys Gln Ser His Gly Lys Ser Leu Glu Trp Ile Gly Tyr Ile Tyr Pro
        40                  45                  50

TAC AAT GGT GGT ACT GGC TAC AAC CAG AAG TTC AAG AGC AAG GCC ACA      238
Tyr Asn Gly Gly Thr Gly Tyr Asn Gln Lys Phe Lys Ser Lys Ala Thr
    55                  60                  65

TTG ACT GTT GAC AAT TCC TCC AGC ACA GCC TAC ATG GAG CTC CGC AGC 286
Leu Thr Val Asp Asn Ser Ser Ser Thr Ala Tyr Met Glu Leu Arg Ser
70 75 80 85

CTG ACA TCT GAG GAC TCT GCA GTC TAT TAC TGT GCA AGA AGT TAC TAT 334
Leu Thr Ser Glu Asp Ser Ala Val Tyr Tyr Cys Ala Arg Ser Tyr Tyr
90 95 100

GCT ATG GAC TAC TGG GGT CAA GGA ACC TCA GTC ACC GTC TCC TCA GAG 382
Ala Met Asp Tyr Trp Gly Gln Gly Thr Ser Val Thr Val Ser Ser Glu
105 110 115

AGT CAG TCC 391
Ser Gln Ser
120

(2) INFORMATION FOR SEQ ID NO: 16:

(i) SEQUENCE CHARACTERISTICS:
(A) LENGTH: 388 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Mus musculus
(G) CELL TYPE: Hybridoma
(H) CELL LINE: CH11

(ix) FEATURE:
(A) NAME/KEY: CDS
(B) LOCATION:2..388

(ix) FEATURE:
(A) NAME/KEY: mat_peptide
(B) LOCATION:29..388

(ix) FEATURE:
(A) NAME/KEY: sig_peptide
(B) LOCATION:2..28

(xi) SEQUENCE DESCRIPTION: SEQ ID NO: 16:

G ATG TTC TGG ATT CCT GCT TCC AGC AGT GAT GTT GTG ATG ACC CAA 46
Met Phe Trp Ile Pro Ala Ser Ser Ser Asp Val Val Met Thr Gln
-9 -5 1 5

AGT CCA CTC TCC CTG CCT GTC AGT CTT GGA GAT CAA GCC TCC ATC TCT 94
Ser Pro Leu Ser Leu Pro Val Ser Leu Gly Asp Gln Ala Ser Ile Ser
10 15 20

TGC AGA TCT AGT AAG AGC CTT GTA CAC AGT AAT GGA AAC ACC TAT TTA 142
Cys Arg Ser Ser Lys Ser Leu Val His Ser Asn Gly Asn Thr Tyr Leu
25 30 35

CAT TGG TAC CTG CAG AAG CCA GGC CAG TCT CCA AAG CTC CTG ATC TAC 190
His Trp Tyr Leu Gln Lys Pro Gly Gln Ser Pro Lys Leu Leu Ile Tyr
40 45 50

AAA GTT TCC AAC CGA TTT TCT GGG GTC CCA GAC AGG TTC AGT GGC AGT 238
Lys Val Ser Asn Arg Phe Ser Gly Val Pro Asp Arg Phe Ser Gly Ser
55 60 65 70

GGA TCA GGG ACA GAT TTC ACA CTC AAG ATC AGC AGA GTG GAG GCT GAG 286
Gly Ser Gly Thr Asp Phe Thr Leu Lys Ile Ser Arg Val Glu Ala Glu
75 80 85

GAT CTG GGA GTT TAT TTC TGC TCT CAA AGT ACA CAT GTT CCT CCG GCG 334
Asp Leu Gly Val Tyr Phe Cys Ser Gln Ser Thr His Val Pro Pro Ala
90 95 100

TTC GGT GGA GGC ACC AAG CTG GAA ATC AAA CGG GCT GAT GCT GCA CCA 382
Phe Gly Gly Gly Thr Lys Leu Glu Ile Lys Arg Ala Asp Ala Ala Pro
105 110 115

ACT GTA 388
Thr Val
120

(2) INFORMATION FOR SEQ ID NO: 17:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 17:

CTAAGGGAAT TCCGCCTCTC CTCAGACACT GAA 33

(2) INFORMATION FOR SEQ ID NO: 18:

(i) SEQUENCE CHARACTERISTICS:
(A) LENGTH: 34 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 18:

TTTTACTCTA GAGACCCAAG GCCTGCCTGG TTGA 34

(2) INFORMATION FOR SEQ ID NO: 19:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 19:

AAATAGGAAT TCCAGTCTCC TCAGGCTGTC TCC 33

(2) INFORMATION FOR SEQ ID NO: 20:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 20:

ATGATCTCTA GAGTGGTGGC ATCTCAGGAC CT 32

(2) INFORMATION FOR SEQ ID NO: 21:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 21:

TTGCGGAATT CCTCACCTGT CCTGGGGTTA TT 32

(2) INFORMATION FOR SEQ ID NO: 22:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 22:

ATTGCCTCTA GAGCCTCTAA GGACAACGAG CT 32

(2) INFORMATION FOR SEQ ID NO: 23:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 23:

TGGGGCCTCA GTGAAGATAT 20

(2) INFORMATION FOR SEQ ID NO: 24:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 24:

CAATGGTGGT ACTGGCTACA 20

(2) INFORMATION FOR SEQ ID NO: 25:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 25:
   TGACATCTGA GGACTCTGCA 20

(2) INFORMATION FOR SEQ ID NO: 26:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(iii) HYPOTHETICAL: NO

(iv) ANTI-SENSE: NO

(xi) SEQUENCE DESCRIPTION: SEQ ID NO: 26:

TCCTCAGAGA GTCAGTCCTT                                                    20

(2) INFORMATION FOR SEQ ID NO: 27:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(iii) HYPOTHETICAL: NO

(iv) ANTI-SENSE: NO

(xi) SEQUENCE DESCRIPTION: SEQ ID NO: 27:

TCCTTCACCT GGAACTACCA                                                    20

(2) INFORMATION FOR SEQ ID NO: 28:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 28:

TCCCAAGAGC ATCCTTGAAG 20

(2) INFORMATION FOR SEQ ID NO: 29:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 29:

AGATCTGCAT GTGCCCATTC 20

(2) INFORMATION FOR SEQ ID NO: 30:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 30:

TCTAAACTCA TCTGCGAGGC [?]

(2) INFORMATION FOR SEQ ID NO: 31:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 31:

GGTGACCATC GAGAACAAAG 20

(2) INFORMATION FOR SEQ ID NO: 32:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 32:

AGGGGTCTCA CCTTCTTGAA 20

(2) INFORMATION FOR SEQ ID NO: 33:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 33:

TCCTTTGCCG ACATCTTCCT 20

(2) INFORMATION FOR SEQ ID NO: 34:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 34:

GTGTGTACTG TGACTCACAG 20

(2) INFORMATION FOR SEQ ID NO: 35:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 35:

AACTGAACCT GAGGGAGTCA 20

(2) INFORMATION FOR SEQ ID NO: 36:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 36:

AACTCTTGCC CCAAGAGAAG 20

(2) INFORMATION FOR SEQ ID NO: 37:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 37:

ATCCTGACTG TGACAGAGGA 20

(2) INFORMATION FOR SEQ ID NO: 38:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 38:

ACAAGTCCAC TGGTAAACCC 20

(2) INFORMATION FOR SEQ ID NO: 39:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 39:

AGGATATCTT CACTGAGGCC 20

(2) INFORMATION FOR SEQ ID NO: 40:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 40:

ATCCACTCAA GGCTCTTTCC 20

(2) INFORMATION FOR SEQ ID NO: 41:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 41:

ACTGCAGAGT CCTCAGATGT 20

(2) INFORMATION FOR SEQ ID NO: 42:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 42:

AGACGGTGAC TGAGGTTCTT 20

(2) INFORMATION FOR SEQ ID NO: 43:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 43:

CAGGTGAAGG AAATGGTGCT                                                    20

(2) INFORMATION FOR SEQ ID NO: 44:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(iii) HYPOTHETICAL: NO

(iv) ANTI-SENSE: NO

(xi) SEQUENCE DESCRIPTION: SEQ ID NO: 44:

ATGCTCTTGG GAGACAGCAA                                                    20

(2) INFORMATION FOR SEQ ID NO: 45:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(iii) HYPOTHETICAL: NO

(iv) ANTI-SENSE: NO

(xi) SEQUENCE DESCRIPTION: SEQ ID NO: 45:

CTCTGTTTTT GCCTCCGTAG                                                    20

(2) INFORMATION FOR SEQ ID NO: 46:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 46:

TGGCCTCGCA GATGAGTTTA 20

(2) INFORMATION FOR SEQ ID NO: 47:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 47:

CCTTTGTTCT CGATGGTCAC 20

(2) INFORMATION FOR SEQ ID NO: 48:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 48:

TGTGGAGGAC ACGTTCTTCA 20

(2) INFORMATION FOR SEQ ID NO: 49:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 49:

ACTTTGAGAA GCCCAGGAGA 20

(2) INFORMATION FOR SEQ ID NO: 50:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 50:

AGATCCCTGT GAGTCACAGT 20

(2) INFORMATION FOR SEQ ID NO: 51:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 51:

AGCAGGTGGA TGTTTGTGCA 20

(2) INFORMATION FOR SEQ ID NO: 52:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 52:

TGAAGCCACT GCACACTGAT 20

(2) INFORMATION FOR SEQ ID NO: 53:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 53:

AGTTCCATTC CTCCTCTGTC 20

(2) INFORMATION FOR SEQ ID NO: 54:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 54:

TGTGTCAGAC ATGATCAGGG 20

(2) INFORMATION FOR SEQ ID NO: 55:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 55:

TGAAGTTGCC TGTTAGGCTG 20

(2) INFORMATION FOR SEQ ID NO: 56:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 56:

CTTGGAGATC AAGCCTCCAT 20

(2) INFORMATION FOR SEQ ID NO: 57:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 57:

GCTGAGGATC TGGGAGTTTA 20

(2) INFORMATION FOR SEQ ID NO: 58:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 58:
   GATGCTGCAC CAACTGTATC 20

(2) INFORMATION FOR SEQ ID NO: 59:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 59:

CGACAAAATG GCGTCCTGAA 20

(2) INFORMATION FOR SEQ ID NO: 60:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 60:

ACGTTGACCA AGGACGAGTA 20

(2) INFORMATION FOR SEQ ID NO: 61:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 61:

ATCTGCAAGA GATGGAGGCT 20

(2) INFORMATION FOR SEQ ID NO: 62:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 62:

ACCCCAGAAA ATCGGTTGGA 20

(2) INFORMATION FOR SEQ ID NO: 63:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 63:

CCGGAGGAAC ATGTGTACTT 20

(2) INFORMATION FOR SEQ ID NO: 64:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 64:

TCGTTCATAC TCGTCCTTGG 20

(2) INFORMATION FOR SEQ ID NO: 65:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 65:

CATCTCAGGA CCTTTGTCTC 20

(2) INFORMATION FOR SEQ ID NO: 66:

(i) SEQUENCE CHARACTERISTICS:
(A) LENGTH: 20 base pairs

(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 66:

CACCTGTCCT GGGGTTATTT 20

(2) INFORMATION FOR SEQ ID NO: 67:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 67:

AGACAAGATG AAGACCCACC 20

(2) INFORMATION FOR SEQ ID NO: 68:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 68:

AAGCGACCAT TCTTGCTGAC 20

(2) INFORMATION FOR SEQ ID NO: 69:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 69:

ATATCTCTGA TCCCACCTCC 20

(2) INFORMATION FOR SEQ ID NO: 70:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 70:

GAAATGCGAT CCTGTGGAAG 20

(2) INFORMATION FOR SEQ ID NO: 71:

(i) SEQUENCE CHARACTERISTICS:
(A) LENGTH: 20 base pairs
(B) TYPE: nucleic acid

(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 71:

CTATACCACT ATGGTCCCAC 20

(2) INFORMATION FOR SEQ ID NO: 72:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 72:

AGAAGCAGGT GGGTCTTCAT 20

(2) INFORMATION FOR SEQ ID NO: 73:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 73:

TAGAGGTAAC TCGGGTACAC 20

(2) INFORMATION FOR SEQ ID NO: 74:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 74:

AAGTTCCTTC TCAGTGGGGA 20

(2) INFORMATION FOR SEQ ID NO: 75:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 75:

GGTGGCAGTA ACAACCTGAT 20

(2) INFORMATION FOR SEQ ID NO: 76:

(i) SEQUENCE CHARACTERISTICS:
(A) LENGTH: 20 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 76:

CATGATACCT AAGTGGGACC 20

(2) INFORMATION FOR SEQ ID NO: 77:

(i) SEQUENCE CHARACTERISTICS:
(A) LENGTH: 720 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(ix) FEATURE:
(A) NAME/KEY: CDS
(B) LOCATION:1..717

(ix) FEATURE:
(A) NAME/KEY: mat_peptide
(B) LOCATION:61..717

(ix) FEATURE:
(A) NAME/KEY: sig_peptide
(B) LOCATION:1..60

(xi) SEQUENCE DESCRIPTION: SEQ ID NO: 77:

ATG AGG CTC CCT GCT CAG CTC CTG GGG CTG CTA ATG CTC TGG GTC CCA 48
Met Arg Leu Pro Ala Gln Leu Leu Gly Leu Leu Met Leu Trp Val Pro
-20 -15 -10 -5

GGA TCC AGT GGG GAT GTT GTG ATG ACT CAG TCT CCA CTC TCC CTG CCC 96
Gly Ser Ser Gly Asp Val Val Met Thr Gln Ser Pro Leu Ser Leu Pro
1 5 10

GTC ACC CTT GGA CAG CCG GCC TCC ATC TCC TGC AGA TCT AGT AAG AGC 144
Val Thr Leu Gly Gln Pro Ala Ser Ile Ser Cys Arg Ser Ser Lys Ser
15 20 25

CTT GTA CAC AGT AAT GGA AAC ACC TAT TTA CAT TGG TAC CTG CAG AAG 192
Leu Val His Ser Asn Gly Asn Thr Tyr Leu His Trp Tyr Leu Gln Lys
30 35 40

CCA GGC CAG TCT CCA AAG CTC CTG ATC TAC AAA GTT TCC AAC CGA TTT 240
Pro Gly Gln Ser Pro Lys Leu Leu Ile Tyr Lys Val Ser Asn Arg Phe
45 50 55 60

TCT GGG GTC CCA GAC AGA TTC AGC GGC AGT GGG TCA GGC ACT GAT TTC 288
Ser Gly Val Pro Asp Arg Phe Ser Gly Ser Gly Ser Gly Thr Asp Phe
65 70 75

ACA CTG AAA ATC AGC AGG GTG GAG GCT GAG GAT GTT GGG GTT TAT TAC 336
Thr Leu Lys Ile Ser Arg Val Glu Ala Glu Asp Val Gly Val Tyr Tyr
80 85 90

TGC TCT CAA AGT ACA CAT GTT CCT CCG GCG TTC GGC CAA GGG ACC AAG 384
Cys Ser Gln Ser Thr His Val Pro Pro Ala Phe Gly Gln Gly Thr Lys
95 100 105

GTG GAA ATC AAA CGT ACT GTG GCT GCA CCA TCT GTC TTC ATC TTC CCG 432
Val Glu Ile Lys Arg Thr Val Ala Ala Pro Ser Val Phe Ile Phe Pro
110 115 120

CCA TCT GAT GAG CAG TTG AAA TCT GGA ACT GCC TCT GTT GTG TGC CTG 480
Pro Ser Asp Glu Gln Leu Lys Ser Gly Thr Ala Ser Val Val Cys Leu
125 130 135 140

CTG AAT AAC TTC TAT CCC AGA GAG GCC AAA GTA CAG TGG AAA GTG GAT 528
Leu Asn Asn Phe Tyr Pro Arg Glu Ala Lys Val Gln Trp Lys Val Asp
145 150 155

AAC GCC CTC CAA TCG GGT AAC TCC CAG GAG AGT GTC ACA GAG CAG GAC 576
Asn Ala Leu Gln Ser Gly Asn Ser Gln Glu Ser Val Thr Glu Gln Asp
160 165 170

AGC AAG GAC AGC ACC TAC AGC CTC AGC AGC ACC CTG ACG CTG AGC AAA 624
Ser Lys Asp Ser Thr Tyr Ser Leu Ser Ser Thr Leu Thr Leu Ser Lys
175 180 185

GCA GAC TAC GAG AAA CAC AAA GTC TAC GCC TGC GAA GTC ACC CAT CAG 672
Ala Asp Tyr Glu Lys His Lys Val Tyr Ala Cys Glu Val Thr His Gln
190 195 200

GGC CTG AGC TCG CCC GTC ACA AAG AGC TTC AAC AGG GGA GAG TGT 717
Gly Leu Ser Ser Pro Val Thr Lys Ser Phe Asn Arg Gly Glu Cys
205 210 215

TAG 720

(2) INFORMATION FOR SEQ ID NO: 78:

(i) SEQUENCE CHARACTERISTICS:
(A) LENGTH: 239 amino acids
(B) TYPE: amino acid

(D) TOPOLOGY: linear

(ii) MOLECULE TYPE: protein
(xi) SEQUENCE DESCRIPTION: SEQ ID NO: 77:

Met Arg Leu Pro Ala Gln Leu Leu Gly Leu Leu Met Leu Trp Val Pro
-20                 -15                 -10                  -5

Gly Ser Ser Gly Asp Val Val Met Thr Gln Ser Pro Leu Ser Leu Pro
                1               5                  10

Val Thr Leu Gly Gln Pro Ala Ser Ile Ser Cys Arg Ser Ser Lys Ser
        15                  20                  25

Leu Val His Ser Asn Gly Asn Thr Tyr Leu His Trp Tyr Leu Gln Lys
    30                  35                  40

Pro Gly Gln Ser Pro Lys Leu Leu Ile Tyr Lys Val Ser Asn Arg Phe
45                  50                  55                  60

Ser Gly Val Pro Asp Arg Phe Ser Gly Ser Gly Ser Gly Thr Asp Phe
                65                  70                  75

Thr Leu Lys Ile Ser Arg Val Glu Ala Glu Asp Val Gly Val Tyr Tyr
            80                  85                  90

Cys Ser Gln Ser Thr His Val Pro Pro Ala Phe Gly Gln Gly Thr Lys
        95                  100                 105


    110                 115                 120


125                 130                 135                 140


                145                 150                 155


            160                 165                 170


        175                 180                 185


    190                 195                 200


205                 210                 215


(2) INFORMATION FOR SEQ ID NO: 79:

(i) SEQUENCE CHARACTERISTICS:
(A) LENGTH: 720 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(ix) FEATURE:
(A) NAME/KEY: CDS
(B) LOCATION:1..717

(ix) FEATURE:
(A) NAME/KEY: mat_peptide
(B) LOCATION:61..717

(ix) FEATURE:
(A) NAME/KEY: sig_peptide
(B) LOCATION:1..60

(xi) SEQUENCE DESCRIPTION: SEQ ID NO: 79:

ATG AGG CTC CCT GCT CAG CTC CTG GGG CTG CTA ATG CTC TGG GTC CCA 48
Met Arg Leu Pro Ala Gln Leu Leu Gly Leu Leu Met Leu Trp Val Pro
-20 -15 -10 -5

GGA TCC AGT GGG GAT GTT GTG ATG ACT CAG TCT CCA CTC TCC CTG CCC 96
Gly Ser Ser Gly Asp Val Val Met Thr Gln Ser Pro Leu Ser Leu Pro
1 5 10

GTC ACC CTT GGA CAG CCG GCC TCC ATC TCC TGC AGA TCT AGT AAG AGC 144
Val Thr Leu Gly Gln Pro Ala Ser Ile Ser Cys Arg Ser Ser Lys Ser
15 20 25

CTT GTA CAC AGT AAT GGA AAC ACC TAT TTA CAT TGG TAC CTG CAG AAG 192
Leu Val His Ser Asn Gly Asn Thr Tyr Leu His Trp Tyr Leu Gln Lys
30 35 40

CCA GGC CAG TCT CCA AAG CTC CTG ATC TAC AAA GTT TCC AAC CGA TTT 240
Pro Gly Gln Ser Pro Lys Leu Leu Ile Tyr Lys Val Ser Asn Arg Phe
45 50 55 60

TCT GGG GTC CCA GAC AGA TTC AGC GGC AGT GGG TCA GGC ACT GAT TTC 288
Ser Gly Val Pro Asp Arg Phe Ser Gly Ser Gly Ser Gly Thr Asp Phe
65 70 75

ACA CTG AAA ATC AGC AGG GTG GAG GCT GAG GAT GTT GGG GTT TAT TTC 336
Thr Leu Lys Ile Ser Arg Val Glu Ala Glu Asp Val Gly Val Tyr Phe
80 85 90

TGC TCT CAA AGT ACA CAT GTT CCT CCG GCG TTC GGC CAA GGG ACC AAG 384
Cys Ser Gln Ser Thr His Val Pro Pro Ala Phe Gly Gln Gly Thr Lys
95 100 105

GTG GAA ATC AAA CGT ACT GTG GCT GCA CCA TCT GTC TTC ATC TTC CCG 432
Val Glu Ile Lys Arg Thr Val Ala Ala Pro Ser Val Phe Ile Phe Pro
110 115 120

CCA TCT GAT GAG CAG TTG AAA TCT GGA ACT GCC TCT GTT GTG TGC CTG 480
Pro Ser Asp Glu Gln Leu Lys Ser Gly Thr Ala Ser Val Val Cys Leu
125 130 135 140

CTG AAT AAC TTC TAT CCC AGA GAG GCC AAA GTA CAG TGG AAA GTG GAT 528
Leu Asn Asn Phe Tyr Pro Arg Glu Ala Lys Val Gln Trp Lys Val Asp
145 150 155

AAC GCC CTC CAA TCG GGT AAC TCC CAG GAG AGT GTC ACA GAG CAG GAC 576
Asn Ala Leu Gln Ser Gly Asn Ser Gln Glu Ser Val Thr Glu Gln Asp
160 165 170

AGC AAG GAC AGC ACC TAC AGC CTC AGC AGC ACC CTG ACG CTG AGC AAA 624
Ser Lys Asp Ser Thr Tyr Ser Leu Ser Ser Thr Leu Thr Leu Ser Lys
175 180 185

GCA GAC TAC GAG AAA CAC AAA GTC TAC GCC TGC GAA GTC ACC CAT CAG 672
Ala Asp Tyr Glu Lys His Lys Val Tyr Ala Cys Glu Val Thr His Gln
190 195 200

GGC CTG AGC TCG CCC GTC ACA AAG AGC TTC AAC AGG GGA GAG TGT 717
Gly Leu Ser Ser Pro Val Thr Lys Ser Phe Asn Arg Gly Glu Cys
205 210 215

TAG 720

(2) INFORMATION FOR SEQ ID NO: 80:

(i) SEQUENCE CHARACTERISTICS:
(A) LENGTH: 239 amino acids
(B) TYPE: amino acid
(D) TOPOLOGY: linear

(ii) MOLECULE TYPE: protein
(xi) SEQUENCE DESCRIPTION: SEQ ID NO: 80:

Met Arg Leu Pro Ala Gln Leu Leu Gly Leu Leu Met Leu Trp Val Pro
-20 -15 -10 -5

Gly Ser Ser Gly Asp Val Val Met Thr Gln Ser Pro Leu Ser Leu Pro
1 5 10

Val Thr Leu Gly Gln Pro Ala Ser Ile Ser Cys Arg Ser Ser Lys Ser
15 20 25

Leu Val His Ser Asn Gly Asn Thr Tyr Leu His Trp Tyr Leu Gln Lys
30 35 40

Pro Gly Gln Ser Pro Lys Leu Leu Ile Tyr Lys Val Ser Asn Arg Phe
45 50 55 60

Ser Gly Val Pro Asp Arg Phe Ser Gly Ser Gly Ser Gly Thr Asp Phe
65 70 75

Thr Leu Lys Ile Ser Arg Val Glu Ala Glu Asp Val Gly Val Tyr Phe
80 85 90

Cys Ser Gln Ser Thr His Val Pro Pro Ala Phe Gly Gln Gly Thr Lys
        95                  100                 105

Val Glu Ile Lys Arg Thr Val Ala Ala Pro Ser Val Phe Ile Phe Pro
    110                 115                 120

Pro Ser Asp Glu Gln Leu Lys Ser Gly Thr Ala Ser Val Val Cys Leu
125                 130                 135                 140

Leu Asn Asn Phe Tyr Pro Arg Glu Ala Lys Val Gln Trp Lys Val Asp
                145                 150                 155

Asn Ala Leu Gln Ser Gly Asn Ser Gln Glu Ser Val Thr Glu Gln Asp
            160                 165                 170

Ser Lys Asp Ser Thr Tyr Ser Leu Ser Ser Thr Leu Thr Leu Ser Lys
        175                 180                 185

Ala Asp Tyr Glu Lys His Lys Val Tyr Ala Cys Glu Val Thr His Gln
    190                 195                 200

Gly Leu Ser Ser Pro Val Thr Lys Ser Phe Asn Arg Gly Glu Cys
205                 210                 215

(2) INFORMATION FOR SEQ ID NO: 81:

     (i) SEQUENCE CHARACTERISTICS:
          (A) LENGTH: 720 base pairs
          (B) TYPE: nucleic acid
          (C) STRANDEDNESS: double
          (D) TOPOLOGY: linear

    (ii) MOLECULE TYPE: cDNA to mRNA

   (iii) HYPOTHETICAL: NO

    (iv) ANTI-SENSE: NO

(ix) FEATURE:
          (A) NAME/KEY: CDS
          (B) LOCATION:1..717

    (ix) FEATURE:
          (A) NAME/KEY: mat_peptide
          (B) LOCATION:61..717

    (ix) FEATURE:
          (A) NAME/KEY: sig_peptide
          (B) LOCATION:1..60

    (xi) SEQUENCE DESCRIPTION: SEQ ID NO: 81:

ATG AGG CTC CCT GCT CAG CTC CTG GGG CTG CTA ATG CTC TGG GTC CCA          48
Met Arg Leu Pro Ala Gln Leu Leu Gly Leu Leu Met Leu Trp Val Pro
-20                 -15                 -10                  -5

GGA TCC AGT GGG GAT GTT GTG ATG ACT CAG TCT CCA CTC TCC CTG CCC 96
Gly Ser Ser Gly Asp Val Val Met Thr Gln Ser Pro Leu Ser Leu Pro
1 5 10

GTC ACC CTT GGA CAG CCG GCC TCC ATC TCC TGC AGA TCT AGT AAG AGC 144
Val Thr Leu Gly Gln Pro Ala Ser Ile Ser Cys Arg Ser Ser Lys Ser
15 20 25

CTT GTA CAC AGT AAT GGA AAC ACC TAT TTA CAT TGG TAC CTG CAG AAG 192
Leu Val His Ser Asn Gly Asn Thr Tyr Leu His Trp Tyr Leu Gln Lys
30 35 40

CCA GGC CAG TCT CCA AGG CTC CTG ATC TAC AAA GTT TCC AAC CGA TTT 240
Pro Gly Gln Ser Pro Arg Leu Leu Ile Tyr Lys Val Ser Asn Arg Phe
45 50 55 60

TCT GGG GTC CCA GAC AGA TTC AGC GGC AGT GGG TCA GGC ACT GAT TTC 288
Ser Gly Val Pro Asp Arg Phe Ser Gly Ser Gly Ser Gly Thr Asp Phe
65 70 75

ACA CTG AAA ATC AGC AGG GTG GAG GCT GAG GAT GTT GGG GTT TAT TAC 336
Thr Leu Lys Ile Ser Arg Val Glu Ala Glu Asp Val Gly Val Tyr Tyr
80 85 90

TGC TCT CAA AGT ACA CAT GTT CCT CCG GCG TTC GGC CAA GGG ACC AAG 384
Cys Ser Gln Ser Thr His Val Pro Pro Ala Phe Gly Gln Gly Thr Lys
95 100 105

GTG GAA ATC AAA CGT ACT GTG GCT GCA CCA TCT GTC TTC ATC TTC CCG 432
Val Glu Ile Lys Arg Thr Val Ala Ala Pro Ser Val Phe Ile Phe Pro
110 115 120

CCA TCT GAT GAG CAG TTG AAA TCT GGA ACT GCC TCT GTT GTG TGC CTG 480
Pro Ser Asp Glu Gln Leu Lys Ser Gly Thr Ala Ser Val Val Cys Leu
125 130 135 140

CTG AAT AAC TTC TAT CCC AGA GAG GCC AAA GTA CAG TGG AAA GTG GAT 528
Leu Asn Asn Phe Tyr Pro Arg Glu Ala Lys Val Gln Trp Lys Val Asp
145 150 155

AAC GCC CTC CAA TCG GGT AAC TCC CAG GAG AGT GTC ACA GAG CAG GAC 576
Asn Ala Leu Gln Ser Gly Asn Ser Gln Glu Ser Val Thr Glu Gln Asp
160 165 170

AGC AAG GAC AGC ACC TAC AGC CTC AGC AGC ACC CTG ACG CTG AGC AAA 624
Ser Lys Asp Ser Thr Tyr Ser Leu Ser Ser Thr Leu Thr Leu Ser Lys
175 180 185

GCA GAC TAC GAG AAA CAC AAA GTC TAC GCC TGC GAA GTC ACC CAT CAG 672
Ala Asp Tyr Glu Lys His Lys Val Tyr Ala Cys Glu Val Thr His Gln
190 195 200

GGC CTG AGC TCG CCC GTC ACA AAG AGC TTC AAC AGG GGA GAG TGT 717
Gly Leu Ser Ser Pro Val Thr Lys Ser Phe Asn Arg Gly Glu Cys
205 210 215

TAG 720

(2) INFORMATION FOR SEQ ID NO: 82:

(i) SEQUENCE CHARACTERISTICS:
(A) LENGTH: 239 amino acids
(B) TYPE: amino acid
(D) TOPOLOGY: linear

(ii) MOLECULE TYPE: protein
(xi) SEQUENCE DESCRIPTION: SEQ ID NO: 82:

Met Arg Leu Pro Ala Gln Leu Leu Gly Leu Leu Met Leu Trp Val Pro
-20 -15 -10 -5

Gly Ser Ser Gly Asp Val Val Met Thr Gln Ser Pro Leu Ser Leu Pro
1 5 10

Val Thr Leu Gly Gln Pro Ala Ser Ile Ser Cys Arg Ser Ser Lys Ser
15 20 25

Leu Val His Ser Asn Gly Asn Thr Tyr Leu His Trp Tyr Leu Gln Lys
30 35 40

Pro Gly Gln Ser Pro Arg Leu Leu Ile Tyr Lys Val Ser Asn Arg Phe
45 50 55 60

Ser Gly Val Pro Asp Arg Phe Ser Gly Ser Gly Ser Gly Thr Asp Phe
65 70 75

Thr Leu Lys Ile Ser Arg Val Glu Ala Glu Asp Val Gly Val Tyr Tyr
80 85 90

Cys Ser Gln Ser Thr His Val Pro Pro Ala Phe Gly Gln Gly Thr Lys
95 100 105

Val Glu Ile Lys Arg Thr Val Ala Ala Pro Ser Val Phe Ile Phe Pro
110 115 120

Pro Ser Asp Glu Gln Leu Lys Ser Gly Thr Ala Ser Val Val Cys Leu
125 130 135 140

Leu Asn Asn Phe Tyr Pro Arg Glu Ala Lys Val Gln Trp Lys Val Asp
145 150 155

Asn Ala Leu Gln Ser Gly Asn Ser Gln Glu Ser Val Thr Glu Gln Asp
160 165 170

Ser Lys Asp Ser Thr Tyr Ser Leu Ser Ser Thr Leu Thr Leu Ser Lys
175 180 185

Ala Asp Tyr Glu Lys His Lys Val Tyr Ala Cys Glu Val Thr His Gln
190 195 200

Gly Leu Ser Ser Pro Val Thr Lys Ser Phe Asn Arg Gly Glu Cys
205 210 215

(2) INFORMATION FOR SEQ ID NO: 83:

(i) SEQUENCE CHARACTERISTICS:
(A) LENGTH: 720 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(ix) FEATURE:
(A) NAME/KEY: CDS
(B) LOCATION:1..717

(ix) FEATURE:
(A) NAME/KEY: mat_peptide
(B) LOCATION:61..717

(ix) FEATURE:
(A) NAME/KEY: sig_peptide
(B) LOCATION:1..60

(xi) SEQUENCE DESCRIPTION: SEQ ID NO: 83:

ATG AGG CTC CCT GCT CAG CTC CTG GGG CTG CTA ATG CTC TGG GTC CCA 48
Met Arg Leu Pro Ala Gln Leu Leu Gly Leu Leu Met Leu Trp Val Pro
-20 -15 -10 -5

GGA TCC AGT GGG GAT GTT GTG ATG ACT CAG TCT CCA CTC TCC CTG CCC 96
Gly Ser Ser Gly Asp Val Val Met Thr Gln Ser Pro Leu Ser Leu Pro
1 5 10

GTC ACC CTT GGA CAG CCG GCC TCC ATC TCC TGC AGA TCT AGT AAG AGC 144
Val Thr Leu Gly Gln Pro Ala Ser Ile Ser Cys Arg Ser Ser Lys Ser
15 20 25

CTT GTA CAC AGT AAT GGA AAC ACC TAT TTA CAT TGG TAC CTG CAG AAG 192
Leu Val His Ser Asn Gly Asn Thr Tyr Leu His Trp Tyr Leu Gln Lys
30 35 40

CCA GGC CAG TCT CCA AGG CTC CTG ATC TAC AAA GTT TCC AAC CGA TTT 240
Pro Gly Gln Ser Pro Arg Leu Leu Ile Tyr Lys Val Ser Asn Arg Phe
45 50 55 60

TCT GGG GTC CCA GAC AGA TTC AGC GGC AGT GGG TCA GGC ACT GAT TTC 288
Ser Gly Val Pro Asp Arg Phe Ser Gly Ser Gly Ser Gly Thr Asp Phe
65 70 75

ACA CTG AAA ATC AGC AGG GTG GAG GCT GAG GAT GTT GGG GTT TAT TTC 336
Thr Leu Lys Ile Ser Arg Val Glu Ala Glu Asp Val Gly Val Tyr Phe
80 85 90

TGC TCT CAA AGT ACA CAT GTT CCT CCG GCG TTC GGC CAA GGG ACC AAG 384
Cys Ser Gln Ser Thr His Val Pro Pro Ala Phe Gly Gln Gly Thr Lys
95 100 105

GTG GAA ATC AAA CGT ACT GTG GCT GCA CCA TCT GTC TTC ATC TTC CCG 432
Val Glu Ile Lys Arg Thr Val Ala Ala Pro Ser Val Phe Ile Phe Pro
110 115 120

CCA TCT GAT GAG CAG TTG AAA TCT GGA ACT GCC TCT GTT GTG TGC CTG 480
Pro Ser Asp Glu Gln Leu Lys Ser Gly Thr Ala Ser Val Val Cys Leu
125 130 135 140

CTG AAT AAC TTC TAT CCC AGA GAG GCC AAA GTA CAG TGG AAG GTG GAT 528
Leu Asn Asn Phe Tyr Pro Arg Glu Ala Lys Val Gln Trp Lys Val Asp
145 150 155

AAC GCC CTC CAA TCG GGT AAC TCC CAG GAG AGT GTC ACA GAG CAG GAC 576
Asn Ala Leu Gln Ser Gly Asn Ser Gln Glu Ser Val Thr Glu Gln Asp
160 165 170

AGC AAG GAC AGC ACC TAC AGC CTC AGC AGC ACC CTG ACG CTG AGC AAA 624
Ser Lys Asp Ser Thr Tyr Ser Leu Ser Ser Thr Leu Thr Leu Ser Lys
175 180 185

GCA GAC TAC GAG AAA CAC AAA GTC TAC GCC TGC GAA GTC ACC CAT CAG 672
Ala Asp Tyr Glu Lys His Lys Val Tyr Ala Cys Glu Val Thr His Gln
190 195 200

GGC CTG AGC TCG CCC GTC ACA AAG AGC TTC AAC AGG GGA GAG TGT 717
Gly Leu Ser Ser Pro Val Thr Lys Ser Phe Asn Arg Gly Glu Cys
205 210 215

TAG 720

(2) INFORMATION FOR SEQ ID NO: 84:

(i) SEQUENCE CHARACTERISTICS:
(A) LENGTH: 239 amino acids
(B) TYPE: amino acid
(D) TOPOLOGY: linear

(ii) MOLECULE TYPE: protein
(xi) SEQUENCE DESCRIPTION: SEQ ID NO: 84:

Met Arg Leu Pro Ala Gln Leu Leu Gly Leu Leu Met Leu Trp Val Pro
-20 -15 -10 -5

Gly Ser Ser Gly Asp Val Val Met Thr Gln Ser Pro Leu Ser Leu Pro
1 5 10

Val Thr Leu Gly Gln Pro Ala Ser Ile Ser Cys Arg Ser Ser Lys Ser
15 20 25

Leu Val His Ser Asn Gly Asn Thr Tyr Leu His Trp Tyr Leu Gln Lys
30 35 40

Pro Gly Gln Ser Pro Arg Leu Leu Ile Tyr Lys Val Ser Asn Arg Phe
45                  50                  55                  60

Ser Gly Val Pro Asp Arg Phe Ser Gly Ser Gly Ser Gly Thr Asp Phe
                65                  70                  75

Thr Leu Lys Ile Ser Arg Val Glu Ala Glu Asp Val Gly Val Tyr Phe
            80                  85                  90

Cys Ser Gln Ser Thr His Val Pro Pro Ala Phe Gly Gln Gly Thr Lys
        95                  100                 105

Val Glu Ile Lys Arg Thr Val Ala Ala Pro Ser Val Phe Ile Phe Pro
    110                 115                 120

Pro Ser Asp Glu Gln Leu Lys Ser Gly Thr Ala Ser Val Val Cys Leu
125                 130                 135                 140

Leu Asn Asn Phe Tyr Pro Arg Glu Ala Lys Val Gln Trp Lys Val Asp
                145                 150                 155

Asn Ala Leu Gln Ser Gly Asn Ser Gln Glu Ser Val Thr Glu Gln Asp
            160                 165                 170

Ser Lys Asp Ser Thr Tyr Ser Leu Ser Ser Thr Leu Thr Leu Ser Lys
        175                 180                 185

Ala Asp Tyr Glu Lys His Lys Val Tyr Ala Cys Glu Val Thr His Gln
    190                 195                 200

Gly Leu Ser Ser Pro Val Thr Lys Ser Phe Asn Arg Gly Glu Cys
205                 210                 215

(2) INFORMATION FOR SEQ ID NO: 85:

(i) SEQUENCE CHARACTERISTICS:
(A) LENGTH: 1768 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(ix) FEATURE:
(A) NAME/KEY: CDS
(B) LOCATION:1..1764

(ix) FEATURE:
(A) NAME/KEY: mat_peptide
(B) LOCATION:58..1764

(ix) FEATURE:

(A) NAME/KEY: sig_peptide
(B) LOCATION:1..57

(xi) SEQUENCE DESCRIPTION: SEQ ID NO: 85:

ATG GGA TGG AGC TGG ATC TTT CTC TTC CTC CTG TCA GGA ACT GCA GGC 48
Met Gly Trp Ser Trp Ile Phe Leu Phe Leu Leu Ser Gly Thr Ala Gly
-19 -15 -10 -5

GTC CAC TCT GAG GTG CAG CTT GTG CAG TCT GGG GCT GAG GTG AAG AAG 96
Val His Ser Glu Val Gln Leu Val Gln Ser Gly Ala Glu Val Lys Lys
1 5 10

CCT GGG GCC TCA GTG AAG GTT TCC TGC AAG GCT TCT GGA TAC ACC TTC 144
Pro Gly Ala Ser Val Lys Val Ser Cys Lys Ala Ser Gly Tyr Thr Phe
15 20 25

ACT GAC TAT AAT ATG CAT TGG GTG CGC CAG GCC CCC GGA CAA GGA CTC 192
Thr Asp Tyr Asn Met His Trp Val Arg Gln Ala Pro Gly Gln Gly Leu
30 35 40 45

GAA TGG ATG GGA TAT ATT TAT CCT TAC AAT GGT GGT ACT GGC TAC AAC 240
Glu Trp Met Gly Tyr Ile Tyr Pro Tyr Asn Gly Gly Thr Gly Tyr Asn
    50                  55                  60

CAG AAG TTC AAG AGC AAG GCC ACA TTG ACT GTT GAC AAT TCC GCG AGC 288
Gln Lys Phe Lys Ser Lys Ala Thr Leu Thr Val Asp Asn Ser Ala Ser
65 70 75

ACA GCC TAC ATG GAG CTG AGC AGC CTG AGA TCT GAA GAC ACG GCT GTG 336
Thr Ala Tyr Met Glu Leu Ser Ser Leu Arg Ser Glu Asp Thr Ala Val
80 85 90

TAT TAC TGT GCG AGA AGT TAC TAT GCT ATG GAC TAC TGG GGC CAG GGA 384
Tyr Tyr Cys Ala Arg Ser Tyr Tyr Ala Met Asp Tyr Trp Gly Gln Gly
95 100 105

ACC CTG GTC ACC GTC TCC TCA GGG AGT GCA TCC GCC CCA ACC CTT TTC 432
Thr Leu Val Thr Val Ser Ser Gly Ser Ala Ser Ala Pro Thr Leu Phe
110 115 120 125

CCC CTC GTC TCC TGT GAG AAT TCC CCG TCG GAT ACG AGC AGC GTG GCC 480
Pro Leu Val Ser Cys Glu Asn Ser Pro Ser Asp Thr Ser Ser Val Ala
    130                 135                 140

GTT GGC TGC CTC GCA CAG GAC TTC CTT CCC GAC TCC ATC ACT TTC TCC 528
Val Gly Cys Leu Ala Gln Asp Phe Leu Pro Asp Ser Ile Thr Phe Ser
145 150 155

TGG AAA TAC AAG AAC AAC TCT GAC ATC AGC AGT ACC CGG GGC TTC CCA 576
Trp Lys Tyr Lys Asn Asn Ser Asp Ile Ser Ser Thr Arg Gly Phe Pro
160 165 170

TCA GTC CTG AGA GGG GGC AAG TAC GCA GCC ACC TCA CAG GTG CTG CTG 624
Ser Val Leu Arg Gly Gly Lys Tyr Ala Ala Thr Ser Gln Val Leu Leu
175 180 185

CCT TCC AAG GAC GTC ATG CAG GGC ACA GAC GAA CAC GTG GTG TGC AAA 672
Pro Ser Lys Asp Val Met Gln Gly Thr Asp Glu His Val Val Cys Lys
190 195 200 205

GTC CAG CAC CCC AAC GGC AAC AAA GAA AAG AAC GTG CCT CTT CCA GTG 720
Val Gln His Pro Asn Gly Asn Lys Glu Lys Asn Val Pro Leu Pro Val
    210                 215                 220

ATT GCC GAG CTG CCT CCC AAA GTG AGC GTC TTC GTC CCA CCC CGC GAC 768
Ile Ala Glu Leu Pro Pro Lys Val Ser Val Phe Val Pro Pro Arg Asp
225 230 235

GGC TTC TTC GGC AAC CCC CGC AAG TCC AAG CTC ATC TGC CAG GCC ACG 816
Gly Phe Phe Gly Asn Pro Arg Lys Ser Lys Leu Ile Cys Gln Ala Thr
240 245 250

GGT TTC AGT CCC CGG CAG ATT CAG GTG TCC TGG CTG CGC GAG GGG AAG 864
Gly Phe Ser Pro Arg Gln Ile Gln Val Ser Trp Leu Arg Glu Gly Lys
255 260 265

CAG GTG GGG TCT GGC GTC ACC ACG GAC CAG GTG CAG GCT GAG GCC AAA 912
Gln Val Gly Ser Gly Val Thr Thr Asp Gln Val Gln Ala Glu Ala Lys
270 275 280 285

GAG TCT GGG CCC ACG ACC TAC AAG GTG ACC AGC ACA CTG ACC ATC AAA 960
Glu Ser Gly Pro Thr Thr Tyr Lys Val Thr Ser Thr Leu Thr Ile Lys
    290                 295                 300

GAG AGC GAC TGG CTC AGC CAG AGC ATG TTC ACC TGC CGC GTG GAT CAC 1008
Glu Ser Asp Trp Leu Ser Gln Ser Met Phe Thr Cys Arg Val Asp His
305 310 315

AGG GGC CTG ACC TTC CAG CAG AAT GCG TCC TCC ATG TGT GTC CCC GAT 1056
Arg Gly Leu Thr Phe Gln Gln Asn Ala Ser Ser Met Cys Val Pro Asp
320 325 330

CAA GAC ACA GCC ATC CGG GTC TTC GCC ATC CCC CCA TCC TTT GCC AGC 1104
Gln Asp Thr Ala Ile Arg Val Phe Ala Ile Pro Pro Ser Phe Ala Ser
335 340 345

ATC TTC CTC ACC AAG TCC ACC AAG TTG ACC TGC CTG GTC ACA GAC CTG 1152
Ile Phe Leu Thr Lys Ser Thr Lys Leu Thr Cys Leu Val Thr Asp Leu
350 355 360 365

ACC ACC TAT GAC AGC GTG ACC ATC TCC TGG ACC CGC CAG AAT GGC GAA 1200
Thr Thr Tyr Asp Ser Val Thr Ile Ser Trp Thr Arg Gln Asn Gly Glu
    370                 375                 380

GCT GTG AAA ACC CAC ACC AAC ATC TCC GAG AGC CAC CCC AAT GCC ACT 1248
Ala Val Lys Thr His Thr Asn Ile Ser Glu Ser His Pro Asn Ala Thr
385 390 395

TTC AGC GCC GTG GGT GAG GCC AGC ATC TGC GAG GAT GAC TGG AAT TCC 1296
Phe Ser Ala Val Gly Glu Ala Ser Ile Cys Glu Asp Asp Trp Asn Ser
400 405 410

GGG GAG AGG TTC ACG TGC ACC GTG ACC CAC ACA GAC CTG CCC TCG CCA 1344
Gly Glu Arg Phe Thr Cys Thr Val Thr His Thr Asp Leu Pro Ser Pro
415 420 425

CTG AAG CAG ACC ATC TCC CGG CCC AAG GGG GTG GCC CTG CAC AGG CCC 1392
Leu Lys Gln Thr Ile Ser Arg Pro Lys Gly Val Ala Leu His Arg Pro
430 435 440 445

GAT GTC TAC TTG CTG CCA CCA GCC CGG GAG CAG CTG AAC CTG CGG GAG 1440
Asp Val Tyr Leu Leu Pro Pro Ala Arg Glu Gln Leu Asn Leu Arg Glu
    450                 455                 460

TCG GCC ACC ATC ACG TGC CTG GTG ACG GGC TTC TCT CCC GCG GAC GTC 1488
Ser Ala Thr Ile Thr Cys Leu Val Thr Gly Phe Ser Pro Ala Asp Val
465 470 475

TTC GTG CAG TGG ATG CAG AGG GGG CAG CCC TTG TCC CCG GAG AAG TAT 1536
Phe Val Gln Trp Met Gln Arg Gly Gln Pro Leu Ser Pro Glu Lys Tyr
480 485 490

GTG ACC AGC GCC CCA ATG CCT GAG CCC CAG GCC CCA GGC CGG TAC TTC 1584
Val Thr Ser Ala Pro Met Pro Glu Pro Gln Ala Pro Gly Arg Tyr Phe
495 500 505

GCC CAC AGC ATC CTG ACC GTG TCC GAA GAG GAA TGG AAC ACG GGG GAG 1632
Ala His Ser Ile Leu Thr Val Ser Glu Glu Glu Trp Asn Thr Gly Glu
510 515 520 525

ACC TAC ATC TGC GTG GTG GCC CAT GAG GCC CTG CCC AAC AGG GTC ACC 1680
Thr Tyr Ile Cys Val Val Ala His Glu Ala Leu Pro Asn Arg Val Thr
    530                 535                 540

GAG AGG ACC GTG GAC AAG TCC ACC GGT AAA CCC ACC CTG TAC AAC GTG 1728
Glu Arg Thr Val Asp Lys Ser Thr Gly Lys Pro Thr Leu Tyr Asn Val
545 550 555

TCC CTG GTC ATG TCC GAC ACA GCT GGC ACC TGC TAC TGA 1767
Ser Leu Val Met Ser Asp Thr Ala Gly Thr Cys Tyr
560 565

(2) INFORMATION FOR SEQ ID NO: 86:

(i) SEQUENCE CHARACTERISTICS:
(A) LENGTH: 588 amino acids
(B) TYPE: amino acid
(D) TOPOLOGY: linear

(ii) MOLECULE TYPE: protein
(xi) SEQUENCE DESCRIPTION: SEQ ID NO: 86:

Met Gly Trp Ser Trp Ile Phe Leu Phe Leu Leu Ser Gly Thr Ala Gly
-19 -15 -10 -5

Val His Ser Glu Val Gln Leu Val Gln Ser Gly Ala Glu Val Lys Lys
1 5 10

Pro Gly Ala Ser Val Lys Val Ser Cys Lys Ala Ser Gly Tyr Thr Phe
15 20 25

Thr Asp Tyr Asn Met His Trp Val Arg Gln Ala Pro Gly Gln Gly Leu
30 35 40 45

Glu Trp Met Gly Tyr Ile Tyr Pro Tyr Asn Gly Gly Thr Gly Tyr Asn
    50                  55                  60

Gln Lys Phe Lys Ser Lys Ala Thr Leu Thr Val Asp Asn Ser Ala Ser
65 70 75

Thr Ala Tyr Met Glu Leu Ser Ser Leu Arg Ser Glu Asp Thr Ala Val
80 85 90

Tyr Tyr Cys Ala Arg Ser Tyr Tyr Ala Met Asp Tyr Trp Gly Gln Gly
95 100 105

Thr Leu Val Thr Val Ser Ser Gly Ser Ala Ser Ala Pro Thr Leu Phe
110 115 120 125

Pro Leu Val Ser Cys Glu Asn Ser Pro Ser Asp Thr Ser Ser Val Ala
    130                 135                 140

Val Gly Cys Leu Ala Gln Asp Phe Leu Pro Asp Ser Ile Thr Phe Ser
145 150 155

Trp Lys Tyr Lys Asn Asn Ser Asp Ile Ser Ser Thr Arg Gly Phe Pro
160 165 170

Ser Val Leu Arg Gly Gly Lys Tyr Ala Ala Thr Ser Gln Val Leu Leu
175 180 185

Pro Ser Lys Asp Val Met Gln Gly Thr Asp Glu His Val Val Cys Lys
190 195 200 205

Val Gln His Pro Asn Gly Asn Lys Glu Lys Asn Val Pro Leu Pro Val
    210                 215                 220

Ile Ala Glu Leu Pro Pro Lys Val Ser Val Phe Val Pro Pro Arg Asp
225 230 235

Gly Phe Phe Gly Asn Pro Arg Lys Ser Lys Leu Ile Cys Gln Ala Thr
240 245 250

Gly Phe Ser Pro Arg Gln Ile Gln Val Ser Trp Leu Arg Glu Gly Lys
255 260 265

Gln Val Gly Ser Gly Val Thr Thr Asp Gln Val Gln Ala Glu Ala Lys
270 275 280 285

Glu Ser Gly Pro Thr Thr Tyr Lys Val Thr Ser Thr Leu Thr Ile Lys
    290                 295                 300

Glu Ser Asp Trp Leu Ser Gln Ser Met Phe Thr Cys Arg Val Asp His
305 310 315

Arg Gly Leu Thr Phe Gln Gln Asn Ala Ser Ser Met Cys Val Pro Asp
      320                 325                 330

Gln Asp Thr Ala Ile Arg Val Phe Ala Ile Pro Pro Ser Phe Ala Ser
    335                 340                 345

Ile Phe Leu Thr Lys Ser Thr Lys Leu Thr Cys Leu Val Thr Asp Leu
350                 355                 360                 365

Thr Thr Tyr Asp Ser Val Thr Ile Ser Trp Thr Arg Gln Asn Gly Glu
                370                 375                 380

Ala Val Lys Thr His Thr Asn Ile Ser Glu Ser His Pro Asn Ala Thr
            385                 390                 395

Phe Ser Ala Val Gly Glu Ala Ser Ile Cys Glu Asp Asp Trp Asn Ser
      400                 405                 410

Gly Glu Arg Phe Thr Cys Thr Val Thr His Thr Asp Leu Pro Ser Pro
    415                 420                 425

Leu Lys Gln Thr Ile Ser Arg Pro Lys Gly Val Ala Leu His Arg Pro
430                 435                 440                 445

Asp Val Tyr Leu Leu Pro Pro Ala Arg Glu Gln Leu Asn Leu Arg Glu
                450                 455                 460

Ser Ala Thr Ile Thr Cys Leu Val Thr Gly Phe Ser Pro Ala Asp Val
            465                 470                 475

Phe Val Gln Trp Met Gln Arg Gly Gln Pro Leu Ser Pro Glu Lys Tyr
      480                 485                 490

Val Thr Ser Ala Pro Met Pro Glu Pro Gln Ala Pro Gly Arg Tyr Phe
    495                 500                 505

Ala His Ser Ile Leu Thr Val Ser Glu Glu Glu Trp Asn Thr Gly Glu
510                 515                 520                 525

Thr Tyr Ile Cys Val Val Ala His Glu Ala Leu Pro Asn Arg Val Thr
                530                 535                 540

Glu Arg Thr Val Asp Lys Ser Thr Gly Lys Pro Thr Leu Tyr Asn Val
            545                 550                 555

Ser Leu Val Met Ser Asp Thr Ala Gly Thr Cys Tyr
      560                 565

(2) INFORMATION FOR SEQ ID NO: 87:

    (i) SEQUENCE CHARACTERISTICS:
        (A) LENGTH: 1768 base pairs
        (B) TYPE: nucleic acid
        (C) STRANDEDNESS: double
        (D) TOPOLOGY: linear

   (ii) MOLECULE TYPE: cDNA to mRNA

(iii) HYPOTHETICAL: NO

(iv) ANTI-SENSE: NO

(ix) FEATURE:
(A) NAME/KEY: CDS
(B) LOCATION:1..1764

(ix) FEATURE:
(A) NAME/KEY: mat_peptide
(B) LOCATION:58..1764

(ix) FEATURE:
(A) NAME/KEY: sig_peptide
(B) LOCATION:1..57

(xi) SEQUENCE DESCRIPTION: SEQ ID NO: 87:

ATG GGA TGG AGC TGG ATC TTT CTC TTC CTC CTG TCA GGA ACT GCA GGC 48
Met Gly Trp Ser Trp Ile Phe Leu Phe Leu Leu Ser Gly Thr Ala Gly
-19 -15 -10 -5

GTC CAC TCT GAG GTG CAG CTT GTG CAG TCT GGG GCT GAG GTG AAG AAG 96
Val His Ser Glu Val Gln Leu Val Gln Ser Gly Ala Glu Val Lys Lys
1 5 10

CCT GGG GCC TCA GTG AAG GTT TCC TGC AAG GCT TCT GGA TAC ACC TTC 144
Pro Gly Ala Ser Val Lys Val Ser Cys Lys Ala Ser Gly Tyr Thr Phe
15 20 25

ACT GAC TAT AAT ATG CAT TGG GTG AAG CAG GCC CAT GGA AAG AGC CTC 192
Thr Asp Tyr Asn Met His Trp Val Lys Gln Ala His Gly Lys Ser Leu
30 35 40 45

GAA TGG ATG GGA TAT ATT TAT CCT TAC AAT GGT GGT ACT GGC TAC AAC 240
Glu Trp Met Gly Tyr Ile Tyr Pro Tyr Asn Gly Gly Thr Gly Tyr Asn
    50                  55                  60

CAG AAG TTC AAG AGC AAG GCC ACA TTG ACT GTT GAC AAT TCC GCG AGC 288
Gln Lys Phe Lys Ser Lys Ala Thr Leu Thr Val Asp Asn Ser Ala Ser
65 70 75

ACA GCC TAC ATG GAG CTG AGC AGC CTG AGA TCT GAA GAC ACG GCT GTG 336
Thr Ala Tyr Met Glu Leu Ser Ser Leu Arg Ser Glu Asp Thr Ala Val
80 85 90

TAT TAC TGT GCG AGA AGT TAC TAT GCT ATG GAC TAC TGG GGC CAG GGA 384
Tyr Tyr Cys Ala Arg Ser Tyr Tyr Ala Met Asp Tyr Trp Gly Gln Gly
95 100 105

ACC CTG GTC ACC GTC TCC TCA GGG AGT GCA TCC GCC CCA ACC CTT TTC 432
Thr Leu Val Thr Val Ser Ser Gly Ser Ala Ser Ala Pro Thr Leu Phe
110 115 120 125

CCC CTC GTC TCC TGT GAG AAT TCC CCG TCG GAT ACG AGC AGC GTG GCC     480
Pro Leu Val Ser Cys Glu Asn Ser Pro Ser Asp Thr Ser Ser Val Ala
                130                 135                 140

GTT GGC TGC CTC GCA CAG GAC TTC CTT CCC GAC TCC ATC ACT TTC TCC     528
Val Gly Cys Leu Ala Gln Asp Phe Leu Pro Asp Ser Ile Thr Phe Ser
            145                 150                 155

TGG AAA TAC AAG AAC AAC TCT GAC ATC AGC AGT ACC CGG GGC TTC CCA     576
Trp Lys Tyr Lys Asn Asn Ser Asp Ile Ser Ser Thr Arg Gly Phe Pro
        160                 165                 170

TCA GTC CTG AGA GGG GGC AAG TAC GCA GCC ACC TCA CAG GTG CTG CTG     624
Ser Val Leu Arg Gly Gly Lys Tyr Ala Ala Thr Ser Gln Val Leu Leu
    175                 180                 185

CCT TCC AAG GAC GTC ATG CAG GGC ACA GAC GAA CAC GTG GTG TGC AAA     672
Pro Ser Lys Asp Val Met Gln Gly Thr Asp Glu His Val Val Cys Lys
190                 195                 200                 205

GTC CAG CAC CCC AAC GGC AAC AAA GAA AAG AAC GTG CCT CTT CCA GTG     720
Val Gln His Pro Asn Gly Asn Lys Glu Lys Asn Val Pro Leu Pro Val
                210                 215                 220

ATT GCC GAG CTG CCT CCC AAA GTG AGC GTC TTC GTC CCA CCC CGC GAC     768
Ile Ala Glu Leu Pro Pro Lys Val Ser Val Phe Val Pro Pro Arg Asp
            225                 230                 235

GGC TTC TTC GGC AAC CCC CGC AAG TCC AAG CTC ATC TGC CAG GCC ACG     816
Gly Phe Phe Gly Asn Pro Arg Lys Ser Lys Leu Ile Cys Gln Ala Thr
        240                 245                 250

GGT TTC AGT CCC CGG CAG ATT CAG GTG TCC TGG CTG CGC GAG GGG AAG     864
Gly Phe Ser Pro Arg Gln Ile Gln Val Ser Trp Leu Arg Glu Gly Lys
    255                 260                 265

CAG GTG GGG TCT GGC GTC ACC ACG GAC CAG GTG CAG GCT GAG GCC AAA     912
Gln Val Gly Ser Gly Val Thr Thr Asp Gln Val Gln Ala Glu Ala Lys
270                 275                 280                 285

GAG TCT GGG CCC ACG ACC TAC AAG GTG ACC AGC ACA CTG ACC ATC AAA     960
Glu Ser Gly Pro Thr Thr Tyr Lys Val Thr Ser Thr Leu Thr Ile Lys
                290                 295                 300

GAG AGC GAC TGG CTC AGC CAG AGC ATG TTC ACC TGC CGC GTG GAT CAC    1008
Glu Ser Asp Trp Leu Ser Gln Ser Met Phe Thr Cys Arg Val Asp His
            305                 310                 315

AGG GGC CTG ACC TTC CAG CAG AAT GCG TCC TCC ATG TGT GTC CCC GAT    1056
Arg Gly Leu Thr Phe Gln Gln Asn Ala Ser Ser Met Cys Val Pro Asp
        320                 325                 330

CAA GAC ACA GCC ATC CGG GTC TTC GCC ATC CCC CCA TCC TTT GCC AGC    1104
Gln Asp Thr Ala Ile Arg Val Phe Ala Ile Pro Pro Ser Phe Ala Ser
    335                 340                 345

ATC TTC CTC ACC AAG TCC ACC AAG TTG ACC TGC CTG GTC ACA GAC CTG 1152
Ile Phe Leu Thr Lys Ser Thr Lys Leu Thr Cys Leu Val Thr Asp Leu
350 355 360 365

ACC ACC TAT GAC AGC GTG ACC ATC TCC TGG ACC CGC CAG AAT GGC GAA 1200
Thr Thr Tyr Asp Ser Val Thr Ile Ser Trp Thr Arg Gln Asn Gly Glu
    370                 375                 380

GCT GTG AAA ACC CAC ACC AAC ATC TCC GAG AGC CAC CCC AAT GCC ACT 1248
Ala Val Lys Thr His Thr Asn Ile Ser Glu Ser His Pro Asn Ala Thr
385 390 395

TTC AGC GCC GTG GGT GAG GCC AGC ATC TGC GAG GAT GAC TGG AAT TCC 1296
Phe Ser Ala Val Gly Glu Ala Ser Ile Cys Glu Asp Asp Trp Asn Ser
400 405 410

GGG GAG AGG TTC ACG TGC ACC GTG ACC CAC ACA GAC CTG CCC TCG CCA 1344
Gly Glu Arg Phe Thr Cys Thr Val Thr His Thr Asp Leu Pro Ser Pro
415 420 425

CTG AAG CAG ACC ATC TCC CGG CCC AAG GGG GTG GCC CTG CAC AGG CCC 1392
Leu Lys Gln Thr Ile Ser Arg Pro Lys Gly Val Ala Leu His Arg Pro
430 435 440 445

GAT GTC TAC TTG CTG CCA CCA GCC CGG GAG CAG CTG AAC CTG CGG GAG 1440
Asp Val Tyr Leu Leu Pro Pro Ala Arg Glu Gln Leu Asn Leu Arg Glu
    450                 455                 460

TCG GCC ACC ATC ACG TGC CTG GTG ACG GGC TTC TCT CCC GCG GAC GTC 1488
Ser Ala Thr Ile Thr Cys Leu Val Thr Gly Phe Ser Pro Ala Asp Val
465 470 475

TTC GTG CAG TGG ATG CAG AGG GGG CAG CCC TTG TCC CCG GAG AAG TAT 1536
Phe Val Gln Trp Met Gln Arg Gly Gln Pro Leu Ser Pro Glu Lys Tyr
480 485 490

GTG ACC AGC GCC CCA ATG CCT GAG CCC CAG GCC CCA GGC CGG TAC TTC 1584
Val Thr Ser Ala Pro Met Pro Glu Pro Gln Ala Pro Gly Arg Tyr Phe
495 500 505

GCC CAC AGC ATC CTG ACC GTG TCC GAA GAG GAA TGG AAC ACG GGG GAG 1632
Ala His Ser Ile Leu Thr Val Ser Glu Glu Glu Trp Asn Thr Gly Glu
510 515 520 525

ACC TAC ATC TGC GTG GTG GCC CAT GAG GCC CTG CCC AAC AGG GTC ACC 1680
Thr Tyr Ile Cys Val Val Ala His Glu Ala Leu Pro Asn Arg Val Thr
    530                 535                 540

GAG AGG ACC GTG GAC AAG TCC ACC GGT AAA CCC ACC CTG TAC AAC GTG 1728
Glu Arg Thr Val Asp Lys Ser Thr Gly Lys Pro Thr Leu Tyr Asn Val
545 550 555

TCC CTG GTC ATG TCC GAC ACA GCT GGC ACC TGC TAC TGAT 1768
Ser Leu Val Met Ser Asp Thr Ala Gly Thr Cys Tyr
560 565

(2) INFORMATION FOR SEQ ID NO: 88:

(i) SEQUENCE CHARACTERISTICS:
(A) LENGTH: 588 amino acids
(B) TYPE: amino acid
(D) TOPOLOGY: linear

(ii) MOLECULE TYPE: protein
(xi) SEQUENCE DESCRIPTION: SEQ ID NO: 88:

Met Gly Trp Ser Trp Ile Phe Leu Phe Leu Leu Ser Gly Thr Ala Gly
-19 -15 -10 -5

Val His Ser Glu Val Gln Leu Val Gln Ser Gly Ala Glu Val Lys Lys
1 5 10

Pro Gly Ala Ser Val Lys Val Ser Cys Lys Ala Ser Gly Tyr Thr Phe
15 20 25

Thr Asp Tyr Asn Met His Trp Val Lys Gln Ala His Gly Lys Ser Leu
30 35 40 45

Glu Trp Met Gly Tyr Ile Tyr Pro Tyr Asn Gly Gly Thr Gly Tyr Asn
    50                  55                  60

Gln Lys Phe Lys Ser Lys Ala Thr Leu Thr Val Asp Asn Ser Ala Ser
65 70 75

Thr Ala Tyr Met Glu Leu Ser Ser Leu Arg Ser Glu Asp Thr Ala Val
80 85 90

Tyr Tyr Cys Ala Arg Ser Tyr Tyr Ala Met Asp Tyr Trp Gly Gln Gly
95 100 105

Thr Leu Val Thr Val Ser Ser Gly Ser Ala Ser Ala Pro Thr Leu Phe
110 115 120 125

Pro Leu Val Ser Cys Glu Asn Ser Pro Ser Asp Thr Ser Ser Val Ala
    130                 135                 140

Val Gly Cys Leu Ala Gln Asp Phe Leu Pro Asp Ser Ile Thr Phe Ser
145 150 155

Trp Lys Tyr Lys Asn Asn Ser Asp Ile Ser Ser Thr Arg Gly Phe Pro
160 165 170

Ser Val Leu Arg Gly Gly Lys Tyr Ala Ala Thr Ser Gln Val Leu Leu
175 180 185

Pro Ser Lys Asp Val Met Gln Gly Thr Asp Glu His Val Val Cys Lys
190 195 200 205

Val Gln His Pro Asn Gly Asn Lys Glu Lys Asn Val Pro Leu Pro Val
    210                 215                 220

Ile Ala Glu Leu Pro Pro Lys Val Ser Val Phe Val Pro Pro Arg Asp
225 230 235

Gly Phe Phe Gly Asn Pro Arg Lys Ser Lys Leu Ile Cys Gln Ala Thr
        240                 245                 250

Gly Phe Ser Pro Arg Gln Ile Gln Val Ser Trp Leu Arg Glu Gly Lys
    255                 260                 265

Gln Val Gly Ser Gly Val Thr Thr Asp Gln Val Gln Ala Glu Ala Lys
270                 275                 280                 285

Glu Ser Gly Pro Thr Thr Tyr Lys Val Thr Ser Thr Leu Thr Ile Lys
                290                 295                 300

Glu Ser Asp Trp Leu Ser Gln Ser Met Phe Thr Cys Arg Val Asp His
            305                 310                 315

Arg Gly Leu Thr Phe Gln Gln Asn Ala Ser Ser Met Cys Val Pro Asp
        320                 325                 330

Gln Asp Thr Ala Ile Arg Val Phe Ala Ile Pro Pro Ser Phe Ala Ser
    335                 340                 345

Ile Phe Leu Thr Lys Ser Thr Lys Leu Thr Cys Leu Val Thr Asp Leu
350                 355                 360                 365

Thr Thr Tyr Asp Ser Val Thr Ile Ser Trp Thr Arg Gln Asn Gly Glu
                370                 375                 380

Ala Val Lys Thr His Thr Asn Ile Ser Glu Ser His Pro Asn Ala Thr
            385                 390                 395

Phe Ser Ala Val Gly Glu Ala Ser Ile Cys Glu Asp Asp Trp Asn Ser
        400                 405                 410

Gly Glu Arg Phe Thr Cys Thr Val Thr His Thr Asp Leu Pro Ser Pro
    415                 420                 425

Leu Lys Gln Thr Ile Ser Arg Pro Lys Gly Val Ala Leu His Arg Pro
430                 435                 440                 445

Asp Val Tyr Leu Leu Pro Pro Ala Arg Glu Gln Leu Asn Leu Arg Glu
                450                 455                 460

Ser Ala Thr Ile Thr Cys Leu Val Thr Gly Phe Ser Pro Ala Asp Val
            465                 470                 475

Phe Val Gln Trp Met Gln Arg Gly Gln Pro Leu Ser Pro Glu Lys Tyr
        480                 485                 490

Val Thr Ser Ala Pro Met Pro Glu Pro Gln Ala Pro Gly Arg Tyr Phe
    495                 500                 505

Ala His Ser Ile Leu Thr Val Ser Glu Glu Glu Trp Asn Thr Gly Glu
510                 515                 520                 525

Thr Tyr Ile Cys Val Val Ala His Glu Ala Leu Pro Asn Arg Val Thr
                530                 535                 540

Glu Arg Thr Val Asp Lys Ser Thr Gly Lys Pro Thr Leu Tyr Asn Val
            545                 550                 555

Ser Leu Val Met Ser Asp Thr Ala Gly Thr Cys Tyr
    .   560                 565

(2) INFORMATION FOR SEQ ID NO: 89:

        (i) SEQUENCE CHARACTERISTICS:
            (A) LENGTH: 116 amino acids
            (B) TYPE: amino acid
            (D) TOPOLOGY: linear

    (ii) MOLECULE TYPE: protein
    (xi) SEQUENCE DESCRIPTION: SEQ ID NO: 89:

Glu Val Gln Leu Gln Gln Ser Gly Pro Glu Leu Val Lys Pro Gly Ala
  1               5                  10                  15

Ser Val Lys Ile Ser Cys Lys Ala Ser Gly Tyr Thr Phe Thr Asp Tyr
             20                  25                  30

Asn Met His Trp Val Lys Gln Ser His Gly Lys Ser Leu Glu Trp Ile
         35                  40                  45

Gly Tyr Ile Tyr Pro Tyr Asn Gly Gly Thr Gly Tyr Asn Gln Lys Phe
     50                  55                  60

Lys Ser Lys Ala Thr Leu Thr Val Asp Asn Ser Ser Ser Thr Ala Tyr
 65                  70                  75                  80

Met Glu Leu Arg Ser Leu Thr Ser Glu Asp Ser Ala Val Tyr Tyr Cys
                 85                  90                  95

Ala Arg Ser Tyr Tyr Ala Met Asp Tyr Trp Gly Gln Gly Thr Ser Val
            100                 105                 110

Thr Val Ser Ser
        115

(2) INFORMATION FOR SEQ ID NO: 90:

      (i) SEQUENCE CHARACTERISTICS:
            (A) LENGTH: 34 base pairs
            (B) TYPE: nucleic acid
            (C) STRANDEDNESS: single
            (D) TOPOLOGY: linear

    (ii) MOLECULE TYPE: other nucleic acid
            (A) DESCRIPTION:   /desc = "synthetic DNA"

   (iii) HYPOTHETICAL: NO

    (iv) ANTI-SENSE: NO

(xi) SEQUENCE DESCRIPTION: SEQ ID NO: 90:

GGGAATTCAT GGACTGGACC TGGAGGWTCC TYTT 34

(2) INFORMATION FOR SEQ ID NO: 91:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 91:

CCTCTAGAGG TTAGTTTGCA TGCACACACA GA 32

(2) INFORMATION FOR SEQ ID NO: 92:

(i) SEQUENCE CHARACTERISTICS:
(A) LENGTH: 112 amino acids
(B) TYPE: amino acid
(D) TOPOLOGY: linear

(ii) MOLECULE TYPE: protein
(xi) SEQUENCE DESCRIPTION: SEQ ID NO: 92:

Asp Val Val Met Thr Gln Ser Pro Leu Ser Leu Pro Val Ser Leu Gly
1               5                   10                  15

Asp Gln Ala Ser Ile Ser Cys Arg Ser Ser Lys Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Ala Phe Gly Gly Gly Thr Lys Leu Glu Ile Lys
            100                 105                 110

(2) INFORMATION FOR SEQ ID NO: 93:

     (i) SEQUENCE CHARACTERISTICS:
          (A) LENGTH: 34 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "synthetic DNA"

   (iii) HYPOTHETICAL: NO

    (iv) ANTI-SENSE: NO

    (xi) SEQUENCE DESCRIPTION: SEQ ID NO: 93:

GCGAATTCTG CCTTGACTGA TCAGAGTTTC CTCA                                34

(2) INFORMATION FOR SEQ ID NO: 94:

     (i) SEQUENCE CHARACTERISTICS:
          (A) LENGTH: 32 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "synthetic DNA"

   (iii) HYPOTHETICAL: NO

    (iv) ANTI-SENSE: NO

    (xi) SEQUENCE DESCRIPTION: SEQ ID NO: 94:

GCTCTAGATG AGGTGAAAGA TGAGCTGGAG GA                                  32

(2) INFORMATION FOR SEQ ID NO: 95:

     (i) SEQUENCE CHARACTERISTICS:
          (A) LENGTH: 20 base pairs
          (B) TYPE: nucleic acid
          (C) STRANDEDNESS: single
          (D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 95:

CCTCGTCTCC TGTGAGAATT 20

(2) INFORMATION FOR SEQ ID NO: 96:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 96:

ACTCTGACAT CAGCAGTACC 20

(2) INFORMATION FOR SEQ ID NO: 97:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 97:

ACGAACACGT GGTGTGCAAA 20

(2) INFORMATION FOR SEQ ID NO: 98:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 98:

AAGTCCAAGC TCATCTGCCA 20

(2) INFORMATION FOR SEQ ID NO: 99:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 99:

TACAAGGTGA CCAGCACACT 20

(2) INFORMATION FOR SEQ ID NO: 100:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 100:

AATGCGTCCT CCATGTGTGT 20

(2) INFORMATION FOR SEQ ID NO: 101:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 101:

AGACCTGACC ACCTATGACA 20

(2) INFORMATION FOR SEQ ID NO: 102:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 102:

TCTGCGAGGA TGACTGGAAT 20

(2) INFORMATION FOR SEQ ID NO: 103:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 103:

ATGTCTACTT GCTGCCACCA 20

(2) INFORMATION FOR SEQ ID NO: 104:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 104:

TTGTCCCCGG AGAAGTATGT 20

(2) INFORMATION FOR SEQ ID NO: 105:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 105:

GTGTCCGAAG AGGAATGGAA 20

(2) INFORMATION FOR SEQ ID NO: 106:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 106:

CTCAGTGAAG GTTTCCTGCA 20

(2) INFORMATION FOR SEQ ID NO: 107:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 107:

AAAGGCTTGA GTGGATGGGA 20

(2) INFORMATION FOR SEQ ID NO: 108:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 108:

TGAGCAGCCT GAGATCTGAA 20

(2) INFORMATION FOR SEQ ID NO: 109:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 109:

GGTACTGCTG ATGTCAGAGT 20

(2) INFORMATION FOR SEQ ID NO: 110:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 110:

AATCACTGGA AGAGGCACGT 20

(2) INFORMATION FOR SEQ ID NO: 111:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 111:

TGGCAGATGA GCTTGGACTT 20

(2) INFORMATION FOR SEQ ID NO: 112:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 112:

AGCCAGTCGC TCTCTTTGAT 20

(2) INFORMATION FOR SEQ ID NO: 113:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 113:

AGGAAGATGC TGGCAAAGGA 20

(2) INFORMATION FOR SEQ ID NO: 114:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 114:

TGGTGTGGGT TTTCACAGCT 20

(2) INFORMATION FOR SEQ ID NO: 115:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 115:

TTCCAGTCAT CCTCGCAGAT 20

(2) INFORMATION FOR SEQ ID NO: 116:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 116:

TGGTGGCAGC AAGTAGACAT 20

(2) INFORMATION FOR SEQ ID NO: 117:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 117:

ACATACTTCT CCGGGGACAA 20

(2) INFORMATION FOR SEQ ID NO: 118:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 118:

GTGTTCCATT CCTCTTCGGA 20

(2) INFORMATION FOR SEQ ID NO: 119:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 119:

TTTACCGGTG GACTTGTCCA 20

(2) INFORMATION FOR SEQ ID NO: 120:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 120:

TATCCAGAAG CCTTGCAGGA 20

(2) INFORMATION FOR SEQ ID NO: 121:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 121:

TGTGTCCCTG GTAATGGTGA 20

(2) INFORMATION FOR SEQ ID NO: 122:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 122:

CTCGCACAGT AATACCACGC 20

(2) INFORMATION FOR SEQ ID NO: 123:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 123:

TATCCGACGG GGAATTCTCA 20

(2) INFORMATION FOR SEQ ID NO: 124:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 124:

TGTCTTCATC TTCCCGCCAT 20

(2) INFORMATION FOR SEQ ID NO: 125:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 125:

ACGCTGAGCA AAGCAGACTA 20

(2) INFORMATION FOR SEQ ID NO: 126:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 126:

TCCAGTGGGG ATGTTGTGAT 20

(2) INFORMATION FOR SEQ ID NO: 127:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 127:

AGTGGGTCAG GCACTGATTT 20

(2) INFORMATION FOR SEQ ID NO: 128:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 128:

TCTCCTGCAG GTCTAGTCAA 20

(2) INFORMATION FOR SEQ ID NO: 129:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 129:

GGGTAACTCC CAGGAGAGTG 20

(2) INFORMATION FOR SEQ ID NO: 130:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 130:

AGGGACCAAG GTGGAAATCA 20

(2) INFORMATION FOR SEQ ID NO: 131:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 131:

TACTTTGGCC TCTCTGTGAT 20

(2) INFORMATION FOR SEQ ID NO: 132:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 132:

ACTTCGCAGG CGTAGACTTT 20

(2) INFORMATION FOR SEQ ID NO: 133:

(i) SEQUENCE CHARACTERISTICS:
(A) LENGTH: 20 base pairs

(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 133:

TCTCCCCTGT TGAAGCTCTT 20

(2) INFORMATION FOR SEQ ID NO: 134:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 134:

TTAAAGCCAA GGAGGAGGAG 20

(2) INFORMATION FOR SEQ ID NO: 135:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 135:

CTCCACCCTG CTGATTTTCA 20

(2) INFORMATION FOR SEQ ID NO: 136:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 136:

TGCAGCCACA GTACGTTTGA 20

(2) INFORMATION FOR SEQ ID NO: 137:

(i) SEQUENCE CHARACTERISTICS:
(A) LENGTH: 1869 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens

(xi) SEQUENCE DESCRIPTION: SEQ ID NO: 137:

ATGGACTGGA CCTGGAGGAT CCTCTTTTTG GTGGCAGCAG CCACAGGTGC CCACTCCCAG 60

GTCCAACTTG TGCAGTCTGG GGCTGAGGTG AAGAAGCCTG GGGCCTCAGT GAAGGTTTCC 120

TGCAAGGCTT CTGGATACAC CTTCACTACC TATGCTATGC ATTGGGTGCG CCAGGCCCCC 180

GGACAAAGGC TTGAGTGGAT GGGATGGATC AACGCTGGCA ATGGTAACAC AAAATATTCA 240

CAGAAGTTCC AGGGCAGAGT CACCATTACC AGGGACACAT CCGCGAGCAC AGCCTACATG 300

GAGCTGAGCA GCCTGAGATC TGAAGACACG GCTGTGTATT ACTGTGCGAG AGGCGAGGAG 360

ATGGGAGCTA CTTCAGGTCC CGGGCGGTAC TACTTTGACT ACTGGGGCCA GGGAACCCTG 420

GTCACCGTCT CCTCAGGGAG TGCATCCGCC CCAACCCTTT TCCCCCTCGT CTCCTGTGAG 480

AATTCCCCGT CGGATACGAG CAGCGTGGCC GTTGGCTGCC TCGCACAGGA CTTCCTTCCC 540

GACTCCATCA CTTTCTCCTG GAAATACAAG AACAACTCTG ACATCAGCAG CACCCGGGGC 600

TTCCCATCAG TCCTGAGAGG GGGCAAGTAC GCAGCCACCT CACAGGTGCT GCTGCCTTCC 660

AAGGACGTCA TGCAGGGCAC AGACGAACAC GTGGTGTGCA AAGTCCAGCA CCCCAACGGC 720

AACAAAGAAA AGAACGTGCC TCTTCCAGTG ATTGCTGAGC TGCCTCCCAA AGTGAGCGTC 780

TTCGTCCCAC CCCGCGACGG CTTCTTCGGC AACCCCCGCA AGTCCAAGCT CATCTGCCAG 840

GCCACGGGTT TCAGTCCCCG GCAGATTCAG GTGTCCTGGC TGCGCGAGGG GAAGCAGGTG 900

GGGTCTGGCG TCACCACGGA CCAGGTGCAG GCTGAGGCCA AAGAGTCTGG GCCCACGACC 960

TACAAGGTGA CCAGCACACT GACCATCAAA GAGAGCGACT GGCTCAGCCA GAGCATGTTC 1020

ACCTGCCGCG TGGATCACAG GGGCCTGACC TTCCAGCAGA ATGCGTCCTC CATGTGTGTC 1080

CCCGATCAAG ACACAGCCAT CCGGGTCTTC GCCATCCCCC CATCCTTTGC CAGCATCTTC 1140

CTCACCAAGT CCACCAAGTT GACCTGCCTG GTCACAGACC TGACCACCTA TGACAGCGTG 1200

ACCATCTCCT GGACCCGCCA GAATGGCGAA GCTGTGAAAA CCCACACCAA CATCTCCGAG 1260

AGCCACCCCA ATGCCACTTT CAGCGCCGTG GGTGAGGCCA GCATCTGCGA GGATGACTGG 1320

AATTCCGGGG AGAGGTTCAC GTGCACCGTG ACCCACACAG ACCTGCCCTC GCCACTGAAG 1380

CAGACCATCT CCCGGCCCAA GGGGGTGGCC CTGCACAGGC CCGATGTCTA CTTGCTGCCA 1440

CCAGCCCGGG AGCAGCTGAA CCTGCGGGAG TCGGCCACCA TCACGTGCCT GGTGACGGGC 1500

TTCTCTCCCG CGGACGTCTT CGTGCAGTGG ATGCAGAGGG GGCAGCCCTT GTCCCCGGAG 1560

AAGTATGTGA CCAGCGCCCC AATGCCTGAG CCCCAGGCCC CAGGCCGGTA CTTCGCCCAC 1620

AGCATCCTGA CCGTGTCCGA AGAGGAATGG AACACGGGGG AGACCTACAT CTGCGTGGTG 1680

GCCCATGAGG CCCTGCCCAA CAGGGTCACC GAGAGGACCG TGGACAAGTC CACCGGTAAA 1740

CCCACCCTGT ACAACGTGTC CCTGGTCATG TCCGACACAG CTGGCACCTG CTACTGACCC 1800

TGCTGGCCTG CCCACAGGCT CGGGGCGGCT GGCCGCTCTG TGTGTGCATG CAAACTAACC 1860

CGTGTCAAC 1869

(2) INFORMATION FOR SEQ ID NO: 138:

(i) SEQUENCE CHARACTERISTICS:
(A) LENGTH: 891 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens

(xi) SEQUENCE DESCRIPTION: SEQ ID NO: 138:

TGCCTTGACT GATCAGGACT CCTCAGTTCA CCTTCTCACA ATGAGGCTCC CTGCTCAGCT 60

CCTGGGGCTG CTAATGCTCT GGGTCCCAGG ATCCAGTGGG GATGTTGTGA TGACTCAGTC 120

TCCACTCTCC CTGCCCGTCA TCCCTGGACA GCCGGCCTCC ATCTCCTGCA GCTCTAGTCA 180

AGGCCTCGTA TTCAGTGATG GAAACACCTA CGTGAATTGG TTTCATCAGA GGCCAGGCCA 240

ACCTCCAAGG CGCCTAATTT ATGAGGTTTC TCACCGGGAC TCTGGGGTCC CAGACAGATT 300

CAGCGGCAGT GGGTCAGGCA CTGATTTCAC ACTGAAAATC AGCAGGGTGG AGGCTGAGGA 360

TGTTGGGGTT TATTACTGCA TGCAAGGTAC ACAGTGGCCG TGGACGTTCG GCCAAGGGAC 420

GAAGGTGGAA ACCAAACGAA CTGTGGCTGC ACCATCTGTC TTCATCTTCC CGCCATCTGA 480

TGAGCAGTTG AAATCTGGAA CTGCCTCTGT TGTGTGCCTG CTGAATAACT TCTATCCCAG 540

AGAGGCCAAA GTACAGTGGA AAGTGGATAA CGCCCTCCAA TCGGGTAACT CCCAGGAGAG 600

TGTCACAGAG CAGGACAGCA AGGACAGCAC CTACAGCCTC AGCAGCACCC TGACGCTGAG 660

CAAAGCAGAC TACGAGAAAC ACAAACTCTA CGCCTGCGAA GTCACCCATC AGGGCCTGAG 720

CTCGCCCGTC ACAAAGAGCT TCAACAGGGG AGAGTGTTAG AGGGAGAAGT GCCCCCACCT 780

GCTCCTCAGT TCCAGCCTGA CCCCCTCCCA TCCTTTGGCC TCTGACCCTT TTTCCACAGG 840

GGACCTACCC CTATTGCGGT CCTCCAGCTC ATCTTTCACC TCATCTAGAG C 891

(2) INFORMATION FOR SEQ ID NO: 139:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 139:

AGCCGGCCTC CATCTCCTGC AGATCTAGTA AGAGCCTTGT 40

(2) INFORMATION FOR SEQ ID NO: 140:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 140:

ACAAGGCTCT TACTAGATCT GCAGGAGATG GAGGCCGGCT 40

(2) INFORMATION FOR SEQ ID NO: 141:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 141:

AAGTTTCCAA CCGATTTTCT GGGGTCCCAG ACAGATTCAG 40

(2) INFORMATION FOR SEQ ID NO: 142:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 142:

CTGAATCTGT CTGGGACCCC AGAAAATCGG TTGGAAACTT 40

(2) INFORMATION FOR SEQ ID NO: 143:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 143:

GGCTGAGGAT GTTGGGGTTT ATTACTGCTC TCAAAGTACA CATGTTCCTC 50

(2) INFORMATION FOR SEQ ID NO: 144:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 144:

GAGGAACATG TGTACTTTGA GAGCAGTAAT AAACCCCAAC ATCCTCAGCC 50

(2) INFORMATION FOR SEQ ID NO: 145:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 145:

GGCTGAGGAT GTTGGGGTTT ATTTCTGCTC TCAAAGTACA CATGTTCCTC 50

(2) INFORMATION FOR SEQ ID NO: 146:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 146:

GAGGAACATG TGTACTTTGA GAGCAGAAAT AAACCCCAAC ATCCTCAGCC 50

(2) INFORMATION FOR SEQ ID NO: 147:

(i) SEQUENCE CHARACTERISTICS:
(A) LENGTH: 52 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 147:

CTCAAAGTAC ACATGTTCCT CCGGCGTTCG GCCAAGGGAC CAAGGTGGAA AT 52

(2) INFORMATION FOR SEQ ID NO: 148:

(i) SEQUENCE CHARACTERISTICS:
(A) LENGTH: 52 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 148:

ATTTCCACCT TGGTCCCTTG GCCGAACGCC GGAGGAACAT GTGTACTTTG AG 52

(2) INFORMATION FOR SEQ ID NO: 149:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 149:

GGGCTCGAGT GCCTTGACTG ATCAGGACTC CTCAGTTCAC 40

(2) INFORMATION FOR SEQ ID NO: 150:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 150:

GGCCAGTCTC CAAGGCTCCT GATCTACAAA G 31

(2) INFORMATION FOR SEQ ID NO: 151:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 151:

CTTTGTAGAT CAGGAGCCTT GGAGACTGGC C 31

(2) INFORMATION FOR SEQ ID NO: 152:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 152:

CCCTCTAGAC TAACACTCTC CCCTGTTGAA G 31

(2) INFORMATION FOR SEQ ID NO: 153:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 153:

CTGCTCTAAA AGCTGCGGAA 20

(2) INFORMATION FOR SEQ ID NO: 154:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 154:

TAGATCTGCA GGAGARGGAG 20

(2) INFORMATION FOR SEQ ID NO: 155:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 155:

TATGTTTCAG GTTCAGGGGG 20

(2) INFORMATION FOR SEQ ID NO: 156:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 156:

GGGCTCGAGC TAAGGGAATT CCGCCTCTCC TCAGACACTG 40

(2) INFORMATION FOR SEQ ID NO: 157:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 157:

GAACTGCAGG CGTCCACTCT GAGGTGCAGC TTGTGCAGTC 40

(2) INFORMATION FOR SEQ ID NO: 158:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 158:

GACTGCACAA GCTGCACCTC AGAGTGGACG CCTGCAGTTC 40

(2) INFORMATION FOR SEQ ID NO: 159:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 159:

AATATGCATA AATTCGAATG GATGGGATAT ATTTATCCTT ACAATGGTGG 50

(2) INFORMATION FOR SEQ ID NO: 160:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 160:

CATCCATTCG AATTTATGCA TATTATAGTC AGTGAAGGTG TATCCAGAAG 50

(2) INFORMATION FOR SEQ ID NO: 161:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 161:

CCACATTGAC TGTTGACAAT TCCGCGAGCA CAGCCTACAT 40

(2) INFORMATION FOR SEQ ID NO: 162:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 162:

ATGTAGGCTG TGCTCGCGGA ATTGTCAACA GTCAATGTGG 40

(2) INFORMATION FOR SEQ ID NO: 163:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 163:

GAAGTTACTA TGCTATGGAC TACTGGGGCC AGGGAACCCT 40

(2) INFORMATION FOR SEQ ID NO: 164:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 164:

TAGTCCATAG CATAGTAACT TCTCGCACAG TAATACACAG 40

(2) INFORMATION FOR SEQ ID NO: 165:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 165:

GGGCTCGAGG CCAAAGAGTC TGGGCCCACG ACCTACAAG 39

(2) INFORMATION FOR SEQ ID NO: 166:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 166:

CTTGTAGGTC GTGGGCCCAG ACTCTTTGGC 30

(2) INFORMATION FOR SEQ ID NO: 167:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 167:

GGGTCTAGAT CAGTAGCAGG TGCCAGCTGT G 31

(2) INFORMATION FOR SEQ ID NO: 168:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 168:

TATGCATTGG GTGCGCCAGG CCCCCGGACA AGGACTCGAA TGGATGGGAT ATATTTATCC 60

(2) INFORMATION FOR SEQ ID NO: 169:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 169:

CGAGTCCTTG TCCGGGGGCC TGGCGCACCC AATGCATATT ATAGTCAGTG AAGGTGTATC 60

(2) INFORMATION FOR SEQ ID NO: 170:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 170:

TATGCATTGG GTGAAGCAGG CCCATGGAAA GAGCCTCGAA TGGATGGGAT ATATTTATCC 60

(2) INFORMATION FOR SEQ ID NO: 171:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 171:

CGAGGCTCTT TCCATGGGCC TGCTTCACCC AATGCATATT ATAGTCAGTG AAGGTGTATC 60

(2) INFORMATION FOR SEQ ID NO: 172:

(i) SEQUENCE CHARACTERISTICS:
(A) LENGTH: 30 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 172:

GAGCGACTGG CTCAGCCAGA GCATGTTCAC 30

(2) INFORMATION FOR SEQ ID NO: 173:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 173:

GTGAACATGC TCTGGCTGAG CCAGTCGCTC 30

(2) INFORMATION FOR SEQ ID NO: 174:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 174:

ACCTACATCT GCGTGGTGGC CCATGAGGCC CTGCCC 36

(2) INFORMATION FOR SEQ ID NO: 175:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 175:

GGCCTCATGG GCCACCACGC AGATGTAGGT CTCCCCCGTG TTCCATTCCT 50

(2) INFORMATION FOR SEQ ID NO: 176:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 176:

GCTTTATTTG TAACCATTAT AAGCTG 26

(2) INFORMATION FOR SEQ ID NO: 177:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 177:

CTAGATCAGT AGCAGGTGCC AGCTGTGTCG 30

(2) INFORMATION FOR SEQ ID NO: 178:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 178:

CATAGTAACT TCTCGCACAG TAAT 24

(2) INFORMATION FOR SEQ ID NO: 179:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 179:

GATACACCTT CACTGACTAT AAT 23

(2) INFORMATION FOR SEQ ID NO: 180:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 180:

@"z—ñ
CGTCGGATAC GAGCAGCGTG 20

(2) INFORMATION FOR SEQ ID NO: 181:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 181:

CACCCCGCGA CGGCTTCTT 19

(2) INFORMATION FOR SEQ ID NO: 182:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 182:

GGATCACAGG GGCCTGACCT 20

(2) INFORMATION FOR SEQ ID NO: 183:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 183:

CTGTGAAAAC CCACACCAAC 20

(2) INFORMATION FOR SEQ ID NO: 184:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 184:

GCTGAACCTG CGGGAGTCGG 20

(2) INFORMATION FOR SEQ ID NO: 185:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 185:

GTGGCCCATG AGGCCCTGCC 20

(2) INFORMATION FOR SEQ ID NO: 186:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 186:

GGGGAATTCC AGTACGGAGT TGGGGAAGAA GCTCTTT 37

(2) INFORMATION FOR SEQ ID NO: 187:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 187:

GTTTCTTCTG CCTCTGTCAC CAAGTTAGAT CTGGA 35

(2) INFORMATION FOR SEQ ID NO: 188:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 188:

TCCAGATCTA ACTTGGTGAC AGAGGCAGAA GAAAC 35

(2) INFORMATION FOR SEQ ID NO: 189:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: NO

(iv) ANTI-SENSE: NO

(xi) SEQUENCE DESCRIPTION: SEQ ID NO: 189:

CCCTCTAGAC GGGTCACGTG GGCATCAC 28

## Description

The present invention relates to humanised anti-human Fas antibodies which recognise the Fas antigen as well as DNA encoding such antibodies. The present invention further relates to pharmaceutical preparations containing such antibodies for the treatment of disease, including particularly autoimmune diseases and rheumatic diseases, the preparations optionally further containing a cell growth inhibitor. In addition, the invention relates to improved methods for the production of humanised antibodies.

Immunoglobulin G (IgG) is composed of two light polypeptide chains (L chains) each having a molecular weight of about 23,000 kD and two heavy polypeptide chains (H chains) each having a molecular weight of about 50,000 kD. Both H and L chains consist of a repeated region of conserved amino acids consisting of about 110 residues. This region is referred to herein as a "domain", and constitutes the basic three-dimensional structural unit of the IgG. The H and L chains consist of four and two consecutive domains, respectively.

When antibody amino acid sequences are compared, the amino-terminal domain of both H and L chains is found to be more variable than the other domains. It is, therefore, referred to as the `variable' domain (V domain). The V domains of H and L chains associate with each other by their complementary nature to form variable regions in the amino-termini of IgG molecules. The other domains associate to form constant regions. The constant region sequences are characteristic for a given species. For example, the constant regions of mouse IgG differ from those of human IgG, and a mouse IgG molecule is recognised as a foreign protein by the human immune system. Administration of a mouse IgG molecule into a human subject results in the production of a human anti-mouse antibody (hereinafter referred to as "HAMA") response [Schroff *et al.,* (1985), Cancer Res., *45,* 879-885]. Accordingly, a mouse antibody cannot be repeatedly administered to a human subject. For effective administration, the antibody must be modified to avoid inducing the HAMA response, but still maintaining the antibody specificity.

Data from X-ray crystallography analysis indicates that the immunoglobulin fold generally forms a long cylindrical structure comprising two layers of antiparallel β-sheets, each consisting of three or four β-chains. In a variable region, three loops from each of the V domains of H and L chains cluster together to form an antigen-binding site Each of these loops is termed a complementarity determining region ("CDR"). The CDR's have the highest variability in amino acid sequence. The portions of the variable region that are not part of a CDR are called "framework regions" ("FR" regions) and generally play a role in maintaining the structure of CDR's.

Kabat and co-workers compared the primary sequences of a number of variable regions of H and L chains and identified putative CDRs or framework regions, based on sequence conservation [E. A. Kabat *et al.,* Sequences of proteins of immunological interest 5th edition, NIH Publication, No.91-3242]. Further, they classified the framework regions into several subgroups which share common amino acid sequences. They also identified framework regions that correspond between mouse and human sequences.

Studies on the structural characteristics of IgG molecules have led to the development of methods for preparing humanised antibodies, which do not provoke a HAMA response, as described below.

Initial suggestions were directed towards the preparation of a chimaeric antibody, by joining the variable region of a mouse antibody to the constant regions of human origin [Morrison, S. L., *et al.,* (1984), Proc. Natl. Acad. Sci. USA *81*, p6851-6855]. Such a chimaeric antibody, however, still contains many non-human amino acid residues, and thus can cause a HAMA response, especially when administered for a prolonged period. [Regent *et al*., (1990), Br. J. Cancer, *62*, p487 *et seq*.].

The grafting of CDR segments alone into a human antibody was then proposed, in order to further reduce the number of non-human amino acid sequences which cause the HAMA response [Jones, P. T. *et al.,* (1986), Nature, *321,* 522-515]. However, the grafting of the CDR portions alone was generally found to be insufficient to maintain the activity of the immunoglobulin against an antigen.

Based on data from X-ray crystallography, Chothia and co-workers [Chothia *et al*., (1987), J. Mol. Biol., *196*, 901-917] determined that:
1) A CDR has a region involved in antigen binding and a region involved in maintaining the structure of the CDR itself. Possible three-dimensional structures for CDRs can be classified into several classes with characteristic patterns (canonical structures); and
2) The classes of canonical structures are determined not only by the CDR sequences but also by the nature of amino acids in specific positions in the framework regions.

As a result, it has been suggested that the CDR-grafting technique should also involve the grafting of certain amino acid residues from the framework regions into the human antibody backbone [Queen *et al.,* Japanese Provisional Patent Publication No. 4-502408].

In the context of the above, an antibody from a non-human mammal from which the CDR's are obtained for grafting is hereinafter termed a 'donor' molecule. A human antibody into which the CDRs are grafted is hereinafter termed an 'acceptor' molecule.

In performing CDR-grafting, the structures of the CDR region should ideally be conserved and the activity of the immunoglobulin molecule should be maintained. The following factors may, therefore, be relevant:
1) the subgroup of the acceptor; and
2) the nature of the amino acid residues that are transferred from the framework regions of the donor.

Queen and co-workers [Queen *et al.,* Japanese Provisional Patent Publication No. 4-502408] have proposed a method for humanising antibodies, in which an amino acid residue from a framework region of a donor is grafted along with the CDR sequence into an acceptor molecule, provided that the residue is close to a CDR, or the amino acid in the framework region of the acceptor is rarely found at that position in the acceptor, whereas the corresponding amino acid in the donor is commonly found at that position in the acceptor.

Immunoglobulin M ("lgM") is normally composed of ten H chains and ten L chains, along with a joining chain ("J chain") located in the centre of the molecule. Mouse IgM has constant regions, like IgG and, thus, cannot be repeatedly administered to a human subject Therefore, CDR grafting is necessary if IgM molecules are to be used as pharmaceutical agents in humans.

Although an IgM molecule is normally present as a pentamer with a J chain, it can also be present as a hexamer lacking the J chain [Troy, *et al.,* J. Biol. Chem., (1992), *26*⁻, (25), 18002-18007]. The complement-binding activity is reportedly enhanced in such an IgM hexamer lacking the J chain [Davis, *et al.,* Eur. J. Immunol., (1988) *18*, 1001-1008]. However, the presence of a J chain has previously been thought to be essential for the maintenance of IgM structure and for the molecule to retain its immunoglobulin activity. At present, it is not known if an IgM molecule lacking a J chain retains its original activity.

The physiological death of cells in a living organism in the natural course of events is known as apoptosis, and is distinguished from the pathological death of cells, i.e. necrosis [*c.f* Kerr *et al.,* (1972), Br. J. Cancer, *26*, 239 *et seq.*]. Apoptosis is an example of programmed cell death, which is where certain cells are programmed, in advance, to die in a living organism in the natural course of events, such as when the cell in question has performed a pre-determined function. Apoptosis is characterised by such morphological changes as curved cell surface, condensed nuclear chromatin and fragmented chromosomal DNA, amongst others.

Apoptosis has an important role to play in disposing of cells that recognise autoantigen during the process of T and B lymphocyte differentiation. Onset of so-called autoimmune diseases is generally brought on by the appearance of auto-reactive lymphocytes resulting from the failure of apoptosis during lymphocyte differentiation [*c.f.* Keiichi Nakayama *et al.,* (1995), Mebio *12(10)*, 79-86].

Fas is a cell membrane molecule involved in the apoptosis of immunocompetent cells [Itoh, N., *et al*., *infra*]. Murine monoclonal antibodies have been generated to the human Fas antigen [Yonehara, S., *et al.,* (1989), J. Exp. Med., *169*, 1747]. These anti-human Fas antibodies have apoptosis-inducing cytotoxic activity in human cells and have been proposed as potential therapeutic agents in the treatment of autoimmune diseases. AIDS and tumours [Japanese Provisional Patent Publications Nos. 2-237935 and 5-503281].

Rheumatism, especially rheumatoid arthritis, is believed to result from the proliferation of synoviocytes, accompanied by a variety of immunological abnormalities. The proliferation of synoviocytes is typically accompanied by inflammatory cellular infiltration and erosion of bone. Tissue erosion around the affected joint in chronic rheumatoid arthritis is apparently caused by abnormal production of cytokines from inflammatory synoviocytes. Examination of joints in patients with rheumatism reveals abnormal proliferation of synoviocytes, hyperplasia of synovial villi, multi-layered synoviocytes, etc. [*c.f.* Daniel J. McCarty (1985), in "Arthritis and allied conditions, A textbook of rheumatology" 10th Edition, Lea & Febiger]. Medication for rheumatism currently predominantly comprises anti-inflammatory drugs such as steroids and immunomodulators. If it were possible to inhibit abnormal proliferation of synoviocytes, then any such agent should be useful in the therapy of rheumatism.

Synoviocytes in rheumatism do not proliferate in an unlimited manner [*c.f*. Daniel J. McCarty (1985), in "Arthritis and allied conditions, A textbook of rheumatology" 10th Edition, Lea & Febiger], and it has been demonstrated that apoptosis occurs in the synoviocytes of patients with rheumatism. Fas antigen is expressed on the membrane of synoviocytes and Nakajima *et al.* [Nakajima, T., *et al.,* (1995), Arthritis Rheum. *38*, 485-491] and Aono *et al.* [Abstracts of the 38th Meeting of Japan Rheumatism Society (1994), p. 487, and articles of 1994 Meeting of Japan Cancer Society, (1994), p. 388] investigated whether cytotoxic anti-human Fas antibodies could induce apoptosis in abnormally proliferated synoviocytes from patients with rheumatism. They were able to induce high levels of apoptosis in abnormally proliferated synoviocytes from patients with rheumatism, compared with a control comprising synoviocytes from patients with diseases other than rheumatism.

Thus, anti-human Fas antibody is able to selectively induce apoptosis not only in lymphocytes but also in abnormally proliferated synoviocytes, so that anti-human Fas antibody should be useful as an anti rheumatic agent.

Several mouse anti-human Fas monoclonal antibodies have been obtained [for example, Yonehara, S., *et al*., (1989) J. Exp. Med. *169,* 1747-1756; Science, (1989), *245*, 301-305]. Further, as described above, it has been reported that such antibodies induce apoptosis *in vitro* in synovial cells from patients with rheumatism [c.f. page 487, Abstracts of the 38th Meeting of the Japan Rheumatology Society (1994), and page 338, Articles of 1994 Annual Meeting of the Japan Oncology Society (1994)]. However, the preparation of a humanised anti-human Fas antibody, whether IgG or IgM, has not been reported. Moreover, the successful preparation of a humanised anti-human Fas IgM antibody lacking a J chain but having the ability to induce apoptosis has never been reported.

To humanise a mouse anti-human Fas monoclonal antibody, for example, it is necessary to select the amino acid sequences of the variable regions which are to be grafted onto the human antibody acceptor. The amino acid sequence should ideally include the predicted CDR sequences, as well as selected amino acid residues of the FR sequence.

When designing a humanised antibody, the subgroup of an acceptor has conventionally been selected in one of two ways:
1) using heavy and light chains from the same known human antibody; or
2) using heavy and light chains derived from different human antibodies, which have high sequence homology to, or share consensus sequences with, the chains of the donor, while at the same time maintaining the combination of the subgroups of the acceptor chains.

Criterion (2), above, has been previously employed because there are only a limited number of naturally occurring combinations of subgroups. It has been considered important to maintain these naturally occurring combinations.

We have now, surprisingly, discovered that it is not necessary to maintain these natural combinations of subgroups, nor is it necessary to use H and L chains from the same antibody. The selection of acceptor H and L chains may be carried out from a library of primary sequences of human antibodies solely based on the homology of the framework regions of donor and acceptor, regardless of the combination of subgroups. This selection process has been used successfully to produce an anti-human Fas antibody.

Thus, in a first aspect, the present invention provides a method for the production of a humanised antibody, comprising at least one light chain and one heavy chain, the method comprising the steps of:
a selecting a non-human antibody having at least one CDR;
b selecting a human antibody heavy chain;
c selecting a human antibody light chain;
d introducing at least one CDR from the non-human antibody heavy chain into the human antibody heavy chain, to form a recombinant heavy chain; and
e introducing at least one CDR from the non-human antibody light chain into the human antibody light chain, to form a recombinant light chain;
wherein the selection of each of the human antibody heavy and light chains is determined solely by sequence homology with the non-human antibody heavy and light chains, respectively.

Anti-human Fas antibodies prepared in accordance with the present invention may be used therapeutically in humans. In addition, such humanised antibodies minimise any potential HAMA response.

The present invention allows the construction of humanised antibodies which have a minimal risk of inducing a HAMA response, whilst still having an effective antibody effector function.

The term 'sequence homology', as used herein, refers either to DNA sequence homology or to amino acid sequence homology. The term 'homology' refers to the similarity between two sequences, and is standard in the art. We prefer that the sequence homology is amino acid sequence homology. Amino acid sequence homology can be assessed by any one of a number of methods, commonly involving the computerised search of sequence databases These methods are well known to the person skilled in the art. We also prefer that the homology is assessed over the length of the framework regions.

As used herein, the term "human", in relation to antibodies, relates to any antibody which is expected to elicit little, or no, immunogenic response in a human subject, the subject in question being an individual or a group.

It will be appreciated that, in general, it is preferred that all of the CDR's from a given antibody be grafted into an acceptor antibody, in order to preserve the epitope binding region. However, there may be occasions when it is appropriate or desirable for less than the total number of CDR's to be grafted into the donor, and these are envisaged by the present invention.

We particularly prefer that all of the CDR's from the non-human antibody be grafted into the human antibody. Further, we prefer that certain areas of the framework regions be incorporated into the acceptor antibody (also referred to as the human antibody, herein) in order to maintain the 3-dimensional structure of the non-human recognition site. Such areas of the framework regions typically comprise individual amino acid residues selected for their importance, in accordance with the guidelines below. In particular, those residues which are rare in human, but common in the relevant non-human antibody, and those residues having a high probability of interacting directly with the epitope or the recognition site, are preferred to be grafted together with the CDR's.

When grafting the CDR's into the human antibody, it will normally be the case that the non-human CDR replaces a relevant human CDR in its entirety, particularly where both are of the same length. However, it may also be the case that only a part of a human CDR is replaced, or only a part of the non-human CDR is grafted, the two usually going hand-in-hand. It may also be the case that one CDR is bigger than the other but, whatever the situation, it is highly preferred to keep the human framework regions intact, other than for the replacements described above.

It will also be appreciated that the CDR's from the non-human antibody should generally be used to replace the corresponding CDR's in the human antibody. However, it is possible that a skeleton human light or heavy chain, in which the CDR regions of the human antibody chain have already been removed, can be used as an acceptor. In this case, CDR's from the non-human antibody can be introduced into the human chain at the positions previously occupied by the original human CDR's.

It will also be understood that the human heavy and light chains need not necessarily come from the same human antibody, nor even from the same class. What is important is that the sequence of the selected acceptor matches, as closely as possible, the sequence of the non-human antibody. The importance of matching the two chains (light/light or heavy/heavy) is that the resulting antibody should have a recognition site as closely resembling that of the original non-human antibody as possible, to ensure the best binding. Thus, the present invention also envisages the possibility of using matches which are not the closest possible, where there is a reasonable expectation that the resulting recombinant antibody will serve the required purpose.

Where antibodies are discussed herein, it will also be understood that similar considerations apply, *mutatis mutandis,* to any nucleic acid sequences encoding them, as appropriate.

A selection method based upon sequence homology alone, with no other constraints, makes it possible for the donor and the acceptor to share at least 70% amino acid identity in the FR portions. By adopting this approach, it is possible to reduce the number of amino acids grafted from the donor, with respect to known methods, and thus to minimise induction of the HAMA response.

It will be appreciated that the role of amino acid residues that occur rarely in the donor subgroup cannot be fully defined, since techniques for predicting the three-dimensional structure of an antibody molecule from its primary sequence (hereinafter referred to as "molecular modelling") have limited accuracy. Known methods, such as the method of Queen and co-workers [Queen *et*. *al*., *supra*], do not indicate whether the amino acid residue from the donor or from the acceptor should be selected in such a position. The selection of an acceptor molecule based upon sequence homology alone can significantly reduce the need to make this type of selection

As used herein, the term 'recombinant' relates to any substance which has been obtained by genetic engineering, insofar as the substance in question is either modified from the original substance or expressed in a different manner or in a different system from the original.

The term 'antibody', as used herein, is well known in the art, and the nature of the antibody is not crucial to the present invention. The antibody may correspond to any antibody class, where the protein actually corresponds to an antibody class. For example, the antibody may be IgG, IgM, IgA or IgE, and the class may be entirely dependent upon the administration path, for example. It will be appreciated that the heavy chain variable region may comprise a human sequence derived from one subtype of antibody, while the light chain variable region may comprise a sequence derived from a different subtype of antibody. In addition, the present invention may provide an antibody with a combination of heavy and light chains subgroups that does not occur naturally.

We prefer that the antibody of the present invention has an anti-Fas activity, although it will be appreciated that the antibodies may potentially be prepared against any antigen. We particularly prefer that the molecule is an IgM molecule with anti-Fas activity. In fact, we have also discovered that if an IgM type construct is used without the J chain, which forms a pentameric antibody structure with 5 heavy and light chain pairs, then apoptotic activity is increased with respect to a molecule containing the J chain.

The terms 'light chain' and 'heavy chain' are well known in the art. It will be appreciated that these terms, as used herein, do not necessarily refer to the full length chains, the only requirement being that the recombinant antibody molecule of the invention is able to maintain activity against an antigen, most preferably the Fas antigen.

We prefer that the amino acid sequence derived from the non-human antibody allows the antibody to cross react with an antigen, and therefore contains a CDR region, or corresponds to a CDR region. It will be appreciated that one, or more, CDR regions may be joined with the human antibody sequence. We particularly prefer that each heavy and light chain contains 3 CDR regions, and wherein the CDR regions are derived from the same non-human antibody.

The non-human region or regions may be derived from any source from which it is possible to generate antibodies. Although this is most conveniently the mouse, other sources, such as rats and rabbits are also possible. We prefer that the non-human regions are essentially CDR regions derived from the mouse CH11 antibody, which reacts with the human Fas antibody.

We prefer that the amino acid regions derived from the human antibody essentially comprise the framework regions ("FRs") of the antibody. In addition, the constant region, or a portion of the constant region, of the antibody may be present.

The FRs are present in the variable region of an H or L chain subunit of an immunoglobulin molecule. For instance, FRH₁ refers to the framework region located at the most N-terminal position in the variable region of an H chain subunit, and FRL₄ refers to the fourth framework region from the N-terminus of the variable region of an L chain subunit. Similarly, CDRH₁, for example, refers to the CDR present at the most N-terminal position in the variable region of an H chain subunit, and CDRL₃ refers to the third CDR from the N-terminus of the variable region of an L chain subunit. The FRs flank the CDR regions in any light or heavy chain.

The antibodies of the present invention have substantially no more immunogenicity in a human patient than a human antibody. This is essentially because the part of the antibody corresponding to a heterologous constant region is not present. Thus, the antibodies of the present invention may have a portion of the variable region originating from a mouse monoclonal antibody, such as CH11, but the mouse constant region has been eliminated. We prefer that the number of amino acids derived from the non-human antibody is further reduced, in order that immunogenicity is eliminated, whilst retaining desired antibody activity. This is achieved by selection of the human antibodies on the basis of sequence homology, as described above.

In addition, we have discovered a further refinement to this method by the provision of an additional selection procedure, designed to identify amino acids from the donor FRs which are important in the maintenance of the structure and function of the donor CDR regions.

Once the human acceptor molecule has been selected for a given chain, then selection of the amino acid residues to be grafted from a FR of a donor is carried out as follows:

The amino acid sequences of the donor and the acceptor are aligned. If the aligned amino acid residues of the FRs differ at any position, it is necessary to decide which residue should be selected. The residue that is chosen should not interfere with, or only have a minimal effect upon, the three-dimensional structure of the CDRs derived from the donor.

Queen *et. al,* [Japanese Provisional Patent Publication No. 4-502408] proposed a method for deciding whether an amino acid residue from the donor FR was to be grafted along with the CDR sequence. According to this method, an amino acid residue from a FR region is grafted onto the acceptor together with the CDR sequence if the residue meets at least one of the following criteria:
1) The amino acid in the human framework region of the acceptor is rarely found at that position in the acceptor, whereas the corresponding amino acid in the donor is commonly found at that position in the acceptor
2) the amino acid is closely located to one of the CDRs; and
3) the amino acid has a side-chain atom within approximately 3 Å of a CDR, as judged by a three-dimensional model of the immunoglobulin, and is potentially able to interact with an antigen or a CDR of a humanised antibody.

A residue identified by criterion (2), above, has often displays the characteristics of criterion (3). Thus, in the present invention, criterion (2) is omitted and two new criteria are introduced. Accordingly, in the present invention, an amino acid residue is grafted from a donor FR along with the CDR if the residue meets at least one of the following criteria:
a) The amino acid in the human framework region of the acceptor is rarely found at that position in the acceptor, whereas the corresponding amino acid in the donor is commonly found at that position in the acceptor;
b) the amino acid has a side-chain atom within approximately 3 Å of a CDR, as judged by a three-dimensional model of the immunoglobulin, and is potentially able to interact with an antigen or a CDR of a humanised antibody;
c) the amino acid is found in a position which is involved in determining the structure of the canonical class of the CDR;
d) the position of the amino acid is found at the contact surface of the heavy and light chains.

With respect to criterion (a), an amino acid is defined as "common" when it is found at that position in 90% or more of the antibodies of the same subclass [Kabat *et. al, supra*]. An amino acid is defined as "rare" when it is found in less than 10% of antibodies of the same subclass.

With respect to criterion (c), the position of a canonical class determinant residues can be determined unambiguously according to the information provided by Chothia and co-workers [Chothia *et. al, supra*].

With respect to criteria (b) and (d), it is necessary to carry out molecular modelling of the variable regions of the antibody in advance. While any commercially available software for molecular modelling can be used, we prefer that the AbM software is used [Oxford Molecular Limited, Inc.].

Predictions made by molecular modelling have limited accuracy. Therefore, in the present invention, the structure prediction obtained by molecular modelling was assessed by comparing it with X-ray crystallography data from the variable regions of various antibodies.

When using a structural model generated by molecular modelling (AbM software), two atoms are presumed to be in contact with each other by van der Waal's forces when the distance between the two atoms is less than the sum of their van der Waal's radii plus 0.5 Å. A hydrogen bond is presumed to be present when the distance between polar atoms, such as an amide nitrogen and a carbonyl oxygen of the main and side chains, is shorter than 2.9 Å, that is, the average length for a hydrogen bond, plus 0.5 Å. Furthermore, when the distance between the two oppositely charged atoms is shorter than 2.85 Å plus 0.5 Å, they are presumed to form an ion pair.

The positions of amino acids in the FR which frequently contact a CDR were identified, based upon X-ray crystallography data from the variable regions of various antibodies. These positions were determined irrespective of subgroups. For the light chains, these are positions 1, 2, 3, 4, 5 23, 35, 36, 46, 48, 49, 58, 69, 71 and 88, and for the heavy chains positions 2, 4, 27, 28, 29, 30, 36, 38, 46, 47, 48, 49, 66, 67, 69, 71, 73, 78, 92, 93, 94 and 103. The above amino acid numbering follows the definition of Kabat *et al.,* [Kabat *et al., supra*]. This numbering system is followed hereinafter. When molecular modelling was used, the amino acid positions listed above were shown to be in contact with CDR residues in two thirds of the antibody variable regions that were examined.

These findings were used to define criterion (b) above. Specifically, if an amino acid position in an FR is predicted both to contact a CDR by molecular modelling and is frequently found experimentally to contact a CDR by X-ray crystallographic analysis, then the grafting of the amino acid residue of the donor is made a priority. In any other case, criterion (b) is not considered.

Similarly, with respect to criterion (d), X-ray crystallography data from the variable regions of a number of antibodies indicates that the amino acid residues at positions 36, 38. 43, 44, 46, 49, 87 and 98 in light chains and those at positions 37, 39, 45, 47, 91, 103 and 104 in heavy chains are frequently involved in the contact between heavy and light chains. If any of these amino acids are predicted to be involved in light and heavy chain contact by molecular modelling, then grafting of the amino acid residue of the donor is given priority. In any other case, the criterion (d) is not considered.

It will be appreciated that the present invention further provides DNA and RNA encoding any of the above identified antibodies, especially DNA. DNA and RNA encoding both the heavy and light chains is provided.

It will be appreciated that the DNA may be in any suitable form so that it may be incorporated into a vector, suitably an expression vector. It may also be associated with any other suitable sequences, such as leader sequences or sequences for the expression of the encoded protein in the form of a fusion protein, for example.

The present invention further envisages a host cell transformed with a vector as defined above, and a system for the expression of a protein of the invention comprising such a host cell transformed with one or more expression vectors containing the above DNA. The protein of the invention may be obtained from such systems, after cultivation of the system, by standard techniques.

Certain preferred aspects and embodiments of the present invention now follow:

A genetically engineered immunoglobulin M(IgM) protein, said IgM protein having an apoptosis-inducing activity without having a J chain protein, wherein the IgM protein is composed solely of one of a light polypeptide chain protein comprising the amino acid sequence as defined in SEQ ID No. 78 of Sequence Listing, a light polypeptide chain protein comprising the amino acid sequence as defined in SEQ ID No. 80 of Sequence Listing, a light polypeptide chain protein comprising the amino acid sequence as defined in SEQ ID No. 82 of Sequence Listing or a light polypeptide chain protein comprising the amino acid sequence as defined in SEQ ID No. 84 of Sequence Listing and one of a heavy polypeptide chain protein comprising the amino acid sequence as defined in SEQ ID No. 86 of Sequence Listing or a heavy polypeptide chain protein comprising the amino acid sequence as defined in SEQ ID No. 88 of Sequence Listing.

It will be appreciated that there are four preferred light chain sequences and two preferred heavy chain sequences. Any of the light chain sequences may be combined with any of the heavy chain sequences. Thus, preferred combinations are:

The light chain as defined by SEQ ID No. 78 and heavy chain defined by Seq ID No. 86.

The light chain as defined by SEQ ID No. 78 and heavy chain defined by Seq ID No. 88.

The light chain as defined by SEQ ID No. 80 and heavy chain defined by Seq ID No. 86.

The light chain as defined by SEQ ID No. 80 and heavy chain defined by Seq ID No. 88.

The light chain as defined by SEQ ID No. 82 and heavy chain defined by Seq ID No. 86.

The light chain as defined by SEQ ID No. 82 and heavy chain defined by Seq ID No. 88

The light chain as defined by SEQ ID No. 84 and heavy chain defined by Seq ID No. 86.

The light chain as defined by SEQ ID No. 84 and heavy chain defined by Seq ID No. 88.

The invention further provides DNA encoding any of the 8 proteins defined above. These sequences are given as SEQ ID Nos. 77, 79, 81, 83, 85 and 87, encoding proteins defined by SEQ ID Nos. 78, 80, 82, 84, 86 and 88 respectively. Also preferred is DNA which hybridises with such DNA, preferably under conditions of 60 - 70 °C and in 6 x SSC.

Further preferred is a recombinant DNA vector containing any of the DNA described above, especially recombinant DNA vectors pHκKY2-58, pHκKF2-19, pHκRY2-10, pHκRF2-52, pHµH5-1 and pHµM1-1. The present invention also includes cells transformed with such vectors, especially *E. coli* strain pHκKY2-58 (FERM BP-5861), *E. coli* strain pHκKF2-19 (FERM BP-5860), *E. coli* strain pHκRY2-10 (FERM BP-5859), *E. coli* strain pHκRF2-52 (FERM BP-5862), *E. coli* strain pHµH5-1 (FERM BP-5863) and *E. coli* strain pHµM1-1 (FERM BP-5864).

A preferred method for producing an immunoglobulin protein of the present invention comprises:
culturing a cell transformed by a DNA vector described above under conditions which enable expression of DNA encoding the immunoglobulin H chain or L chain subunit contained in the vector, and
recovering the immunoglobulin protein from the culture.

The present invention further provides use of a humanised anti-Fas antibody as defined above in the manufacture of a medicament for the treatment or prophylaxis of one of the physiological conditions referred to herein, especially autoimmune diseases and rheumatic diseases.

Essentially, we have successfully cloned the genes coding for the H and L chains of a mouse IgM anti-human Fas monoclonal antibody from a cDNA library prepared from antibody-producing hybridoma cells. The full-length nucleotide sequences were determined. The positions of the CDRs regions were then identified in each chain. Amino acid sequences were selected containing these CDRs regions, along with several amino acid residues from the framework regions. These sequences were grafted into the H and L chains of human IgM immunoglobulins, in order to obtain complete H and L chains of humanised anti-human Fas antibodies.

DNA encoding the humanised H and L chains was cloned into expression vectors. Co-transfection of an H chain expression vector and an L-chain expression vector into cultured animal cells allowed the production of a protein having an apoptosis-inducing activity and that was functional as an anti-human Fas antibody.

The DNA of the present invention may be obtained by first preparing poly(A)⁺ RNA from mouse hybridoma cells producing anti-human Fas monoclonal antibody, such as CH11. The poly(A)⁺ RNA may then be converted to cDNA using a reverse transcriptase, and purifying the cDNA encoding the H and L chains of the antibody. Yonehara *et al*. [(1989), J. Exp. Med.; *169*, 1747 *et seq*.] obtained an anti-human Fas monoclonal antibody, which was designated CH11, by fusion of mouse myeloma cells with mouse lymphocytes after the mice had been immunised with the Fas-expressing human diploid fibroblast cell-line FS-7. CH11 derived from the hybridoma is itself commercially available from Igaku-seibutsugaku Kenkyujo, K.K.

Poly(A)⁺ RNA may be obtained either by first preparing total RNA and then purifying poly(A)⁺ RNA from the total RNA using, for example an affinity column packed with oligo(dT) cellulose, oligo(dT) latex beads etc., or it may be obtained by directly purifying poly(A)⁺ RNA from cell lysates using such affinity materials as described above. Total RNA may be prepared, for example, by such methods as: alkaline sucrose density gradient ultracentrifugation [*c.f.* Dougherty, W. G. and Hiebert, E., (1980), Virology, *101,* 466-474]; the guanidine thiocyanate-phenol method; the guanidine thiocyanate-trifluorocaesium method; and the phenol-SDS method. The preferred method, however, employs guanidine thiocyanate and caesium chloride [*c.f*. Chirgwin, J. M., *et al.* (1979), Biochemistry, *18*, 5294-5299].

The single stranded (ss) cDNA obtained by the use of reverse transcriptase, as described above, can then be converted to double stranded (ds) cDNA. Suitable methods for obtaining the ds cDNA include the S1 nuclease method [*c.f.* Efstratiadis, A., *et al*.,(1976), Cell, *7*, 279-288] and the Gubler-Hoffman method [*c.f.* Gubler, U. and Hoffman, B. J., (1983), Gene, *25*, 263-269]. However, we prefer to employ the Okayama-Berg method [*c.f*. Okayama, H. and Berg, P., (1982), Mol. Cell. Biol. *2*, 161-170].

The ds cDNA obtained above may then be integrated into a cloning vector and the resulting recombinant vector can then be used to transform a suitable micro-organism, such as *E. coli*. The transformant can be selected using a standard method, such as by selecting for tetracycline resistance or ampicillin resistance encoded by the recombinant vector. If *E. coli* is used, then transformation may be effected by the Hanahan method [*c.f*. Hanahan, D (1983), J. Mol. Biol., *166,* 557-580]. Alternatively, the recombinant vector may be introduced into competent cells prepared by co-exposure to calcium chloride and either magnesium chloride or rubidium chloride. If a plasmid is used as a vector, then it is highly desirable that the plasmid harbours a drug-resistant gene, such as mentioned above, in order to facilitate selection. Brute force selection is possible, but not preferred. Although plasmids have been discussed, it will be appreciated that other cloning vehicles, such as lambda phages, may be used.

Methods for selecting transformants having the desired DNA include the following:

### (1) Screening using a synthetic oligonucleotide probe

If all or part of the amino acid sequence of the desired protein has been elucidated, then a short contiguous sequence, which is also representative of the desired protein, may be used to construct an oligonucleotide probe. The probe encodes the amino acid sequence but, owing to the degeneracy of the genetic code, there may be a large number of probes that can be prepared. Thus, an amino acid sequence will normally be selected which can only be encoded by a limited number of oligonucleotides. The number of oligonucleotides which it is necessary to produce can be further reduced by the substitution of inosine where any of the four normal bases can be used. The probe is then suitably labelled, such as with ³²P, ³⁵S or biotin, and is then hybridised with denatured, transformed DNA from the transformant which has been immobilised on a nitrocellulose filter. Positive strains show up by detection of the label on the probe.

### (2) Screening by polymerase chain reaction

If all or part of the amino acid sequence of the desired protein has been elucidated, then sense and antisense oligonucleotide primers corresponding to separate non-contiguous parts of the amino acid sequence can be synthesised. These primers can then be used in the polymerase chain reaction technique [*c.f.* Saiki, R. K., *et al.* (1988), Science, *239,* 487-491] to amplify the desired DNA fragment coding for the mouse anti-human Fas monoclonal antibody subunit. The template DNA used herein may be cDNA synthesised by a reverse transcriptase reaction using mRNA obtained from a hybridoma producing anti-human Fas antibody, such as that which expresses CH11. The DNA fragment thus synthesised may either be directly integrated into a plasmid vector, such as by using a commercial kit, or may be labelled with, for example, ³¹P, ³⁵S or biotin, and then used as a probe for colony hybridisation or plaque hybridisation to obtain the desired clone.

Monoclonal antibody CH11 is an immunoglobulin M ("IgM") molecule, a complex comprising five subunits each of the H (µ chain) and L chains, and one J chain. Thus, in order to elucidate partial amino acid sequences for the subunits, the subunits must be separated, and this can be done using any suitable technique, such as electrophoresis, column chromatography, etc. well known to those skilled in the art. Once the subunits have been separated, they can be sequenced, such as by the use of an automatic protein sequencer (for example, PPSQ-10 of Shimadzu), in order to determine the amino acid sequence of at least the N-terminal of each subunit. Oligonucleotides/primers can then be produced using this knowledge.

Harvesting of DNA encoding each subunit of anti-human Fas monoclonal antibody from the appropriate transformants obtained above may be performed by well known techniques, such as those described by Maniatis, T., *et al*. [in "Molecular Cloning A Laboratory Manual" Cold Spring Harbor Laboratory, NY, (1982)]. For example, the region of DNA coding for the desired subunit may be excised from plasmid DNA after separating the fraction corresponding to the vector DNA from a transformant which has been determined to possess the necessary plasmid.

*E. coli* DH5α has been transformed with plasmids containing DNA encoding the heavy and light chains of CH11, prepared as described above, and the resulting two transformants (designated *E. coli* pCR3-H123 and *E. coli* pCR3-L103 respectively) have been deposited in accordance with the terms of the Budapest Treaty on the Deposition of Microorganisms at the Kogyo Gijutsuin Seimei-Kogaku Kogyo Gijutsu Kenkyujo (NIBH) on February 28, 1996, and have been allocated deposit Nos. FERM BP-5427 and FERM BP-5428, respectively. *E. coli* DH5α containing these plasmids may be cultivated in a directly comparable manner to *E. coli* DH5α not possessing these plasmids. All deposited strains may be selected by their resistance to ampicillin. The DNA of the present invention, therefore, may be obtained using these deposits. This can be done, for example, either by cultivating the deposits and isolating the plasmids, or by using the polymerase chain reaction (PCR) using the plasmids as templates.

Wherever appropriate, DNA sequences may be sequenced in accordance by various well known methods in the art including, for example, the Maxam-Gilbert chemical modification technique [*c.f.* Maxam, A. M. and Gilbert, W. (1980) in "Methods in Enzymology" *65*, 499-276] and the dideoxy chain termination method using M13 phage [*c.f.* Messing, J. and Vieira, J. (1982), Gene, *19*, 269-276]. In recent years, a further method for sequencing DNA has gained wide acceptance, and involves the use of a fluorogenic dye in place of the conventional radioisotope in the dideoxy method. The whole process is computerised, including the reading of the nucleotide sequence after electrophoresis. Suitable machinery for the process is, for example, the Perkin-Elmer Sequence robot "CATALYST 800" and the Perkin-Elmer model 373A DNA Sequencer. The use of this technique renders the determination of DNA nucleotide sequences both efficient and safe.

Based on the data of the thus determined nucleotide sequences and the respective N-terminal amino acid sequences of the H and L chains of CH11, the entire amino acid sequences of the H and L chains of CH11 can be determined.

Accordingly, from the thus determined nucleotide sequences of the DNA encoding the H and L chains of CH11, in conjunction with the sequence data for the N-termini of the H and L chains, it was possible to determine the entire amino acid sequence of the H and L chains of CH11.

The CDR regions, FR regions and the constant region of the H and L chains of CH11 were identified by comparing amino acid sequence of the H and L chains with the known amino acid sequences of immunoglobulins determined by Kabat [Kabat *et al.,* supra]

The DNA encoding the variable regions of the H and L chains of a humanised anti-human Fas antibody of the present invention may be prepared in a number of ways.

In one method, polynucleotide fragments of between 60 and 70 nucleotides in length may be synthesised which represent partial nucleotide sequences of the desired DNA. The synthesis process is arranged such that the ends of fragments of the sense strand alternate with those of the antisense strand. The resulting polynucleotide fragments can be annealed to one another and ligated by DNA ligase. In this way the desired DNA fragment encoding the variable regions of the H and L chains of the humanised anti-human Fas antibody may be obtained.

Alternatively, DNA coding for the entire variable region of the acceptor may be isolated from human lymphocytes. Site directed mutagenesis may be used to introduce restriction sites into the regions encoding the CDRs of the donor. The CDRs may then be excised from the acceptor using the relevant restriction enzyme. DNA encoding the CDRs of the donor can then be synthesised and ligated into the acceptor molecule, using DNA ligase.

We prefer that DNA encoding the variable regions of the H and L chains of a desired humanised anti-human Fas antibody is obtained by the technique of overlap extension PCR [Horton, *e. al.,* (1989), Gene, *77*, 61-68].

Overlap extension PCR allows two DNA fragments, each coding for a desired amino acid sequence, to be joined. For the sake of example, the two fragments are herein designated as (A) and (B). A sense primer (C) of 20 to 40 nucleotides which anneals with a 5' region of (A) is synthesised, along with an antisense primer of 20 to 40 nucleotides (D), which anneals with a 3'-region of (B). Two further primers are required. First, a chimaeric sense primer (E), which comprises 20 to 30 nucleotides from a 3'-region of (A) joined to 20 to 30 nucleotides from a 5'- region of (B). Secondly, an antisense primer (F) is required, complementary to the sense primer.

A PCR reaction may be carried out using primers (C) and (F), in combination with a DNA template containing fragment A. This allows a DNA product to be produced comprising 20 to 30 nucleotides of the 5'- region of (B) joined to the 3'-end of (A). This fragment is termed fragment (G).

Similarly, PCR may be carried out using primers (D) and (E), in combination with a DNA template containing fragment B. This allows a DNA product to be produced comprising 20 to 30 nucleotides of the 3'- region of (A) joined to the 5'-end of (B). This fragment is termed fragment (H).

The (G) and (H) fragments carry complementary sequences of 40 to 60 nucleotides in the 3'- region of (G) and 40 to 60 nucleotides in the 5'-region of (H), respectively. A PCR reaction may be carried out using a mixture of the (G) and (H) fragments as a template. In the first denaturation step, the DNA becomes single stranded. Most of the DNA returns to the original form in the subsequent annealing step. However, a part of the DNA forms a heterologous DNA duplex, due to the annealing of (G) and (H) fragments in the region of sequence overlap. In the subsequent extension step, the protruding single-stranded portions are repaired to result in chimaeric DNA which represents a ligation of (A) and (B). This DNA fragment is hereinafter referred to as (I). Fragment (I) can be amplified using primer (C) and primer (D).

In embodiments of the present invention, fragments (A) and (B) may represent DNA encoding the CDR regions of the H and L chains of a mouse humanised anti-human Fas monoclonal antibody, DNA coding for the FR regions of human immunoglobulin IgM or DNA coding for the secretion signal of human immunoglobulin IgM.

The codon or codons which correspond to a desired amino acid are known. When designing a DNA sequence from which to produce a protein, any suitable codon may be selected. For example, a codon can be selected based upon the codon usage of the host. Partial modification of a nucleotide sequence can be accomplished by the standard technique of site directed mutagenesis, utilising synthetic oligonucleotide primers encoding the desired modifications [Mark, D. F., *et. al*, (1984) Proc. Natl. Acad. Sci. USA *81*, 5662-5666]. By using selected primers to introduce a specific point mutation or mutations, DNA coding for the variable regions of the H and L chains of any desired humanised anti-human Fas antibody can be obtained.

Integration of DNA of the present invention thus obtained into an expression vector allows transformation of prokaryotic or eukaryotic host cells. Such expression vectors will typically contain suitable promoters, replication sites and sequences involved in gene expression, allowing the DNA to be expressed in the host cell.

The four transformant strains carrying plasmids encoding the variable regions of the L chains of a humanised anti-human Fas antibody were deposited with the Kogyo Gijutsuin Seimei-Kogaku Kogyo Gijutsu Kenkyujo on March 11, 1997 in accordance with the Budapest Treaty. These strains were *E. coli* pHκKY2-58, *E. coli* pHκ.KF2-19, *E. coli* pFκRY2-10 and *E. coli* pHκRF2-52, having the accession numbers FERM BP-5861, BP-5860, BP-5859 and BP-5862, respectively.

The two transformant strains carrying plasmids encoding the variable regions of the H chains of a humanised anti-human Fas antibody were deposited with the Kogyo Gijutsuin Seimei-Kogaku Kogyo Gijutsu Kenkyujo on March 11, 1997 in accordance with the Budapest Treaty. These strains were *E. coli* pHµH5-1 and *E. coli* pHµM1-1, having the accession numbers FERM BP-5863 and BP-5864 respectively.

The DNA of the present invention may be obtained using these deposits. This can be done, for example by cultivating the deposits and isolating the plasmids, or by using PCR using the plasmids as templates.

Suitable prokaryotic host cells include, for example, *E. coli* (*Escherichia coli*) and *Bacillus subulis.* In order to express the gene of interest in such host cells, these host cells may be transformed with a plasmid vector containing a replicon derived from a species compatible with the host, typically having an origin of replication and a promoter sequence, such as lac UV5. These vectors preferably have sequences capable of conferring a selection phenotype on the transformed cell.

A suitable strain of *E. coli* is strain JM109 derived from *E. coli* K12. Suitable vectors include pBR322 and the pUC series plasmids. Suitable promoters include the lactose promoter (lac) and the tryptophan lactose promoter (trc). In general, it will be appreciated that the present invention is not limited to the use of such hosts, vectors, promoters, etc., as exemplified herein and that any suitable systems may be used, as desired.

A suitable preferred strain of *Bacillus subtilis* is strain 207-25, and a preferred vector is pTUB228 [*c.f.* Ohmura, K., *et. al,* (1984), J. Biochem., *95*, 87-93]. A suitable promoter is the regulatory sequence of the *Bocillus subtilis α*-amylase gene. If desired, the DNA sequence encoding the signal peptide sequence of α-amylase may be linked to the DNA of the present invention to enable extracellular secretion.

Suitable eukaryotic cell hosts include those from vertebrates, yeasts, etc. Suitable vertebrate cells include, for example, the monkey cell line COS [*c.f.* Gluzman, Y. (1981), Cell, *23*, 175-182]. Suitable yeasts include *Saccharomyces cerevisiae* and *Schizosaccharomyces pombe.*

In general, the requirements for suitable expression vectors for vertebrate cells are that they comprise: a promoter usually upstream of the gene to be expressed; an RNA splicing site; a polyadenylation site; and a transcription termination sequence, etc. As desired, they may additionally contain, as needed, an origin of replication. A suitable plasmid is pSV2dhfr containing the SV40 early promoter [*c.f.* Subramani, S., *et. al,* (1981), Mol. Cell. Biol., *1,* 854-884].

Suitable eukaryotic micro-organisms are the yeasts, such as *S. cerevisiae,* and suitable expression vectors for yeasts include pAH301, pAH82 and YEp51. Suitable vectors contain, for example, the promoter of the alcohol dehydrogenase gene [*c.f.* Bennetzen, J. L. and Hall, B. D. (1982), J. Biol. Chem., *257*, 3018-3025] or of the carboxypeptidase Y GAL10 promoter [*c.f.* Ichikawa, K., *et. al,* (1993), Biosci. Biotech. Biochem., 5*7*, 1686-1690]. It desired, the DNA sequence encoding the signal peptide sequence of carboxypeptidase Y may be linked to the DNA to be expressed in order to enable extracellular secretion.

In the case of COS cells being used as hosts, suitable vectors comprise the SV40 replication origin, enabling autonomous growth, a transcription promoter, a transcription termination signal and an RNA splicing site. The expression vectors can be used to transform the cells by any suitable method, such as the DEAE-destran method [*c.f*. Luthman, H, and Magnusson, G. (1983), Nucleic Acids Res., *11,* 1295-1308], the phosphate calcium-DNA co-precipitation method [*c.f.* Graham, F. L. and van der Eb, A. J. (1973), Virology, *52*, 456-457] and the electric pulse electroporation method [*c.f.* Neumann, E., *et*. *al,* (1982), EMBO J., *1,* 841-845]. In a preferred embodiment COS cells are co-transfected with two separate expression vectors - one containing DNA encoding a protein comprising the variable region of the H chain of CH11 and one containing DNA encoding a protein comprising the variable region of the L chain of CH11, these vectors being expressed simultaneously to generate a humanised recombinant anti-human Fas antibody.

Transformants of the present invention may be cultured using conventional methods, the desired proteins being expressed either intra- or extra- cellularly. Suitable culture media include various commonly used media, and will generally be selected according to the host chosen. For example, suitable media for COS cells include RPMI-1640 and Dulbecco's Modified Eagle Minimum Essential medium which can be supplemented with, as desired, foetal bovine serum (FBS). The culture temperature may be any suitable temperature which does not markedly depress the protein synthesis capability of the cell, and is preferably in the range of 32 to 42°C, most preferably 37°C, especially for mammalian cells. If desired, culture may be effected in an atmosphere containing 1 to 10% (v/v) carbon dioxide.

The protein expressed by the transformants of the present invention may be isolated and purified by various well known methods of separation according whether the protein is expressed intra- or extra- cellularly and depending on such considerations as the physical and chemical properties of the protein. Suitable specific methods of separation include: treatment with commonly used precipitating agents for protein; various methods of chromatography such as ultrafiltration, molecular sieve chromatography (gel filtration), adsorption chromatography, ion exchange chromatographing, affinity chromatography and high performance liquid chromatography (HPLC); dialysis; and combinations thereof.

By the use of such methods as described above, the desired protein can be readily obtained in high yields and high purity. Even though they lack the J chain. the humanised anti-human Fas antibodies of the present invention have a cytotoxic activity equivalent to, or better than that of CH11.

The specific binding activity of proteins of the present invention for Fas antigen may be determined, for example, by enzyme-linked immunosorbent assay (ELISA). This technique comprises immobilising a test antigen on the bottom surface of wells of a 96-well plate, then introducing a test sample into the wells. After a washing step, the wells are then exposed to an enzyme-labelled antibody that specifically recognises the H chain (µ chain) of human IgM. The cells are then washed again, and any label remaining in the wells is detected. cDNA encoding for human Fas antigen has previously been disclosed and methods for introducing the cDNA into animal cells for expression thereof are also known [*c.f.* Itoh, N., *et, al,* (1991), Cell, *66*, 233-243]. Antigen for use in the above ELISA method can be obtained from the culture supernatant of cells which have been transformed with an expression vector containing the gene encoding a fusion protein comprising the extracellular region of the human Fas antigen and the extracellular region of mouse interleukin 3 receptor. as disclosed in Itoh (*supra*).

The ability of the proteins of the present invention to induce apoptosis can be established, for example, by culturing cells such as the human lymphocyte cell line HPB-ALL (Morikawa, S., *et. al,* (1978) Int. J. Cancer 21, 166-170) or Jurkat (American Type Culture No. TIB-152) etc.) in medium in which the test sample has been or will be added. The survival rate may then be determined by an MTT assay (Green, L. M., *et. al,* (1984) J. Immunological Methods 70, 257-268).

Using the DNA of the present invention, it is possible to produce a Fv fragment composed essentially only of the variable regions of H and L chains, or a single-strand Fv in which H and L chains are connected via a flexible peptide ['scFv', Huston, J. S., *et al*. (1988) Proc. Natl. Acad. Sci. USA *85*, 5879].

The present invention also provides methods and therapeutic compositions for treating the conditions referred to above. Such compositions typically comprise a therapeutically effective amount of the protein of the present invention in admixture with a pharmaceutically acceptable carrier therefor. The composition may be administered in any suitable manner, such as by parenteral, intravenous, subcutaneous or topical administration. In particular, where the condition to be treated is local, then it is preferred to administer the protein as close as possible to the site. For example, serious rheumatic pain may be experienced in major joints, and the protein may be administered at such locations. Systemically administered proteins of the present invention are particularly preferably administered in the form of a pyrogen-free, therapeutically, particularly parenterally, acceptable aqueous solution. The preparation of such pharmaceutically acceptable protein solutions with regard to aspects such as pH, isotonicity, stability and the like, is well within the skill of the person skilled in the art. In addition, the compositions of the present invention may comprise such further ingredients as may be deemed appropriate, such as cell growth retardants and other medicaments.

The dosage regimen for the various conditions treatable with the proteins of the present invention will be readily apparent to one skilled in the art, taking into account various factors, such as the condition, body weight, sex and diet of the patient, the severity of any symptoms, time, the desirability of repeat treatment, as well as any other appropriate clinical factors. As a general guide, the daily dose should typically be in the range of 1 - 1000 µg protein per kilogram of body weight.

The humanised anti-human Fas antibodies of the present invention are able to bind to the human Fas antigen as well as having a superior apoptosis-inducing activity. Therefore, the antibodies provided in the present invention are useful as an anti-rheumatic agents. In addition, the anti-rheumatic agents provided by the present invention all involve genetically-engineered humanised immunoglobulins, which reduces the potential toxicity of the preparations.

The invention will now be explained in more detail with reference to the following Examples, the Examples being illustrative of, but not binding upon, the present invention. The Examples represent specific embodiments of the present invention. A summary of the Figures referred to in the Examples is as follows:

Figure 1 shows the construction of a cDNA library for cloning of the full-length DNA coding for the subunits of CH11.

Figure 2 shows the cloning of the full-length DNA coding for the subunits of CH11.

Figure 3 shows the strategy used for sequencing of the H chain.

Figure 4 shows the strategy used for sequencing of the L chain.

Figure 5 shows the first step PCR for the preparation of VL-KY and VL-KF DNA fragments.

Figure 6 shows the second step PCR for the preparation of VL-KY and VL-KF DNA fragments.

Figure 7 shows the third step PCR for the preparation of VL-KY and VL-KF DNA fragments.

Figure 8 shows the construction of plasmids pHκKY2-58 and pHκKF2-19.

Figure 9 shows the first step PCR for the preparation of VL-RY and VL-RF DNA fragments.

Figure 10 shows the second step PCR for the preparation of VL-RY and VL-RF DNA fragments.

Figure 11 shows the construction of plasmids pHκRY2-10 and pHκRF2-52.

Figure 12 shows the preparation of MEC DNA fragment.

Figure 13 shows the construction of plasmid pMEC22.

Figure 14 shows first step PCR for the preparation of the VH1234 DNA fragment.

Figure 15 shows the second step PCR for the preparation of the VH1234 DNA fragment.

Figure 16 shows the third step PCR for the preparation of the VH1234 DNA fragment.

Figure 17 shows the construction of plasmid pMEHC20.

Figure 18 shows the first step PCR for the preparation of HUMFR5' DNA, HUMFR3' DNA, MOUFR5' DNA and MOUFR3' DNA fragments.

Figure 19 shows the second step PCR for the preparation of HUMFR2 DNA and MOUFR2 DNA fragments.

Figure 20 shows the construction of plasmids pHFR3 and pHFR4.

Figure 21 shows the first step PCR for the preparation of the HHC123 DNA fragment.

Figure 22 shows the second step PCR for the preparation of the HHC123 DNA fragment.

Figure 23 shows the third step PCR for the preparation of HHC123 DNA fragment.

Figure 24 shows the construction of plasmid pMECW5.

Figure 25 shows the construction of plasmids pHCµH and pHCµM.

Figure 26 shows the first step PCR for the preparation of FASAIC DNA fragment.

Figure 27 shows the second step PCR for the preparation of FASAIC DNA fragment.

Figure 28 shows the construction of plasmid phFas-AIC2.

Figure 29 shows the determination of Fas-binding activity by ELISA.

Figure 30 shows the determination of Fas-binding activity by ELISA.

Figure 31 shows the determination of cytotoxic activity in HPB-ALL cells.

Any methods, preparations, solutions and such like which are not specifically defined may be found in 'Molecular cloning - A laboratory Handbook'. (*supra*). All solutions are aqueous and made up in sterile deionised water, unless otherwise specified.

### REFERENCE EXAMPLE 1

### Cloning of DNA Encoding the Variable Region of Mouse Monoclonal Antibody CH11 against the Human Fas Antigen

### (1-1) Preparation of poly(A)± RNA

Total RNA was prepared from a CH11-producing hybridoma [obtained from Yonehara, see Yonehara *et al.,* (1989), J. Exp. Med., *169*, 1747 *et seq*.] in accordance with the method described by Chirgwin and co-workers [Chirgwin, J. M., *et al.,* (1979) Biochemistry, 18, 5294, *et seq*]. Specifically, the CH11-producing hybridoma [Yonehara, S., *et al.,* (1994), International Immunology *6*, 1849-1856] was cultured in ASF104 medium [Ajinomoto] containing 10% (v/v) foetal bovine serum [Gibco]. Approximately 6.7 x 10⁸ cells were harvested by centrifugation and the supernatant was discarded. The resulting pellet of cells was then mixed straightaway with 60 ml of 4 M guanidine thiocyanate solution [Fluka]. The cells in the resulting suspension were subsequently lysed by aspirating the cell suspension through a syringe equipped with a 21-gauge needle three times. The cell lysate thus obtained was layered onto 3 ml of 5.7 M caesium chloride/0.1 M EDTA solution (pH 7.5) in an ultracentrifugation tube [13PA:Hitachi Koki] and the tube was spun in an Hitachi RPS-40T Rotor (13PA tube, 150,000 x g at 20°C for 18 hours) to precipitate the RNA. The precipitated RNA was dissolved in water, extracted with chloroform/1-butanol (4 : 1, v/v) and then re-precipitated with 100% ethanol.

Poly (A)⁺ RNA was purified next from the total, resulting RNA, prepared above, by routine methods [*c.f.* Sambrook, J. *et al.,* (1989), "Molecular Clonine-: A Laboratory Manual" (2nd Edition), Cold Spring Harbor Lab., 7.26 - 7.28]. More specifically, a disposable polystyrene column (diameter 0.7 cm) was packed with 100 mg of oligo dT cellulose [Pharmacia, Type 7]. The column was equilibrated with a loading buffer, comprising 20 mM tris-hydrochloric acid (pH 7.6), 0.5 M sodium chloride, 1 mM ethylenediamine tetraacetate (EDTA) and 0.1% (w/v) sodium dodecylsulphate (SDS). Total RNA (approximately 1.2 mg), was then dissolved in a total volume of 400 µl of water by heating at 65°C for 5 minutes, and then 400 µl of loading buffer (made up at double the above concentration) was added to the solution. The resulting mixture was cooled to room temperature and then poured onto the column. The fraction that passed straight through the column was recovered, heated at 65°C for a further 5 minutes, and poured back onto the column.

The column was next washed with 10 ml of loading buffer, and then further washed with 5 ml of loading buffer containing 0.1 M sodium chloride to remove both non-adsorbates and also non-specific adsorbates. Subsequently, 5 ml of elution buffer [10 mM tris-hydrochloric acid (pH 7.5), 1 mM EDTA and 0.05% (w/v) SDS] was poured onto the column in order to elute specific adsorbates. The resulting eluate was recovered in fractions of 200 µl. The third and fourth 200 µl elution fractions (400 µl in total) were combined, and mixed with 40 µl of 3 M sodium acetate (pH 4.0) and 1 ml of 100% ethanol. The resulting mixture was stored at -20°C overnight. The next day the mixture was spun in a centrifuge (10,000 x g, 4°C for 10 minutes) to recover the pellet. This pellet was used as the poly (A)⁺ RNA sample and was stored at -80°C until it was required for use.

### (1-2) Cloning of DNA coding for variable regions

cDNA fragments coding for the variable regions of the H chain and L chain of mouse anti-Fas antigen (CH11) were cloned by 'RT-PCR', which combines reverse transcription (using reverse transcriptase -RT) with the polymerase chain reaction (PCR). The combination of these techniques allowed the specific amplification of a desired sequence from the poly(A)⁺ RNA sample derived from the CH11-producing hybridoma prepared in (1-1).

Two sets of primers for the RT-PCR reaction were selected from the Ig-Prime Set [Novagen]. MulgV_{H}5'-B and MulgMV_{H}3'-1 were used to amplify a region of the H chain, while MulgκV_{L}5' and MuIgMV_{L}3'-1 were used to amplify a region of the L chain. RT-PCR reactions were carried out using both the H chain primer sets and L chain primer sets, respectively.

### a) Reverse transcriptase reaction

A reverse transcriptase reaction solution (44 µl) was made up as follows 10 mM tris-hydrochloric acid (pH 8.3), 50 mM potassium chloride, 0.1 mM dATP, 0.1 mM dGTP, 0.1 mM dCTP, 0.1 mM dTTP, 1.5 mM magnesium chloride, 2.5 pmol of H chain or L chain 3'-side primer, 50 ng of the poly (A)⁺ RNA prepared in (1.1) and 20 units of reverse transcriptase [BIOCHEMICAL KOGYO CO., LTD.] derived from Moloney murine leukaemia virus (MMLV) were combined and the resulting mixture was incubated at 42°C for one hour.

### b) Amplification by PCR

The reverse transcriptase reaction solution prepared in a) was mixed with 25 pmol of H chain or L chain 5' primer, as appropriate, with 5 units of Taq DNA polymerase [ampliTaq DNA Polymerase obtained from Perkin Elmer, Japan] to a final volume of 100 µl of reaction buffer supplied with kit (buffers and solutions for enzymes are as supplied with supplier's kit, unless otherwise specified). The total, resulting, reaction mixture was heated at 94°C for 2 minutes, and then subjected to a heat cycle of 94°C for one minute, 50°C for one minute and 72°C for 2 minutes. This cycle was repeated 30 times. The solution was then kept at 72°C for a further 10 minutes. A gene amplifier PCR system 9600 [Perkin Elmer, Japan] was used to control the reaction temperature in all of the PCR reactions.

### c) Assay of PCR product

A portion of the PCR reaction mixture prepared in b) was analysed by gel electrophoresis on a 1.5% (w/v) agarose gel [FMC Bioproducts]. The product of each of the H and L chain PCR reactions was obtained as a band of about 430 bp The band size was estimated by comparing it with molecular weight markers that had also been run on the same gel.

### d) Cloning of PCR product

Each of the PCR products obtained in b) was ligated into separate plasmid vectors using an original TA cloning kit [Invitrogen]. More specifically, 50 ng of the pCRII vector and four units of T4 DNA ligase (both included in the kit) were added to a ligase reaction buffer [6 mM tris-hydrochloric acid (pH 7.5), 6 mM magnesium chloride, 5 mM sodium chloride, 7 mM β-mercaptoethanol, 0.1 mM ATP, 2 mM dithiothreitol (DTT), 1 mM spermidine and 0.1 mg/ml bovine serum albumin]. The ligase reaction buffer also contained a portion of the PCR reaction mixture which was selected such that it contained approximately 10 ng of the desired PCR product, as estimated by gel electrophoresis in c) above. The resulting mixture was incubated at 14°C for 15 hours.

Subsequently, 2 µl of the ligase reaction mixture was mixed with 50 µl of *E. coli,* strain TOP10F' (included in the kit), which had previously been made competent by the addition of 2 µl of 0.5 M β-mercaptoethanol. The resulting transformation mixture was placed on ice for 30 minutes, heated at 42°C for 30 seconds and then placed on ice again for a further 2 minutes. After this time, the mixture was then added to 500 µl of SOC medium [2% (w/v) tryptone, 0.5% (w/v) yeast extract, 0.05% (w/v) sodium chloride, 2.5 mM potassium chloride, 1 mM magnesium chloride, 20 mM glucose], and the resulting mixture was cultured with rotational shaking for one hour (37°C, 110 rpm). The resulting culture was then spread onto L-broth agar medium plates [1%(w/v) tryptone, 0.5% (w/v) yeast extract, 0.5% (w/v) sodium chloride, 0.1% (w/v) glucose, 0.6% (w/v) Bacto Agar (Difco)] containing 100 µg/ml of ampicillin and the plates were cultured at 37°C overnight without shaking.

Ampicillin-resistant colonies generated by this procedure were selected and scraped off with platinum picks. Cells from the selected colonies were separately cultured in 5 ml of L-broth medium containing 100 µg/ml of ampicillin, at 37°C, overnight. The cultures were then centrifuged to pellet the cells and plasmid DNA was prepared from the cells using the alkaline lysis method [*c.f.* Sambrook, J., *et al, supra*]. A plasmid for each of the H and L primer sets was obtained, and these were designated pVH4 (the plasmid containing the fragment amplified using the H chain primer set) and pVL8 (the plasmid containing the fragment amplified using the L chain primer set).

### REFERENCE EXAMPLE 2

### Determination of the Amino Acid Sequence and Nucleotide Sequence of the Variable Regions of CH11

### (2-1) Determination of the N-terminal amino acid sequences of the variable regions of the H chain and L chain of CH11

### a) Preparation of CH11

The CH11-producing hybridoma (see Example 1) was grown to a cell number of 2 x 10⁸ in an ASF 104 medium [Ajinomoto] containing 10% (v/v) of bovine serum [Gibco], and this preparation was then cultured in 50 ml of serum free ASF 104 medium at 37°C for 5 days. After this time, the culture was centrifuged (Tommy Seiko's No. 4 rotor, 15,000 x g, 4°C for 15 minutes) and the supernatant was collected. CH11 was obtained from the culture supernatant using an E-Z-Sep antibody purification kit [Pharmacia Biotech].

b) A portion of the purified CH-11, corresponding to 100 µl of the supernatant prepared in a), was added to 10 µl of 100 mM tris-hydrochloric acid buffer (pH 6.8) containing 10% (v/v) of β-mercaptoethanol and 4% (w/v) SDS. The resulting mixture was denatured by heating at 95°C for 5 minutes. The denatured sample was then subjected to electrophoresis on a 12% (w/v) polyacrylamide gel. After electrophoresis the gel was immersed in transfer buffer [25 mM tris-boric acid (pH 9.5), 10% methanol (v/v)] and shaken at room temperature for 15 minutes. The protein bands on the gel were then transferred onto a polyvinylidene difluoride (PVDF) membrane [Nippon Millipore Ltd.], using a semidrive blotting apparatus [Iwaki Glass Co., Ltd.], at a constant current of 0.2 A at 4°C for 1 hour. After this time, the PVDF membrane was stained with a 0.1% (w/v) Coomassie Brilliant Blue solution and destained with 100% methanol. Only two major protein spots were seen, corresponding to the H chain and L chain. These protein spots were excised, and the gel containing them was dried at room temperature.

c) The amino acid sequence of the proteins transferred onto the PVDF membrane in b) was analysed using a gas phase protein sequencer [PPSQ-10; Shimadzu Corporation] using the automatic Edman method [see Edman, P., *et al.,* (1967), Eur. J. Biochem. *1*, 80 *et seq*.] The N-terminal amino acid sequence of the variable region of the H chain of CH11, and the N-terminal amino acid sequence of the L chain were thus determined, and are shown as SEQ ID NOs. 13 and 14 of the sequence listing, respectively.

### (2-2) Determination of DNA nucleotide sequence

The total nucleotide sequences of the cDNA coding for the variable regions of the H and L chains of CH11 were determined by sequencing the inserts in plasmids pVH4 and pVL8 respectively (prepared in Example 1).

The pCRII vector has an SP6 promoter sequence and a T7 promoter sequence, and these flank any inserted cDNA, thus allowing the sequence of the inserts of pVH4 and pVL8 to be determined using oligonucleotide primers [Perkin Elmer, Japan] corresponding to the sequences. Samples for sequence analysis were prepared using these primers and a dye primer cycle-sequencing kit [Perkin Elmer, Japan]. Plasmid DNA from plasmids pVH4 or pVL8 was used as a template. The sequence of each cDNA insert was determined using a DNA sequencer [Model 373, Perkin Elmer, Japan]. The cDNA nucleotide sequence of the H chain variable region is shown as SEQ ID NO. 15 and the cDNA nucleotide sequence of the L chain variable region is shown as SEQ ID NO. 16.

The N-terminal amino acid sequence of the H chain of CH11, represented by amino acid Nos. 1 to 15 of SEQ ID NO. 13 in the sequence listing, corresponds completely to the amino acid sequence encoded by nucleotide Nos. 32 to 76 of SEQ ID NO. 15. Therefore, it was deduced that plasmid pVH4 contains DNA coding for the variable region of the H chain of CH11.

The N-terminal amino acid sequence of the L chain of CH11, represented by amino acid Nos. 1 to 21 of SEQ ID NO. 14 in the sequence listing, corresponds completely to the amino acid sequence encoded by nucleotide Nos. 29 to 91 of SEQ ID NO. 16. Therefore, it was deduced that plasmid pVL8 contains DNA coding for the variable region of the L chain of the CH11.

### REFERENCE EXAMPLE 3

### Cloning of DNA Encoding the Complete H Chain, L Chain and J Chains of CH11

### (3-1) Preparation of a cDNA library

A cDNA library was prepared by the Okayama-Berg method [Okayama, H. *et al.,* (1987). Methods in Enzymology *154*, 3-28]. More specifically, 5 µg of poly(A)⁺RNA, as prepared in Example 1-1 (a) from the CH11-producing hybridoma, were added to 30 µl of reaction mixture [50 mM tris-hydrochloric acid (pH 8.3), 6 mM magnesium chloride, 40 mM potassium chloride, 2 mM dATP, 2 mM dGTP, 2 mM dCTP, 2 mM dTTP, 2 µg vector primer (3'-oligo(dT)-tailed, pcDV-1): Pharmacia] containing 75 units of reverse transcriptase [Seikagaku Kogyo, Co., Ltd.] derived from Avian myeloblastosis virus (AMV). The resulting mixture was incubated at 37°C for 30 minutes.

After this time, an equivalent volume of phenol-chloroform (1 : 1, v/v) was added to the reaction mixture and thoroughly mixed. The resulting mixture was centrifuged (10,000 x g, room temperature, 5 minutes) and the aqueous layer was recovered (this procedure of extracting with phenol:chloroform and recovering the aqueous supernatant is referred hereinafter as "phenol-chloroform extraction"). To the resulting aqueous layer were added 35 µl of 4 M ammonium acetate and 140 µl of 100% ethanol, and the resulting mixture was cooled at -70°C for 15 minutes, then centrifuged (10,000 x g, 4°C, 15 minutes). The pellet was washed with a 75% (v/v) solution of ethanol and then dried under reduced pressure.

The dried precipitate was then dissolved in 13 µl of distilled water, and then 5.6 µl of terminal transferase reaction mixture [140 mM sodium cacodylate, 30 mM tris-hydrochloric acid (pH 6.8), 1 mM cobalt chloride, 0.5 mM DTT, 0.3 µg polyadenylic acid (polyA, Pharmacia), 0.2 mM dCTP] was added and the resulting reaction mixture was incubated at 37°C for 5 minutes. Terminal deoxynucleotidyl transferase [21 units, Pharmacia] was then added, in accordance with the supplier's instructions, and the reaction was allowed to proceed for 5 minutes. The reaction mixture was then subjected to phenol-chloroform extraction. Then, 20 µl of 4 M ammonium acetate and 80 µl of 100% ethanol were added to the recovered aqueous layer, and the mixture was cooled at -70°C for 15 minutes, then centrifuged (10,000 x g at 4°C for 15 minutes). The pellet was washed with a 75% (v/v) solution of ethanol and dried under reduced pressure.

The DNA precipitate obtained in this way was dissolved in 30 µl of reaction mixture [10 mM tris-hydrochloric acid (pH 7.5), 60 mM sodium chloride, 7 mM magnesium chloride], and 30 units of restriction enzyme HindIII were added to the resulting solution. In general, where a restriction enzyme is used, but no buffer is specified, then the buffer which is used is the buffer supplied with the enzyme. In the case where DNA is digested with two enzymes, digestion is carried out with the two enzymes sequentially. After the first digestion, the DNA is precipitated, resuspended and then digested with the second enzyme. Precipitation and resuspension techniques are well known in the art [*c.f*. Sambrook *et al., supra*]. All of the restriction enzymes and buffers used in the present Examples were supplied by Takara Schuzo.

The DNA was allowed to be digested at 37°C overnight in the digestion solution. Subsequently, the reaction mixture was subjected to phenol-chloroform extraction. Then, 35 µl of 4 M ammonium acetate and 140 µl of 100% ethanol were added to the recovered aqueous layer, and the mixture was cooled at -70°C for 15 minutes. The mixture was centrifuged (10,000 x g, 4°C x 15 minutes) to precipitate the DNA, and the resulting pellet was washed with a 75% (v/v) solution of ethanol and dried under reduced pressure. The thus prepared precipitate DNA was used as a cDNA sample in subsequent procedures.

In parallel, plasmid DNA from the vector pcDL-SRα296 [*c.f.* Takebe, Y *et al*., (1989), "JIKKEN IGAKU (Experimental Medicine)", *7*, pp. 95-99] was digested with the restriction enzyme PstI. The product of the digestion was treated with dGTP and terminal deoxynucleotidyl transferase [Pharmacia], in order to add oligo dG to the 3' terminal end, as follows:

pcDL-SRα296 DNA (100µg, present in 50µl) was added to 10µl of 10x terminal deoxynucleotidyl transferase buffer [1x buffer: 1.4 M sodium cacodylate, 0.3M Tris-HCl, (pH 7.6), 10µl of DTT (1mM), 20µl of 0.1mM ³H-dGTP Dupont) and 10µl of terminal transferase (210 IU, Pharmacia)]. The mixture was incubated for 40 minutes at 37°C, and then mixed with an equal volume of TE buffer-saturated phenol. After standing, the aqueous layer was removed and subjected to a phenol-chloroform extraction. After both of the phenol and phenol-chloroform extractions had been performed, then the DNA was precipitated using 100% ethanol and resuspended in 50µl of TE buffer.

The total precipitated DNA was digested with the restriction enzyme HindIII, and the products of the digestion were separated by gel electrophoresis on a 1.8% (w/v) agarose gel. A band of 800 bp was excised from the gel, and extracted from the gel using a GENECLEAN kit [Funakoshi] according to the manufacturer's instructions. The resulting DNA was dissolved in 100 µl of TE buffer, and 100µl of 100% ethanol was added. The final concentration of the DNA was 0.09 µg/µl.

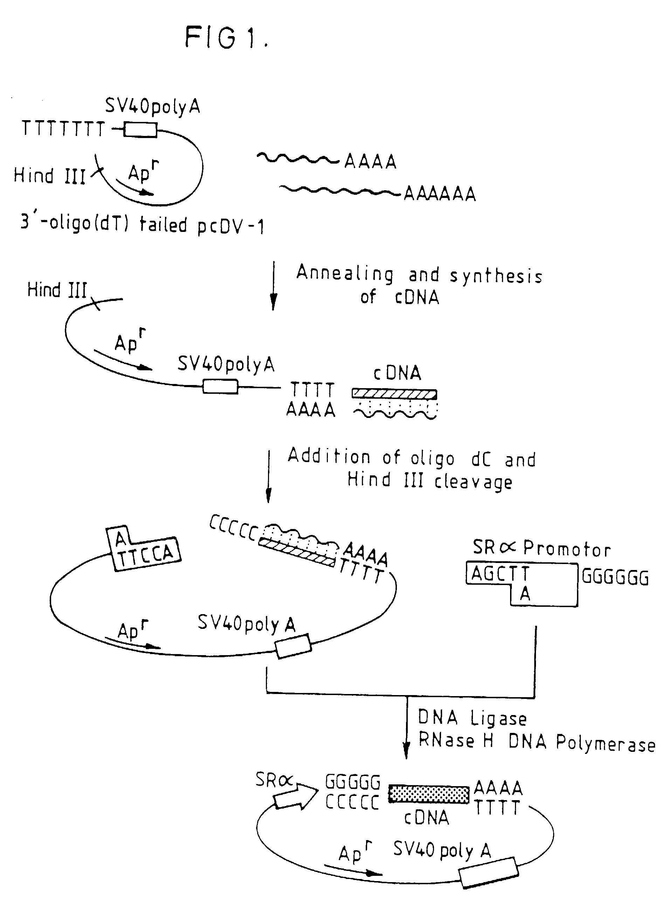

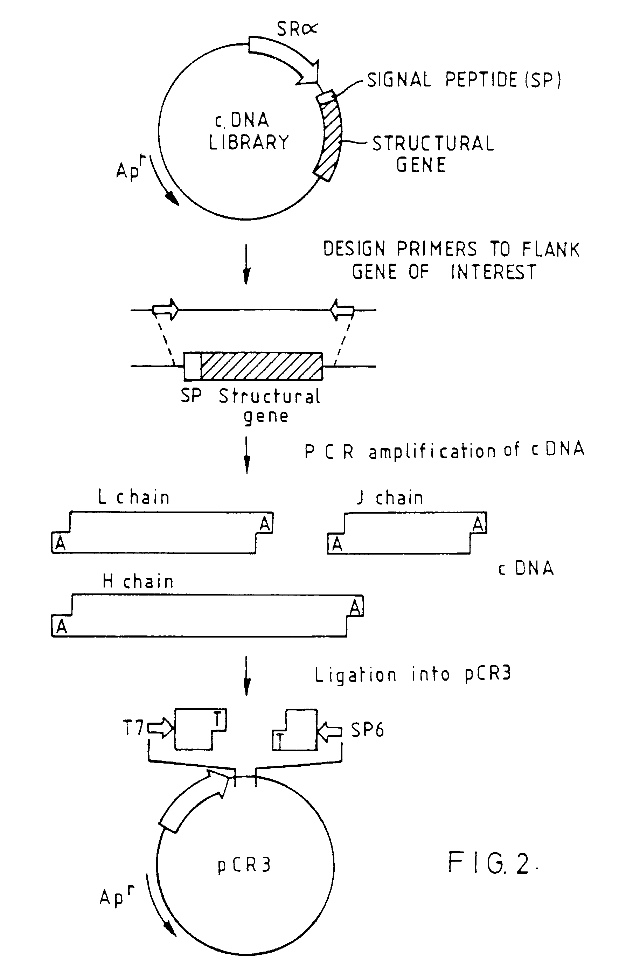

The resulting product yielded a linker-DNA in which oligo (dG) is attached to the SRα promoter *(c.f*. Figure 1, which is a schematic view of the construction of a cDNA library to enable cloning of DNA encoding the total length of each subunit of CH11. Figure 2 is a diagram showing the process of cloning and amplifying DNA encoding the total length of each subunit of CH11).

The precipitated, dried, cDNA sample, prepared above, was dissolved in 10 µl TE buffer [10 mM tris-hydrochloric acid (pH 7.5), 1 mM EDTA]. A portion of the resulting solution (1 µl) was added to reaction buffer [10 mM, tris hydrochloric acid (pH 7.5), 1 mM EDTA, 100 mM sodium chloride] containing 0.08 pmol of the linker-DNA prepared above. The resulting mixture was heated at 65°C for 5 minutes and then incubated at 42°C for 30 minutes. After this time, 10 µl of 10x ligase buffer [10 mM ATP, 660 mM tris-hydrochloric acid (pH 7.5), 66 mM magnesium chloride, 100 mM DTT], 76 µl of distilled water and 1 µl of 10 m M β-nicotinamide adenine dinucleotide [NAD, Boehringer Nlannheim] were added to the reaction mixture, and the resulting mixture was cooled on ice for 10 minutes. *E. coli* DNA ligase [8.4 µg equivalent, Pharmacia] was then added to the cooled reaction mixture, and the whole was incubated at 12°C, overnight.

After this incubation, 2 µl of nucleotide solution [2 mM dATP,2 mM dCTP, 2 mM dGTP, 2 mM dTTP], 0.5 µl of 10 mM NAD, 42 µg equivalent of *E. coli* DNA ligase [Pharmacia], 4.1 units of DNA polymerase I [Pharmacia], and 5.5 units of ribonuclease H [Pharmacia] were added to the reaction mixture. The resulting mixture was then incubated at 12°C for one hour and then at 22°C for a further hour. The cDNA library prepared in this way was stored at -20°C until it was needed.

### (3-2) Cloning by PCR

### a) Preparation of primer

In the case of the H chain, the amino acid sequence of the variable region of the H chain of CH11, as determined in Example 2, was compared with the antibody amino acid sequence database prepared by Kabat *et al*. [Kabat E. A. *et al.,* (1991), in "Sequences of Proteins of Immunological Interest Vol. II", U.S. Department of Health and Human Services]. It was determined that the H chain (µ chain) of CH11 was sub class 2A. Therefore, an oligonucleotide primer was synthesised such that it would hybridise with a part of the 5'-non-translated region of the DNA coding for mouse H chain, sub class 2a. The oligonucleotide primer which was selected had the sequence: 5'-CTAAGGGAAT TCCGCCTCTC CTCAGACACT GAA-3' (H5-1; SEQ ID NO. 17 of the sequence listing).

An oligonucleotide primer was also designed that would hybridise with a part of the 3' non-translated region of the CH11 H-chain. The design of the olignucleotide was based on the nucleotide sequence of the DNA coding for the mouse immunoglobulin M chain constant region reported by Goldberg, *et al.* [see Goldberg, I.G., *et al.,* (1981), Gene *15*, 33-42], and the sequence which was selected was: 5'-TTTTACTCTA GAGACCCAAG GCCTGCCTGG TTGA-3' (H3-1; SEQ ID NO. 18 of the sequence listing).

For the L chain, the amino acid sequence of the variable region of the L chain of CH11, as determined in Example 2, was compared with the antibody amino acid sequence database prepared by Kabat and co-workers [*supra*]. It was found that the L chain of CH11 was sub-class κ2. Therefore, an oligonucleotide primer was designed such that it would hybridise with a part of the 5'-terminal, non-translated region of the DNA coding for mouse L chain, sub-class κ2. The oligonucleotide primer which was selected had the sequence 5'-AAATAGCAAT TCCAGTCTCC TCAGGCTGTC TCC-3' (L5-1; SEQ ID NO. 19 of the sequence listing).

An oligonucleotide primer was also designed that would hybridise with a part of the 3' non-translated region. The design of the olignucleotide was based on the nucleotide sequence of the DNA coding for the mouse immunoglobulin κ chain constant region registered under the registration name MUSIGB1L1 (Accession No. D14630). The sequence used was: 5'-ATGATCTCTA GAGTGGTGGC ATCTCAGGAC CT-3' (L3-1; SEQ ID NO. 20 of the sequence listing).

In the case of the J chain, there is no variable region and both the sequence of the DNA coding for the J chain, and the amino acid sequence of the J chain are known [*c.f.* Cann, G. M., *et al.,* (1982), Proc. Natl. Acad. Sci. USA, *79*, 6656-6660]. Based on this finding, oligonucleotide primers were synthesised that would hybridise with a part of the 5' and 3' non-translated regions of the DNA coding for the J chain. These oligonucleotides had the sequences: 5'-TTGCGGAATT CCTCACCTGT CCTGGGGTTA TT-3' (J5-1; SEQ ID NO. 21 of the sequence listing) and 5'-ATTGCCTCTA GAGCCTCTAA GGACAACGAC CT-3' (J3-1; SEQ ID NO. 22 of the sequence listing).

These oligonucleotide primers were all synthesised using an automatic DNA synthesiser 380 B [Perkin Elmer, Japan] by the phosphoamidite method [see Mattrucci, M. D. and Caruthers, M. H. (1981), J. Am. Chem. Soc., *103*, 3185-3191]. After synthesis was complete, each primer was cleaved from the support and deprotected, and then freeze dried. The resulting product was dissolved in distilled water and stored at -20°C until it was needed.

### b) Amplification of target gene by PCR

PCR reaction solution [10 mM tris-hydrochloric acid (pH 8.3), 50 mM potassium chloride, 1.5 mM magnesium chloride, 2.5 mM dATP, 2.5 mM dGTP, 2.5 mM dCTP, 2.5 mM dTTP] was prepared, and 0.1 µl of the cDNA library described in Example 4-1, 1 unit of Taq DNA polymerase [Perkin Elmer, Japan] and 15 pmol of the oligonucleotide primer (prepared in 3-2 a) were added to 100 µl of the PCR reaction solution and heated at 94°C for 2 minutes. The resulting moisture was then subjected to a heat cycle of 94°C for one minute, 55°C for one minute and 72°C for 2 minutes. This cycle was repeated 30 times. After the last cycle, the solution was kept at 72°C for a further 10 minutes.

The combinations of the primers that were used in the respective reactions are as follows:
H5-1 and H3-1 (for H chain);
L5-1 and L3-1 (for L chain); and
J5-1 and J3-1 (for J chain).

### c) Assay of PCR product

After the PCR reaction in b) had been performed, a portion of the reaction mixture was analysed by gel electrophoresis on a 0.8% (w/v) agarose gel in the case of the H chain For the L and J chains, a 1.5% (w/v) agarose gel [agarose was obtained from FMC Bioproducts] was used. The product of the PCR reaction was a band of approximately 1900 bp for the H chain, 800 bp for the L chain and 650 bp for the J chain. The band sizes were estimated by comparison with molecular weight markers run on the same gel.

### d) Cloning of PCR product

Each of the PCR products obtained in b) was ligated into a plasmid vector, using a eukaryote TA cloning kit [Invitrogen]. More specifically, 60 ng of pCR3 vector (included in the kit) and four units of T4 DNA ligase were added to ligase reaction buffer [6 mM tris-hydrochloric acid (pH 7.5), 6 mM magnesium chloride, 5 mM sodium chloride, 7 mM β-mercaptoethanol, 0.1 mM ATP, 2 mM DTT, 1 mM spermidine, 0.1 mg/ml bovine serum albumin], containing a portion of the PCR reaction mixture. The volume of the PCR reaction mixture was selected such that it contained about 10 ng of the desired PCR product, as estimated by gel electrophoresis. The resulting mixture was incubated at 14°C for 15 hours.

A portion of the ligase reaction mixture (2µl) was mixed with 50 µl of *E. coli* cells, strain TOP10F' (included in the kit), made competent by the addition of 2 µl of 0.5 M β-mercaptoethanol. The resulting mixture was placed on ice for 30 minutes, warmed at 42°C for 30 seconds, then placed on ice again for 2 minutes. SOC medium (500 µl, as described above) was then added to this mixture, and the resulting mixture was cultured at 37°C with rotational shaking (110 rpm) for one hour. The culture liquid as then spread onto L-broth agar medium plates containing 100 µg/ml of ampicillin and cultured at 37°C overnight. Ampicillin-resistant colonies which appeared were then scraped off with a platinum pick and cultured in 5 ml of L-broth medium containing 100 µg/ml of ampicillin at 37°C overnight. These cultures were centrifuged to precipitate cells which were then used to prepare plasmid DNA by the alkaline lysis method [Sambrook *et al*., *supra*].

Three of the resulting plasmids were designated pCR3-H123 (the plasmid including H chain-coding cDNA), pCR3-L103 (the plasmid including L chain-coding cDNA) and pCR3-J1123 (the plasmid including J chain-coding cDNA). Competent cells of *E. coli* strain DH5α [Gibco] were transformed with one of the plasmids pCR3-H123, pCR3-L103 or pCR3-J1123 and the resulting transformants were deposited at the Research Institute of Life Science and Technology of the Agency of Industrial Science and Technology on February 28, 1996 under the deposit Nos. FERM BP-5427, FERM BP-5428 and FERM BP-5429, respectively. DNA encoding the H, L and J chains of CH11 are readily prepared from these strains by well known methods.

### REFERENCE EXAMPLE 4

### Determination of Total Nucleotide Sequence of the cDNA Coding for CH11 H Chain, L Chain and J chains

### (4-1) Determination of nucleotide sequence of DNA

The mouse immunoglobulin M chain consists of an N-terminal variable region containing about 110 residues and a constant region containing about 470 residues, adjacent to the variable region. The mouse immunoglobulin κ chain consists of an N-terminal variable region containing about 110 residues and a constant region containing 107 residues adjacent the variable region. It was predicted that the complete nucleotide sequences of the cDNAs coding for the CH11 H chain and L chain would consist of nucleotide sequences coding for known constant regions and which were ligated to nucleotide sequences coding for the variable regions of the chains, as identified in Example 2 [*c.f.* Kabat E. A. *et. al., supra*].

The nucleotide sequence encoding the J chain of CH11 was presumed to be the same as that of the known J chain sequence.

Based on these presumed nucleotide sequences, oligonucleotide primers of 20 nucleotides in length were synthesised, corresponding to sequences of the H, L and J chains, separated by coding intervals of 60 to 200 bp. These primers where used for sequence analysis.

The sequences of the synthesised oligonucleotide primers were as follows:

For the H chain:

5'-TGGGGCCTCA GTGAAGATAT -3' (SHF-2; SEQ ID NO. 23 of the sequence listing)
5'-CAATGGTGGT ACTGGCTACA -3' (SHF-3; SEQ ID NO. 24 of the sequence listing)
5'-TGACATCTGA GGACTCTGCA -3' (SHF-4; SEQ ID NO. 25 of the sequence listing)
5'-TCCTCAGAGA GTCAGTCCTT -3' (SHF-6; SEQ ID NO. 26 of the sequence listing)
5'-TCCTTCACCT GGAACTACCA -3' (SHF-7; SEQ ID NO. 27 of the sequence listing)
5'-TCCCAAGAGC ATCCTTGAAG -3' (SHF-8; SEQ ID NO. 28 of the sequence listing)
5'-AGATCTGCAT GTGCCCATTC -3' (SHF-9; SEQ ID NO. 29 of the sequence listing)
5'-TCTAAACTCA TCTGCGAGGC -3' (SHF-10; SEQ ID NO. 30 of the sequence listing)
5'-GGTGACCATC GAGAACAAAG -3' (SHF-11; SEQ ID NO. 31 of the sequence listing)
5'-AGGGGTCTCA CCTTCTTGAA -3' (SHF-12; SEQ ID NO. 32 of the sequence listing)
5'-TCCTTTGCCG ACATCTTCCT -3' (SHF-13; SEQ ID NO. 33 of the sequence listing)
5'-GTGTGTACTG TGACTCACAG -3' (SHF-15; SEQ ID NO. 34 of the sequence listing)
5'-AACTGAACCT GAGGGAGTCA -3' (SHF-16; SEQ ID NO. 35 of the sequence listing)
5'-AACTCTTGCC CCAAGAGAAG -3' (SHF-17; SEQ ID NO. 36 of the sequence listing)
5'-ATCCTGACTG TGACAGAGGA -3' (SHF-18; SEQ ID NO. 37 of the sequence listing)
5'-ACAAGTCCAC TGGTAAACCC -3' (SHF-19; SEQ ID NO. 38 of the sequence listing)
5'-AGGATATCTT CACTGAGGCC -3' (SHR-1; SEQ ID NO. 39 of the sequence listing)
5'-ATCCACTCAA GGCTCTTTCC -3' (SHR-2; SEQ ID NO. 40 of the sequence listing)

5'-ACTGCAGAGT CCTCAGATGT -3' (SHR-3, SEQ ID NO. 41 of the sequence listing)
5'-AGACGGTGAC TGAGGTTCTT -3' (SHR-4; SEQ ID NO. 42 of the sequence listing)
5'-CAGGTGAAGG AAATGGTGCT -3' (SHR-5; SEQ ID NO. 43 of the sequence listing)
5'-ATGCTCTTGG GAGACAGCAA -3' (SHR-6; SEQ ID NO. 44 of the sequence listing)
5'-CTCTGTTTTT GCCTCCGTAG -3' (SHR-7; SEQ ID NO. 45 of the sequence listing)
5'-TGGCCTCGCA GATGAGTTTA -3' (SHR-8; SEQ ID NO. 46 of the sequence listing)
5'-CCTTTGTTCT CGATGGTCAC -3' (SHR-9; SEQ ID NO. 47 of the sequence listing)
5'-TGTGGAGGAC ACGTTCTTCA -3' (SHR-10; SEQ ID NO. 48 of the sequence listing)
5'-ACTTTGAGAA GCCCAGGAGA -3' (SHR-12; SEQ ID NO. 49 of the sequence listing)
5'-AGATCCCTGT GAGTCACAGT -3' (SHR-13; SEQ ID NO. 50 of the sequence listing)
5'-AGCAGGTGGA TGTTTGTGCA -3' (SHR-14; SEQ ID NO. 51 of the sequence listing)
5'-TGAAGCCACT GCACACTGAT -3' (SHR-15; SEQ ID NO. 52 of the sequence listing)
5'-AGTTCCATTC CTCCTCTGTC -3' (SHR-16; SEQ ID NO. 53 of the sequence listing)
5'-TGTGTCAGAC ATGATCAGGG -3' (SHR-18; SEQ ID NO. 54 of the sequence listing)

For the L chain:

5`-TGAAGTTGCC TGTTAGGCTG -3' (SLF-1; SEQ ID NO. 55 of the sequence listing)
5`-CTTGGAGATC AAGCCTCCAT -3' (SLF-2; SEQ ID NO. 56 of the sequence listing)
5'-GCTGAGGATC TGGGAGTTTA -3' (SLF-3; SEQ ID NO. 57 of the sequence listing)
5'-GATGCTGCAC CAACTGTATC -3' (SLF-4; SEQ ID NO. 58 of the sequence listing)
5'-CGACAAAATG GCGTCCTGAA -3' (SLF-5; SEQ ID NO. 59 of the sequence listing)
5'-ACGTTGACCA AGGACGAGTA -3' (SLF-6; SEQ ID NO. 60 of the sequence listing)
5'-ATCTGCAAGA GATGGAGGCT -3' (SLR-2; SEQ ID NO. 61 of the sequence listing)
5'-ACCCCAGAAA ATCGGTTGGA -3' (SLR-3; SEQ ID NO. 62 of the sequence listing)
5'-CCGGAGGAAC ATGTGTACTT -3' (SLR-4; SEQ ID NO. 63 of the sequence listing)
5'-TCGTTCATAC TCGTCCTTGG -3' (SLR-6; SEQ ID NO. 64 of the sequence listing)
5'-CATCTCAGGA CCTTTGTCTC -3' (SLR-7; SEQ ID NO. 65 of the sequence listing)

For the J chain:

5'-CACCTGTCCT GGGGTTATTT -3' (SJF-1; SEQ ID NO. 66 of the sequence listing)
5'-AGACAAGATG AAGACCCACC -3' (SJF-2; SEQ ID NO. 67 of the sequence listing)

5`-AAGCGACCAT TCTTGCTGAC -3' (SJF-3; SEQ ID NO 68 of the sequence listing)
5`-ATATCTCTGA TCCCACCTCC -3' (SJF-8, SEQ ID NO. 69 of the sequence listing)
5`-GAAATGCGAT CCTGTGGAAG -3' (SJF-5; SEQ ID NO. 70 of the sequence listing)
5`-CTATACCACT ATGGTCCCAC -3' (SJF-6; SEQ ID NO. 71 of the sequence listing)
5`-AGAAGCAGGT GGGTCTTCAT -3' (SJR-2; SEQ ID NO. 72 of the sequence listing)
5`-TAGAGGTAAC TCGGGTACAC -3' (SJR-3; SEQ ID NO. 73 of the sequence listing)
5`-AAGTTCCTTC TCAGTGGGGA -3' (SJR-8; SEQ ID NO. 74 of the sequence listing)
5`-GGTGGCAGTA ACAACCTGAT -3' (SJR-5; SEQ ID NO. 75 of the sequence listing)
5`-CATGATACCT AAGTGGGACC -3' (SJR-6; SEQ ID NO. 76 of the sequence listing)

Each oligonucleotide primer was synthesised by the phosphoamidite method using an automatic DNA synthesiser [Model 350B: Perkin Elmer, Japan]. Samples for sequence analysis of the H chain were prepared using DNA from plasmid pCR3-H123. Samples for sequence analysis of the L chain were prepared using DNA from plasmid pCR3-L103. Samples for sequence analysis of the J chain were prepared using DNA from pCR3-J1123. The PCR reaction was carried out using a Prism Ready Reaction Terminator Cycle Sequencing Kit [Perkin Elmer, Japan], as follows.

pCR3-H123 (1.5 µg) and 4.8 pmol of primer (SHF-2) were mixed to a final volume of 16 µl in distilled water. A portion of this pCR3-H123/primer mixture (9.5 µl) was mixed with 10.5 µl of a premix containing Taq DNA polymerase. All of this procedure was in accordance with the instructions in the kit. The resulting mixture was placed in an automatic reactor [Catalyst: Perkin Elmer, Japan]. The reaction cycle used was as follows: 95°C for 30 seconds, 50°C for 15 seconds and 60°C for 4 minutes, repeated 25 times.

After completion of the reaction cycles, 80 µl of sterilised water was added to the resulting solution, and the DNA in the resulting mixture was extracted twice by the phenol/chloroform method. The recovered aqueous layer was mixed with 15 µl of 2 M sodium acetate and 300 µl of 100% ethanol, followed by centrifugation to recover the precipitate. The precipitate was washed with a 70% (v/v) solution of ethanol and dried under reduced pressure, then dissolved in 3 µl of sample solution [4 µl 0.25 M EDTA, 100 µl formamide and 15 µl sterilised water].

Sequencing reactions were run and analysed on a DNA sequencer [Model 373A: Perkin Elmer, Japan], for the 32 H chain primers, the 11 L chain primers and the 11 J chain primers.

The sequence data obtained for each primer were combined and integrated in order to determine the complete nucleotide sequence of the H, L and J chains of CH11. The cDNA nucleotide sequences of each plasmid insert are shown by SEQ ID NOs. 7, 9 and 11 of the sequence listing, respectively. The amino acid sequences that correspond to these nucleotide sequences are shown by SEQ ID NOs. 8, 10 and 12 of the sequence listing, respectively.

### (4-2) Primary structure of the H chain of CH11

The nucleotide sequence of the H chain variable region, shown as nucleotide Nos. 32 to 379 of SEQ ID NO. 15 of the sequence listing, was found to be identical with that of the nucleotide Nos. 58 to 405 of SEQ ID NO 7.

The amino acid sequence shown as amino acid Nos. 117 to 571 of SEQ ID NO. 8 was found to be identical with the amino acid sequence in the H chain constant region derived from mouse IgM, when compared with the database of antibody amino acid sequences [Kabat E.A. *et. al., supra*].

The amino acid sequence shown as amino acid Nos. -19 to -1 of SEQ ID NO. 8 was concluded to be a signal sequence of the H chain of CH11.

The nucleotide sequence shown as nucleotide Nos. 406 to 1770 of SEQ ID NO. 7 was found to be identical with that of the H chain constant region of mouse IgM.

Based on these results, the total nucleotide sequence could be established, together with the total amino acid sequence.

### (4-3) Primary structure of CH11 L chain

The nucleotide sequence of the L chain variable region, shown as nucleotide Nos. 29 to 364 of SEQ ID NO. 16 in the sequence listing, was found to be identical with that of nucleotide Nos. 58 to 393 of SEQ ID NO. 9.

The amino acid sequence shown as amino acid Nos. 113 to 219 of SEQ ID NO. 10 was found to be identical with the amino acid sequence in the mouse κL chain constant region, when compared with Kabat's database of antibody amino acid sequences.

The amino acid sequence shown as amino acid Nos. -19 to -1 of SEQ ID NO. 10 was concluded to be a signal sequence for the L chain.

The nucleotide sequence shown as nucleotide Nos. 394 to 714 of SEQ ID NO. 9 was established to be completely identical with that in the mouse κL chain constant region.

Based on these results, the total nucleotide sequence could be established, together with the total amino acid sequence.

### (4-4) Primary structure of J chain of CH11

The amino acid sequence shown as amino acid Nos. 1 to 137 of SEQ ID NO. 12 was compared with the antibody amino acid sequence database, and found to be identical to the known mouse J chain.

The nucleotide sequence shown as nucleotide Nos. 67 to 477 of SEQ ID NO. 11 was found to be identical with that of the known mouse J chain.

The amino acid sequence shown as amino acid Nos. -22 to -1 of SEQ ID NO. 12 was concluded to be a signal sequence for the J chain of CH11.

Based on these results, the total nucleotide sequence could be established, together with the total amino acid sequence.

### (4-5) Determination of Complementarity Determining Regions (CDR)

Both the position and the amino acid sequence of each CDR in the variable regions of the H chain and L chain of CH11, determined as described above, were identified by comparison with Kabat's antibody amino acid sequence database [*supra*]. This database shows that the amino acid chain length of the framework area in the variable region is substantially constant throughout different antibodies, provided that the sub-type is the same, and provided that that the amino acid sequences have some common characteristics. However, the CDR's, present between such framework regions, are sequences specific to each antibody.

By comparison of the amino acid sequence of the variable region of CH11 H chain with the sequence of mouse µ2a sub type, the CDR in the CH11 H chain was shown to be represented by amino acid Nos. 31 to 35 of SEQ ID NO. 8 (CDRH_{1,} corresponding to SEQ ID NO. 11 of the sequence listing), 50 to 66 of SEQ ID NO. 8 (CDRH_{2,} corresponding to SEQ ID NO. 2 of the sequence listing) and 99 to 105 of SEQ ID NO. 8 (CDRH_{3,} corresponding to SEQ ID NO. 3 of the sequence listing).

When the amino acid sequence of the variable region of CH11 L chain was compared to the sequence of the mouse κ2 sub-type, the CDR of the L chain was shown to be represented by the amino acid Nos. 24 to 39 of SEQ ID NO. 10 (CDRL_{1,} corresponding to SEQ ID NO. 4 of the sequence listing), 55 to 61 of SEQ ID NO. 10 (CDRL_{2,} corresponding to SEQ ID NO. 5 of the sequence listing) and 94 to 102 of SEQ ID NO. 10 (CDRL_{3,} corresponding to SEQ ID NO. 6 of the sequence listing).

When the amino acid sequence of the variable region of CH11 L chain was compared to the sequence of the mouse κ2 sub-type, the CDR of the L chain was shown to be represented by the amino acid Nos. 24 to 39 (CDRL_{1,} corresponding to SEQ ID NO. 4 of the sequence listing), 55 to 61 (CDRL_{2,} corresponding to SEQ ID NO. 5 of the sequence listing) and 94 to 102 (CDRL_{3,} corresponding to SEQ ID NO. 6 of the sequence listing) of SEQ ID NO. 10 of the sequence listing.

The present invention is further illustrated by the following Examples, the Examples being illustrative of, but not binding upon, the present invention.

### EXAMPLE 1

### Molecular modelling of the variable regions of CH11

Molecular modelling of the variable regions of CH11 was carried out by the method of 'homology modelling' [Andrew *et al.,* (1991) Methods in Enzymology, *203*, p. 121-153].

The primary sequences of variable regions of human immunoglobulins for which the X-ray crystal structure has been determined are registered in the Protein Data Bank (hereinafter referred to as "PDB"; Chemistry Department, Building 555, Brookhaven National Laboratory, P. O. Box 5000, Upton, NY 11973-5000, USA). The sequences contained in the Data Bank were compared with the sequence of the framework regions of CH11. Two human immunoglobulins, 1NBV and 1IGI, were identified as having the highest degree of homology with the CH11 L Land H chains, respectively.

A model of the three-dimensional structure of the framework regions of CH11 was constructed based upon the known structure of these human FR regions. This model is hereinafter referred to as the "framework model".

The CDRs of CH11 were classified using the method of Chothia *et al.* [Chothia *et al,* J. Mol. Biol., (1987), *196*, 901-917]. Using this method, CDRL₁ was classified into the canonical class 4, CDRL₂ into the canonical class 1, CDRL₃ into canonical class 1, and CDRH₁ into canonical class 1. CDRH₂ and CDRH₃ did not correspond to a defined canonical class. The CDR loops of CDRL₁, CDRL₂, CDRL₃ and CDRH₁ were given the conformations inherent to the respective canonical classes, and then integrated into the framework model.

The conformations of CDRH₂ and CDRH₃ were determined as follows. First, sequences with high homologies to these CDR's were identified from the PDB. The conformation of CDRH₂ and CDRH₃ were modelled upon the conformations of these known sequences. These conformations were combined with results of energy calculation, and the conformations of the CDR loops with the highest probabilities were constructed and integrated into the framework model. Finally, an energy calculation was carried out to eliminate any energetically unfavourable atomic contacts, in order to obtain a molecular model of CH11. The above procedure was performed using the AbM molecular modelling software [Oxford Molecular Limited, Inc.].

The accuracy of the structure of the molecular model obtained was evaluated using the PROCHECK software, [Laskowski, R. A. J., (1993), Appl. Cryst. *26*, 283-291]. The degree of surface exposure of each residue was calculated using the method of Lee and Richards [Lee, B., and Richards, F. M., J. Mol. Biol., (1971), *55*, 379 - 400], allowing the degree of contact between atoms to be determined.

### EXAMPLE 2

### Selection of the acceptors

The sequence of the H and L chains of CH11 was compared with the consensus sequences of the respective subgroups of human antibodies. The L chain of CH11 was found to have 83 % identity with human subgroup kappa II and the H chain of CH11 was found to have 74 % identity with human subgroup I. The human antibodies RPMI6410'CL (subgroup κ II) and 21•28'CL (subgroup I) were selected as the acceptor molecules for the L and H chains, respectively, on the basis of sequence homology.

### EXAMPLE 3

### Selection of donor residues from CH11 to be grafted onto the acceptors

The amino acid sequence of each of the H and L chains of CH11 was aligned with that of the respective acceptor molecule using 'Cameleon' software [Oxford Molecular Limited, Inc.]. Humanised sequences were designed according to criteria (a) to (d), described above. Four light chain sequences and two heavy chain sequences were designed, with which to form the basis for producing humanised anti-human Fas antibodies. These amino acid sequences and the corresponding nucleotide sequences coding for these proteins are listed below.

### L chains (κ chains):

polypeptide VL-KY (SEQ ID No. 78) and its encoding DNA sequence (SEQ ID No. 77);
polypeptide VL-KF (SEQ ID No. 80) and its encoding DNA sequence (SEQ ID No. 79);
polypeptide VL-RY (SEQ ID No. 82) and its encoding DNA sequence (SEQ ID No. 81); and
polypeptide VL-RF (SEQ ID No. 84) and its encoding DNA sequence (SEQ ID No. 83).

### H chains (µ chains):

polypeptide HµH chain (SEQ ID No. 86) and its encoding DNA sequence (SEQ ID No. 85); and
polypeptide HµM chain (SEQ ID No. 88) and its encoding DNA sequence (SEQ ID No. 87).

### EXAMPLE 4

### Cloning and sequencing of DNA encoding the full-length human H and L chains (having subgroups I and II, respectively, in the variable regions)

### 1) Preparation of the primers

### a) H chain

The amino acid sequence of the variable region of the H chain of the mouse monoclonal antibody CH11 (SEQ ID No. 89) was compared with the database of amino acid sequences of antibodies produced by Kabat *et al.* [Kabat E. A., *et al., supra*], in order to identify any homologous sequences. The amino acid sequence of the framework regions of the variable region of the H chain (µ chain) of CH11 was found to be homologous to the H chain of human antibody subgroup I. Thus, the oligonucleotide primer:
HVHI5-1; (SEQ ID No. 90)
was synthesised, that hybridises with a portion of the 5' - untranslated region of DNA coding for the human immunoglobulin H chain subgroup I in the database.

The nucleotide sequence of DNA coding for the constant region of human immunoglobulin H chain has been reported by Dorai and Gillies [(1989), Nucleic Acids Res., *17*, 6412]. Based upon this, the oligonucleotide primer
HCµ3-1; (SEQ ID No. 91)
was synthesised, that hybridises with a portion of the nucleotide sequence of the 3' - untranslated region

### b) L chain

The amino acid sequence of the variable region of the L chain of the mouse monoclonal antibody CH11 (SEQ ID No. 92) was compared with the database of amino acid sequences of antibodies produced by Kabat *et al*. [*supra*], in order to identify any homologous sequences. It was found that the amino acid sequence of the framework regions of the variable region of the L chain (κ chain) of CH11 was homologous to the L chain of human antibody subgroup II. Thus, the oligonucleotide primer:
HVK II5-4; (SEQ ID No. 93)
was synthesised, that hybridises with a portion of the 5' -untranslated region of DNA coding for the human immunoglobulin L chain subgroup II in the database.

The nucleotide sequence of DNA coding for the constant region of human immunoglobulin L chain has been reported by Hieter *et al*. [Hieter, P. A., *et al.* (1980), Cell, *22*, 197 *et seq*]. Based upon this, the oligonucleotide primer:
HKCL3-3; (SEQ ID No. 94)
was synthesised, that hybridises with a portion of the 3'-untranslated region of DNA.

The above oligonucleotide primers were all synthesised by the phosphoamidide method [Mattrucci, M. D., and Caruthers, M. H., (1981) J. Am. Chem. Soc., *103*, 3185 *et seq*] using the automated DNA synthesiser Model 380B [Perkin Elmer, Japan]. After synthesis, each primer was dissociated from the support, deprotected, and then lyophilised. The primers were dissolved in 100 µl of distilled water and stored at -20 °C until used.

### 2) Amplification of the target gene by the polymerase chain reaction (PCR).

### H chain

The DNA fragment coding for the H chain of human IgM was amplified and isolated using PCR. A human lymphocyte cDNA library was used as the starting source of DNA.

Specifically, the reaction solution defined below was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

Composition of the reaction solution:
human lymphocyte cDNA library [Life Technologies], 25 ng;
oligonucleotide primer HVHI5-1, 50 pmol;
oligonucleotide primer HCµ3-1, 50 pmol;
25 mM dNTPs cocktail, 10 µl;
100 mM Tris-HCl buffer (pH 8.5), 10 µl;
1 M potassium chloride [KCI], 5 µl;
25 mM magnesium chloride [MgCl₂], 10µl;
Taq DNA polymerase [Perkin Elmer Japan], 1 unit.

The total volume was adjusted to a final volume of 100 µl by adding redistilled water. The term '25 mM dNTPs cocktail' refers to a cocktail of "dNTPs" ('deoxynucleotide triphosphates) comprising dATP (deoxyadenosine triphosphate), dCTP (deoxycytosine triphosphate), dGTP (deoxyguanosine triphosphate) and dTTP (deoxythymidine triphosphate), each at a concentration of 25mM.

### L chain

The DNA fragment coding for L chain of human IgM was amplified and isolated using the polymerase chain reaction. A human lymphocyte cDNA library was used as the starting source of DNA.

Specifically, the reaction solution defined below was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

Composition of the reaction solution:
human lymphocyte cDNA library [Life Technologies], 25 ng;
oligonucleotide primer HVKII5-4, 50 pmol;
oligonucleotide primer HKCL3-3, 50 pmol;
25 mM dNTPs cocktail, 10 µl;
100 mM Tris-HCl buffer (pH 8.5), 10 µl;
1 M potassium chloride [KCl], 5 µl;
25 mM magnesium chloride [MgCl₂], 10µl;
Taq DNA polymerase [Perkin Elmer Japan], 1 unit.
The total volume was adjusted to a final volume of 100 µl by adding redistilled water.

### 3) Assay for PCR products

After PCR amplification, the products of each reaction were analysed by agarose gel electrophoresis. Aliquots of each reaction solution described in section (2), above, corresponding to 200 ng of DNA, were electrophoresed on a 0.8 % (w/v) agarose gel. The size of the PCR product was assessed relative to the mobilities of bands of molecular markers run in parallel with the samples. The human immunoglobulin H chain fragment was found to be approximately 2,000 base pairs (hereinafter abbreviated as "bp") in size, and the human immunoglobulin L chain was found to be approximately 800 bp in size.

### 4) Cloning of the PCR products

Each of the PCR products obtained in section 3, above, was ligated into a plasmid vector using a eukaryote TA Cloning Kit [Invitrogen].

More specifically, 60 ng of pCR3 vector DNA (included in the kit) and four units of T4 DNA ligase were added to ligase reaction buffer [6 mM Tris-HCI (pH 7.5), 6 mM magnesium chloride (MgCl₂), 5 mM sodium chloride (NaCl), 7 mM β-mercaptoethanol, 0.1 mM ATP, 2 mM DTT_{,} 1 mM spermidine, and 0.1 mg/ml bovine serum albumin], containing a proportion of the PCR reaction mixture. The volume of the PCR reaction mixture was selected such that it contained about 10 ng of the desired PCR product. The resulting mixture was incubated at 14 °C for 15 hours.

A portion of the ligase reaction mixture (2 µl) was mixed with 50 µl of *E. coli* cells, strain TOP10F' (included in the kit), made competent by the addition of 2 µl of 0.5 M β-mercaptoethanol. The resulting mixture was kept on ice for 30 minutes. SOC medium, 500 µl (included in the kit) was added, and the resulting mixture was incubated at 37 °C for 1 hour with shaking. The culture liquid was then spread onto L-broth agar medium plates [1 % (w/v) bactotrypton (Difco), 0.5 % (w/v) bacto-yeast extract (Difco), 0.1 % (w/v) glucose, 0.5 % (w/v) NaCl, 1.2 % (w/v) bacto-agar (Difco)] containing 100 µg/ml ampicillin and incubated at 37 °C overnight.

Ampicillin resistant colonies which appeared were then scraped off with a platinum pick, and individually cultured in 5 ml of liquid L-broth medium [1 % (w/v) bactotrypton (Difco), 0.5 % (w/v) bacto-yeast extract (Difco), 0.5 % (w/v) NaCI] containing 100 µg/ml ampicillin at 37 °C overnight with shaking. These cultures were then centrifuged to harvest the cells, from which plasmid DNA was prepared by the alkaline lysis method [Sambrook, J. *et al. supra*].

Plasmid DNA (1µg) prepared in this way was digested with the restriction enzyme EcoR1, using the buffer supplied with the enzyme. All restriction digests carried out hereinafter were carried out used the buffer supplied with the enzyme [Takara Shuzo]. In the case of a double digest, a restriction buffer was used that was compatible with both enzymes. The digestion products were separated by electrophoresis on a 0.8 % (w/v) agarose gel. Plasmids containing DNA inserts of approximately 2,000 bp and approximately 800 bp, corresponding to the human immunoglobulin H and L chains, respectively, were identified, by comparison with molecular markers run on the same gel. Plasmids containing these fragments were selected.

Specifically, the following two plasmids were selected:

Plasmid pHH1-5, containing a DNA fragment encoding the human immunoglobulin H chain. Specifically, the plasmid contains a cDNA insert encoding the human immunoglobulin H chain having a variable region of subgroup I.

Plasmid pHL15-27, containing a DNA fragment encoding the human immunoglobulin L chain. Specifically, the plasmid contains a cDNA insert encoding the human immunoglobulin L chain having a variable region of subgroup II.

### 5) Verification of the cloned full-length nucleotide sequences of cDNA coding for human immunoglobulin H and L chains

A human immunoglobulin H chain consists of an N-terminal variable region of about 110 residues and an adjacent constant region of about 510 residues. On the other hand, a human immunoglobulin L chain consists of an N-terminal variable region of about 110 residues and an adjacent constant region of about 107 residues.

Therefore, the nucleotide sequence of the cDNA encoding the H chain of human immunoglobulin, cloned in the section (4) above, was predicted to consist of a variable region and a constant region. The variable region was predicted to be highly homologous to the variable region of a human immunoglobulin H chain sequence of subgroup I [for example, clone 21/28'CL; Kabat E. A., *et al.* (1991), *supra*]. The nucleotide sequence coding for the constant region of human immunoglobulin H chain is known [Kabat E. A., *et al.,* (1991), *supra*].

The nucleotide sequence of the cDNA encoding the L chain of human immunoglobulin, cloned in the section (4) above, was predicted to consist of a variable region and a constant region. The variable region was predicted to be highly homologous to the variable region of a human immunoglobulin L chain of subgroup II (for example, clone RPMI1640'CL; Kabat E. A., *et al*., *supra*). The nucleotide sequence coding for the constant region of human immunoglobulin L chain is known [Kabat E. A., *et al*., *supra*].

Oligonucleotide primers of 20 nucleotides in length were synthesised, in order to carry out sequence analysis. The primers were designed based on known well-conserved sequences within the framework regions of the variable regions and known nucleotide sequences within the constant regions. The primers were designed to correspond to sequences separated by 100 to 200 bp intervals, and were used in conjunction with the primers HVHI5-1, HCµ3-1, HVKII5-4 and HKCL3-1, already used in the PCR (section 2, above).

The sequences of the oligonucleotide primers synthesised for sequence analysis of the H chain are as follows:
SHHF-1; (SEQ ID No. 95);
SHHF-2; (SEQ ID No. 96);
SHHF-3; (SEQ ID No. 97);
SHHF-4; (SEQ ID No. 98);
SHHF-5; (SEQ ID No. 99);
SHHF-6; (SEQ ID No. 100);
SHHF-7; (SEQ ID No. 101);
SHHF-8; (SEQ ID No. 102);
SHHF-9; (SEQ ID No. 103);
SHHF-10; (SEQ ID No. 104);
SHHF-11; (SEQ ID No. 105);
SHHF-13; (SEQ ID No. 106);
SHHF-14; (SEQ ID No. 107);
SHHF-15; (SEQ ID No. 108);
SHHR-1; (SEQ ID No. 109);
SHHR-2; (SEQ ID No. 110);
SHHR-3; (SEQ ID No. 111);
SHHR-4; (SEQ ID No. 112);
SHHR-5; (SEQ ID No. 113);
SHHR-6; (SEQ ID No. 114);
SHHR-7; (SEQ ID No. 115);
SHHR-8; (SEQ ID No. 116);
SHHR-9; (SEQ ID No. 117);
SHHR-10; (SEQ ID No. 118);
SHHR-11; (SEQ ID No. 119);
SHHR-12; (SEQ ID No. 120);
SHHR-13; (SEQ ID No. 121);
SHHR-14; (SEQ ID No. 122); and
SHHR-15; (SEQ ID No. 123).

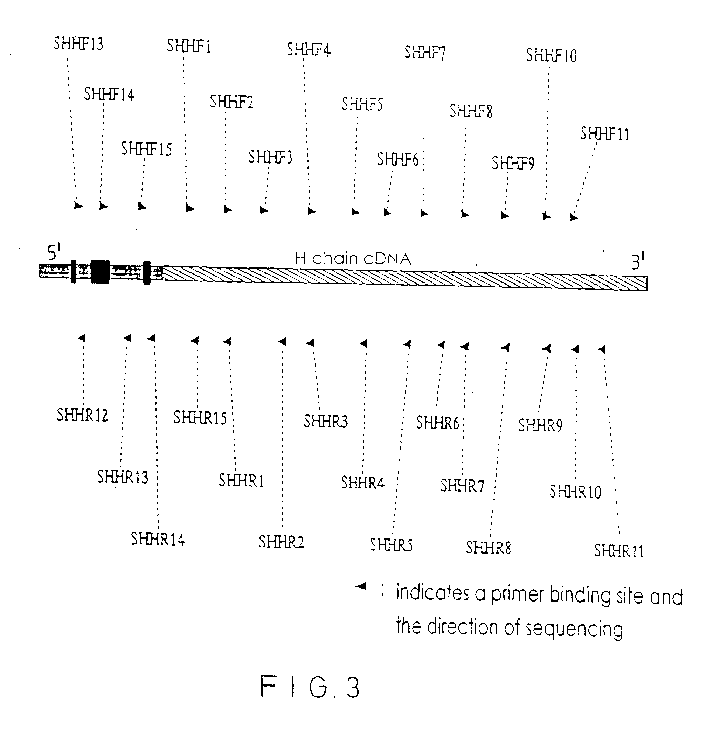

Figure 3 indicates the positions to which the respective primers bind.

The sequences of the oligonucleotide primers synthesised for sequence analysis of the L chain are as follows:
SHKF-1; (SEQ ID No. 124);
SHKF-2; (SEQ ID No. 125);
SHKF-4; (SEQ ID No. 126);
SHKF-5; (SEQ ID No. 127);
SHKF-6; (SEQ ID No. 128);
SHKF-11; (SEQ ID No. 129);
SHKF-12; (SEQ ID No. 130);
SHKR-1; (SEQ ID No. 131);
SHKR-2; (SEQ ID No. 132);
SHKR-3; (SEQ ID No. 133);
SHKR-4; (SEQ ID No. 134);
SHKR-6; (SEQ ID No. 135); and
SHKR-13; (SEQ ID No. 136).

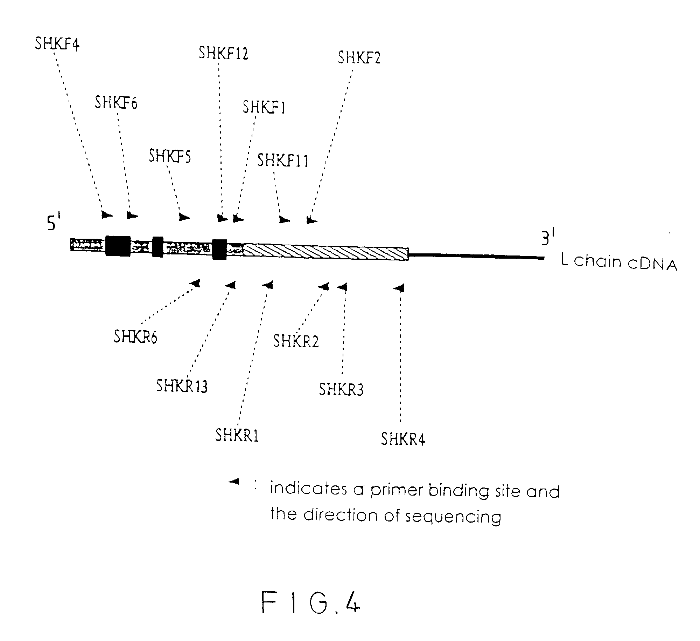

Figure 4 indicates the positions to which the respective primers bind.

Samples for sequence analyses were prepared using the above primers and the Prism Ready Reaction Terminator Cycle Sequencing kit [Perkin Elmer, Japan]. Plasmid DNA from plasmids pHH1-5 DNA or plasmid pHL 15-27 DNA, as described in the section 4, above, was used as a template.

Specifically, purified plasmid DNA (1.5 µg) was mixed with 4.8 pmol of an appropriate primer, made up to a final volume of 16 µl with distilled water (this solution is hereinafter referred to as the "plasmid DNA/primer mixture"). A portion of this plasmid DNA/primer mixture (9.5 µl), corresponding to each primer, was added to 10.5 µl of the premix solution provided in the kit, containing Taq DNA polymerase. The reaction solution was placed in an automated reactor [Catalyst; Perkin Elmer Japan]. The reaction cycle used was as follows: a thermal cycle of 95 °C for 30 seconds, 50 °C for 15 seconds, and 60 °C for 4 minutes, repeated 25 times.

After completion of the reaction cycles, 80 µl of distilled water was added to the resulting solutions and the DNA in the resulting mixture was extracted twice by the phenol-chloroform method [Sambrook *et al., supra*]. The recovered aqueous layer was mixed with 15 µl of 2 M sodium acetate and 300 µl of 100% ethanol, followed by centrifugation to recover the DNA precipitate. The precipitate was washed with 70 % (v/v) ethanol and dried under reduced pressure, then dissolved in 3 µl of the sample solution [4 µl of 0.25 M EDTA. 100 µl of formamide and 16 µl of distilled water].

The sequencing reactions were run and analysed on a DNA sequencer [Model 373A; Perkin Elmer Japan]. Analysis was carried out on 30 samples for the human immunoglobulin H chain and 17 samples for the human immunoglobulin L chain.

Analysis of the data verified that plasmid pHH1-5 contained a DNA insert encoding a human immunoglobulin H chain with a variable region of subgroup I. On the other hand, plasmid pHL15-27 was shown to contain a DNA insert encoding a human immunoglobulin L chain with a variable region of subgroup II.

The nucleotide sequences of the DNA inserts carried by plasmid pHH1-5 and plasmid PHL 15-27 are shown as SEQ ID Nos. 137 and 138, respectively.

### EXAMPLE 5

### Construction of expression vectors for humanised versions of CH11 L chain

### 1) Preparation of the primers

The following DNA fragments were synthesised using PCR:
DNA (SEQ ID No. 77) coding for the polypeptide chain of VL-KY chain (SEQ ID No. 78),
DNA (SEQ ID No. 79) coding for the polypeptide chain of VL-KF chain (SEQ ID No. 80),
DNA (SEQ ID No. 81) coding for the polypeptide chain of VL-RY chain (SEQ ID No 82),
DNA (SEQ ID No. 83) coding for the polypeptide chain of VL-RF chain (SEQ ID No. 84).

The following 14 primers were synthesised for use in the PCR process:
VL1P; (SEQ ID No. 139);
VL1N; (SEQ ID No. 140);
VL2P; (SEQ ID No. 141);
VL2N; (SEQ ID No. 142);
VL3TYRP; (SEQ ID No. 143);
VL3TYRN; (SEQ ID No. 144);
VL3PHEP; (SEQ ID No. 145);
VL3PHEN; (SEQ ID No. 146);
VL4P; (SEQ ID No. 147);
VL4N; (SEQ ID No. 148);
VL5P; (SEQ ID No. 149);
VL50RP; (SEQ ID No. 150);
VL50RN; (SEQ ID No. 151); or
VLTERM; (SEQ ID No. 152).

### 2) Construction of plasmid pHκY2-58 and plasmid pHκKF2-19

### a) First PCR step

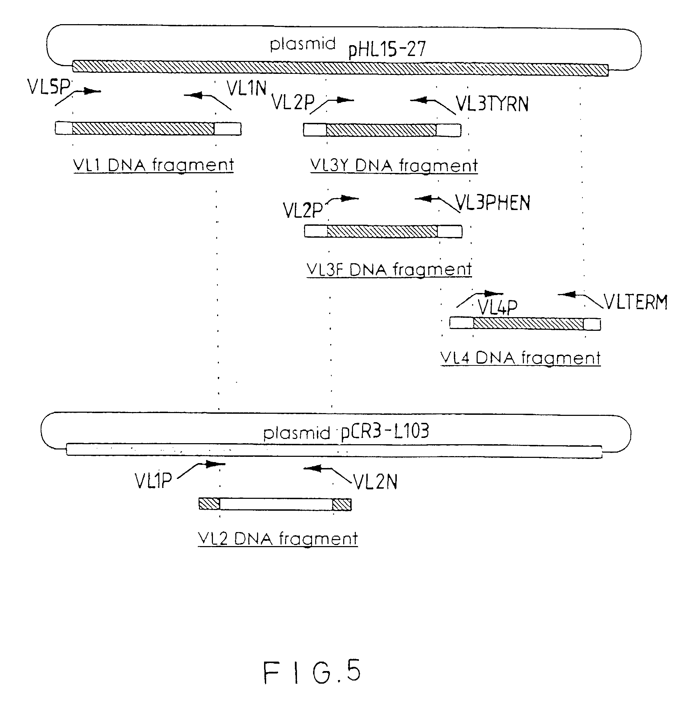

The outline of the first PCR step is shown in Figure 5.

### VL1

A DNA fragment was prepared encoding a secretion signal sequence, the FRL₁ region and the amino-terminal portion (hereinafter referred to as the "N-terminus") of the CDRL₁ region. This fragment is herein referred to as the "VL1 DNA fragment". The PCR reaction conditions were as follows:
Composition of the reaction solution:
plasmid pHL 15-27 DNA, 1 µg;
oligonucleotide primer VL5P, 80 pmol;
oligonucleotide primer VL1N, 80 pmol;
25 mM dNTPs cocktail, 20 µl;
10x Pfu buffer, 20 µl;
Pfu DNA polymerase [Stratagene], 10 units.

Redistilled water was added to a final volume of 200 µl. The10x Pfu buffer was provided with the Pfu polymerase.

Specifically, the reaction solution was initially, heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL2

A DNA fragment was prepared encoding the carboxyl-terminal portion (hereinafter referred to as the "C-terminus") of the FRL₁ region, the CDRL₁ region and the N-terminus of the FRL₂ region. This fragment is herein referred to as the "VL2 DNA fragment". The PCR reaction conditions were as follows:
Composition of the reaction solution:
plasmid pCR3-L103 DNA, 1 µg;
oligonucleotide primer VL1P, 80 pmol;
oligonucleotide primer VL2N, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL3Y

A DNA fragment was prepared encoding the CDRL₂ region, the FRL₃ region (in which the amino acid residue at position 87 had been altered to a tyrosine residue) and the CDRL₃ region. In this and all other examples, the amino acid numbering follows that given in Kabat [Kabat *et al., supra*]. This fragment is herein referred to as the "VL3Y DNA fragment". The PCR reaction conditions were as follows:
Composition of the reaction solution:
plasmid pHL 15-27 DNA, 1 µg;
oligonucleotide primer VL2P, 80 pmol;
oligonucleotide primer VL3TYRN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL3F

A DNA fragment was prepared encoding the CDRL₂ region, FRL₃ region (in which the amino acid residue at position 87 had been altered to a phenylalanine residue) and the CDRL₃ region. This fragment is herein referred to as the "VL3F DNA fragment". The PCR reaction conditions were as follows:
Composition of the reaction solution:
plasmid pHL 15-27 DNA, 1 µg;
oligonucleotide primer VL2P, 80 pmol;
oligonucleotide primer VL3PHEN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL4

A DNA fragment was prepared encoding the CDRL₃ region, the FRL₄ region, and Ck region (a portion of a constant region). This fragment is herein referred to as the "VL4 DNA fragment". The PCR reaction conditions were as follows:
Composition of the reaction solution:
plasmid pHL 15-27 DNA, 1 µg;
oligonucleotide primer VL4P, 80 pmol;
oligonucleotide primer VLTERM, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The VL1, VL2, VL3Y, VL3F and VL4 DNA fragments amplified by PCR in this way were extracted with phenol. The DNA was then precipitated using 100% ethanol. The DNA (approximately 20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### b) Second step PCR

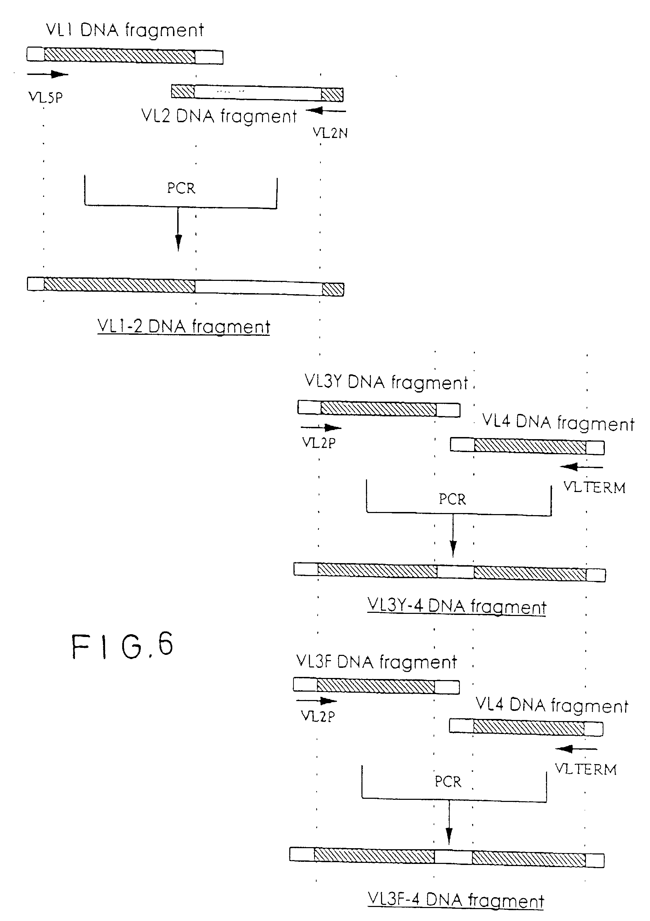

The outline of the second step PCR is shown in Figure 6.

### VL1-2

A fusion of the VL1 DNA fragment and VL2 DNA fragment, described above, was prepared using PCR. This fragment is hereinafter referred to as "VL1-2 DNA fragment". The following PCR reaction conditions were used:
Composition of the reaction solution:
VL1 DNA solution prepared in the first step PCR, 10 µl;
VL2 DNA solution prepared in the first step PCR, 10 µl;
oligonucleotide primer VL5P, 80 pmol;
oligonucleotide primer VL2N, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL3Y-4

A fusion of the VL3Y DNA fragment and VL4 DNA fragment, described above, was prepared using PCR. This fragment is hereinafter referred to as the "VL3Y-4 DNA fragment". The following PCR reaction conditions were used:
Composition of the reaction solution:
VL3Y DNA solution prepared in the first step PCR, 10 µl;
VL4 DNA solution prepared in the first step PCR, 10 µl;
oligonucleotide primer VL2P, 80 pmol;
oligonucleotide primer VLTERM, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL3F-4

A fusion of the VL3F DNA fragment and VL4 DNA fragment, described above, was prepared using PCR. This fragment is hereinafter referred to as "VL3F-4 DNA fragment". The following PCR reaction conditions were used:
Composition of the reaction solution:
VL3F DNA solution prepared in the first step PCR, 10 µl;
VL4 DNA solution prepared in the first step PCR, 10 µl;
oligonucleotide primer VL2P, 80 pmol;
oligonucleotide primer VLTERM, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The VL1-2, VL3Y-4 and VL3F-4 DNA fragments amplified by PCR in this way were extracted with phenol. The DNA was then precipitated using 100% ethanol. The DNA (approximately 20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### c) Third step PCR

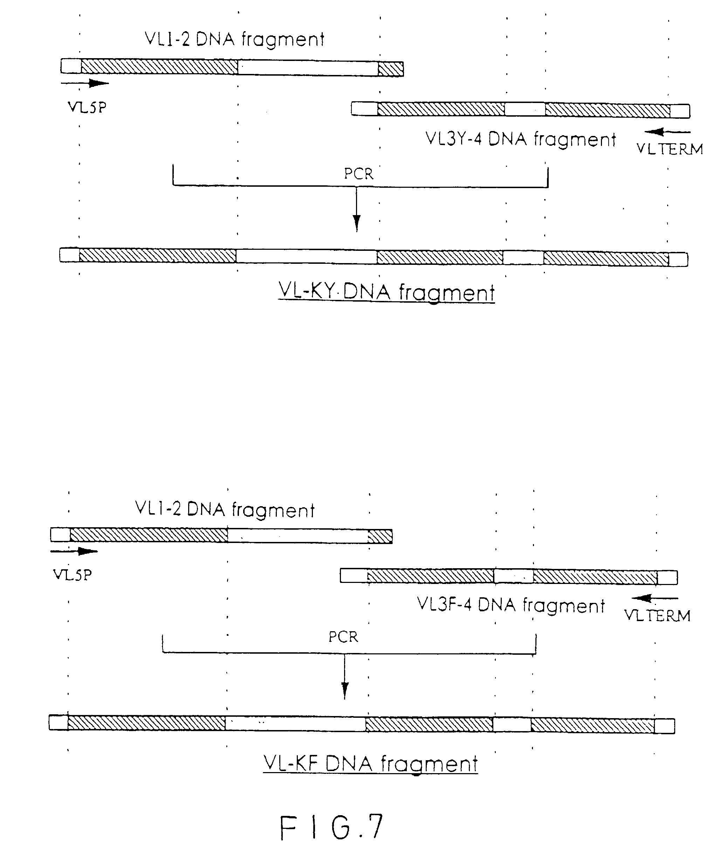

The outline of the third step PCR is shown in Figure 7.

### VL-KY

A fusion of the VL1-2 DNA fragment and VL3Y-4 DNA fragment, described above, was prepared using PCR. This fragment is hereinafter referred to as "VL-KY DNA fragment". The following PCR reaction conditions were used:
Composition of the reaction solution:
VL1-2 DNA solution prepared in the second step PCR, 10 µl;
VL3Y-4 DNA solution prepared in the second step PCR, 10 µl;
oligonucleotide primer VL5P, 80 pmol;
oligonucleotide primer VLTERM, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL-KF

A fusion of the VL1-2 DNA fragment and VL3F-4 DNA fragment described above was prepared using PCR. This fragment is hereinafter referred to as "VL-KF DNA fragment". The following PCR reaction conditions were used:
Composition of the reaction solution:
VL1-2 DNA solution prepared in the second step PCR, 10 µl;
VL3F-4 DNA solution prepared in the second step PCR, 10 µl;
oligonucleotide primer VL5P, 80 pmol;
oligonucleotide primer VLTERM, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The amplified VL-KY and VL-KF DNA fragments amplified by PCR in this way were extracted with phenol. The DNA was then precipitated using 100% ethanol. The DNA - (approximately 20-30 µg was electrophoresed on a 5% (w/v) polyacrylamide gel. The gel was stained with 1 µg/ml of ethidium bromide, such that the DNA fragments were visible when viewed under UV light. The DNA fragments detected in this way where excised from the gel with a razor blade and electro-eluted from the acrylamide gel using a Centriruter [Amicon] equipped with Centricon 10 [Amicon].

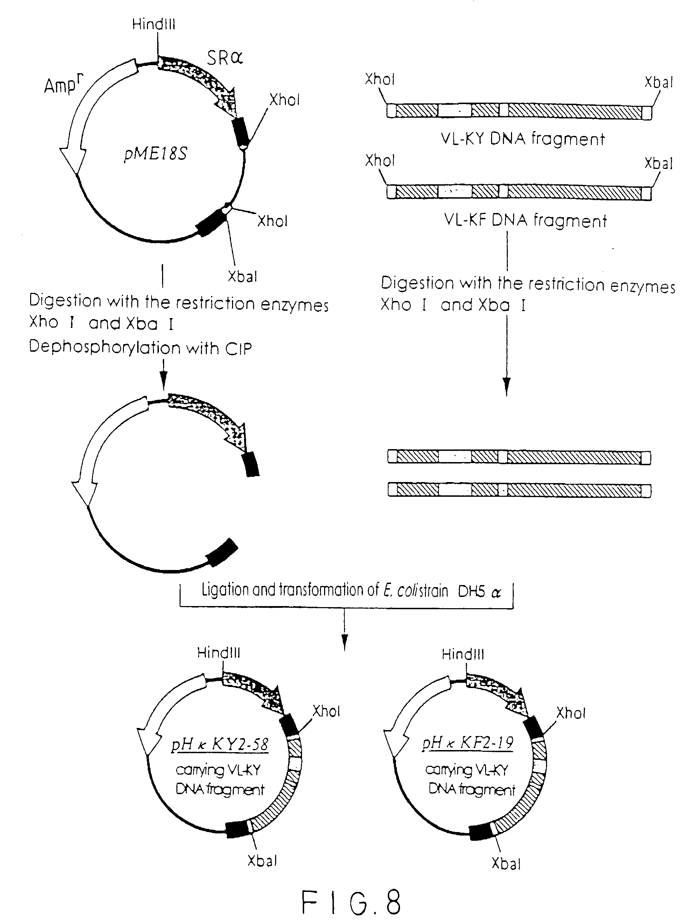

The construction of a plasmid carrying VL-KY or VL-KF DNA fragment is outlined in Figure 8.

The VL-KY and VL-KF DNA obtained in this way was further purified by phenol extraction, followed by ethanol precipitation. A portion of the DNA (approximately 1 µg) was then digested with the 10 units of restriction enzymes Xho1 and Xba1, at 37°C, using a compatible restriction buffer supplied with the enzymes.

A portion (1 µg) of plasmid pME18S DNA [Hara, T. and Miyajima, A., (1992), EMBO J., *11,* 1875] was also digested with the restriction enzymes Xho1 and Xba1. The resulting DNA was treated with calf intestine alkaline phosphatase (hereinafter abbreviated as "CIP"; Takara Shuzo] in order to remove any 5' phosphate groups. A portion (100 ng) of the dephosphorylated pME18S plasmid DNA was ligated to 0.5 µg of each of the Xba-1, Xho1 digested VL-KY and VL-KF DNA fragments. Ligation was carried out using a ligation kit [Takara Shuzo], and the ligation product was transformed into *E*. *coli* strain DH5α [Gibco-BRL] by electroporation.

Specifically, 50 µl of competent cells were thawed and mixed with 5 µl of the ligation mix. The mixture was transferred into an electroporation cuvette [BioRad]. One pulse of 25µF, 1.8kV and 200 Ω was applied. After the pulse, the cells were resuspended in 1 ml of SOC medium. The cell suspensions were transferred into a sterile tube and incubated at 37°C for 1 hour. The resulting cells were plated onto LB plates containing 50 µg of ampicillin.

Restriction analysis of plasmid DNA contained in transformant colonies was carried out, to identify plasmids containing the DNA insert of interest. Specifically, plasmid DNA was prepared from an overnight culture of transformant cells by the method given in Working Example 4, section 4. Plasmid DNA was digested with the original restriction enzymes, (Xho1 and Xba1 in the present Example) in order to confirm that a fragment of the correct size had been cloned.

All ligation reactions, transformation and analysis of transformants, detailed hereinafter, were carried out using the methodology outlined above, except where specifically indicated.

Plasmid pHκKY2-58 was identified containing the VL-KY DNA fragment and plasmid pHκKF2-19 was identified containing the VL-KF DNA fragment. The fragments in both cases were inserted downstream of the SRα promoter in pME18S, in the correct orientation for expression of the immunoglobulin protein product.

### 3) Construction of plasmid pHκRY2-10 and plasmid pHκRF2-52

Using DNA from the plasmids pHκKY2-58 DNA and pHκKF2-19 DNA as a template, two further expression vectors were constructed.

### a) First step PCR

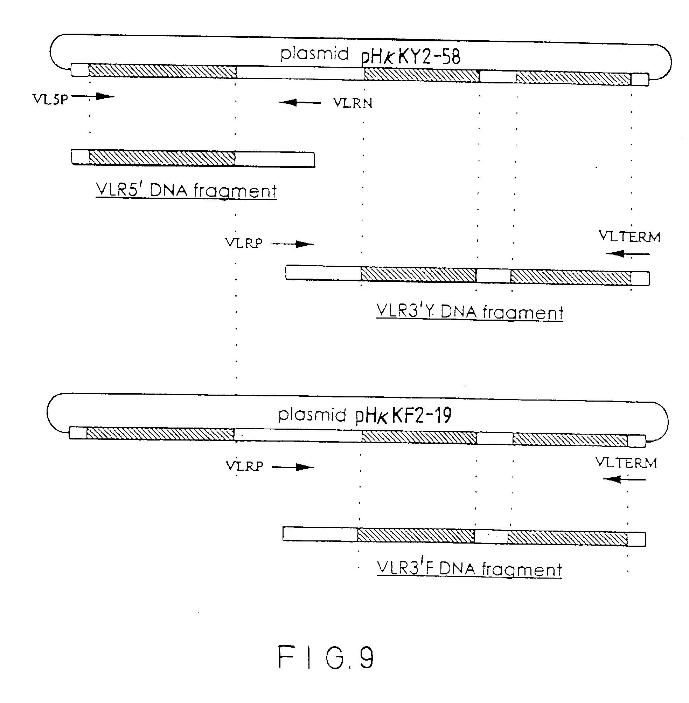

The outline of the first step PCR is shown in Figure 9.

### VLR5'

A DNA fragment was prepared encoding a secretion signal sequence, the FRL₁ region, the CDRL₁ region and FRL₂ region (in which the lysine residue at position 45 was substituted for an arginine residue). This fragment is herein referred to as the "VLR5' DNA fragment". The PCR reaction conditions were as follows:
Composition of the reaction solution:
plasmid pHκKY2-58 DNA, 1 µg;
oligonucleotide primer VL5P, 80 pmol;
oligonucleotide primer VLRN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VLR3'Y

A DNA fragment was prepared encoding the FRL₂ region (in which the lysine residue at position 45 was substituted for an arginine residue), the CDRL₂ region, the FRL₃ region (in which position 87 was a tyrosine residue), the FRL₄ region and a Ck region. This fragment is herein referred to as the "VLR3'Y DNA fragment". The PCR reaction conditions were as follows:
Composition of the reaction solution
plasmid pHκKY2-58 DNA, 1 µg;
oligonucleotide primer VLRP, 80 pmol;
oligonucleotide primer VLTERM, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VLR3'F

A DNA fragment was prepared encoding the FRL₂ region (wherein the lysine residue of position 45 was substituted for an arginine residue ), the CDRL₂ region, the FRL₃ region (wherein position 87 was a phenylalanine residue), CDRL₃ region, FRL₄ region and the Ck region. This fragment is herein referred to as the "VLR3'F DNA fragment". The PCR reaction conditions were as follows:
Composition of the reaction solution:
plasmid pHκKF2-19 DNA, 1 µg;
oligonucleotide primer VLRP, 80 pmol;
oligonucleotide primer VLTERM, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The VLR5', VLR3'Y and VLR3'F DNA fragments amplified by PCR in this way were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### b) Second step PCR

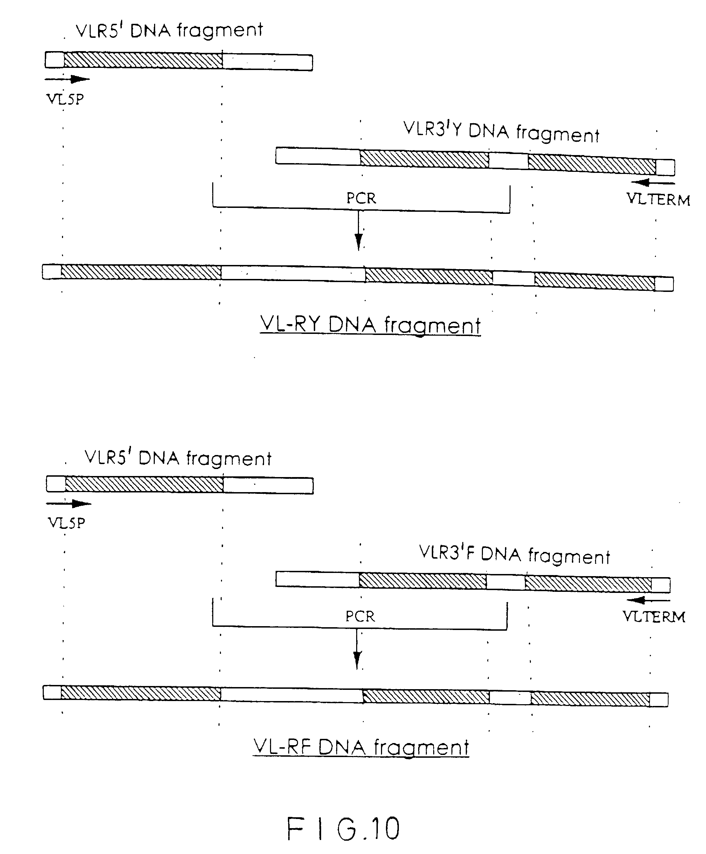

The outline of the second step PCR is shown in Figure 10.

### VL-RY

A fusion of the VLR5' DNA fragment and VLR3'Y DNA fragment described above (hereinafter referred to as "VL-RY DNA fragment") was prepared using PCR under the following conditions.
Composition of the reaction solution:
VLR5' DNA solution prepared in the first step PCR, 10 µl;
VLR3'Y DNA solution prepared in the first step PCR, 10 µl;
oligonucleotide primer VL5P, 80 pmol;
oligonucleotide primer VLTERM, 80 pmol;
25 mM dNTPs cocktail, 20 µl;
10 x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VL-RF

A fusion of the VLR5' DNA fragment and VLR3'F DNA fragment described above (hereinafter referred to as "VL-RF DNA fragment") was prepared using PCR under the following conditions:
Composition of the reaction solution:
VLR5' DNA solution prepared in the first step PCR, 10 µl;
VLR3'F DNA solution prepared in the first step PCR, 10 µl;
oligonucleotide primer VL5P, 80 pmol;
oligonucleotide primer VLTERM, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The VL-RY and VL-RF DNA fragments amplified by PCR in this way were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

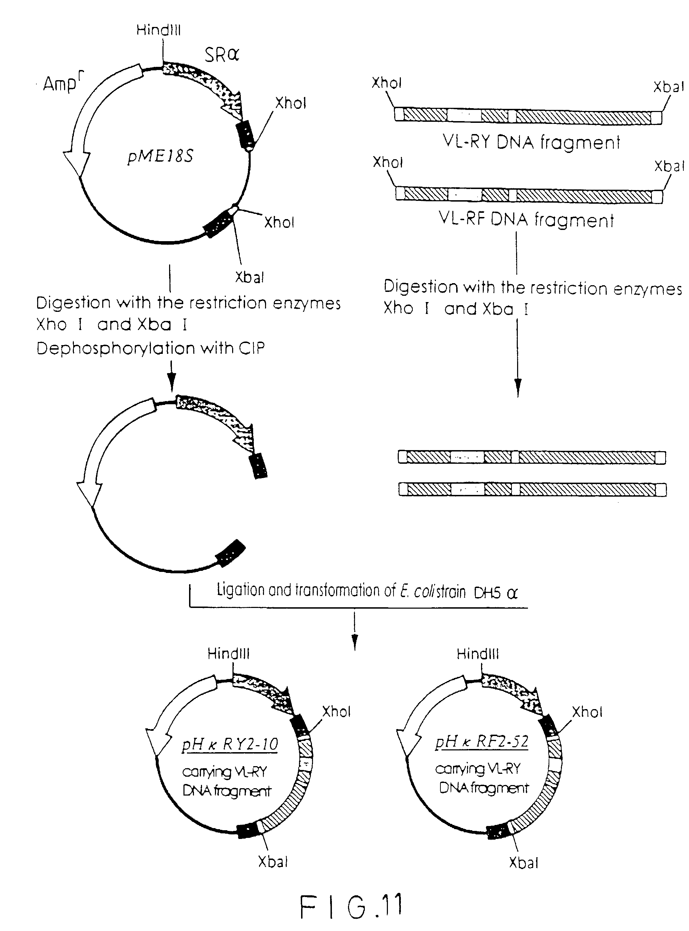

The construction of a plasmid carrying VL-RY or VL-RF DNA fragment is outlined in Figure 11.

The VL-RY and VL-RF DNA obtained in this way was further purified by phenol extraction followed by ethanol precipitation. The DNA (1 µg) was then digested with the restriction enzymes Xho1 and Xba1.

A portion (1 µg) of plasmid pME18S DNA [Hara, T. and Miyajima, A. *supra*] was also digested with the restriction enzymes Xho1 and Xba1. The resulting DNA was treated with CIP. A portion (200 ng) of the dephosphorylated pME18S plasmid DNA was ligated to 0.5 µg of each of the Xba-1, Xho1 digested VL-RY and VL-RF DNA fragments. Ligation was carried out using a ligation kit [Takara Shuzo], and the ligation product was transformed into *E. coli* strain DH5α.

Restriction analysis of plasmid DNA contained in transformant colonies was carried out, as described above, to identify plasmids containing the DNA insert of interest. Plasmid pHκRY2-10 was identified containing the VL-RY DNA fragment and plasmid pHκRF2-52 was identified containing the VL-RF DNA fragment. The fragments in both cases were inserted downstream of the SRα promoter in pME18S, in the correct orientation for expression of the immunoglobulin protein product.

### 4) Verification of the nucleotide sequences

The DNA inserts of the plasmids pHκKY2-58, pHκKF2-19, pHκRY2-10 and pHκRF2-52 were sequenced. The primers used in the sequencing process were SHKF-4, SHKF-5, SHKF-6, SHKF-12, SHKR-13, SHKF-11, SHKF-2 and SHKR-3, described above. In addition, three new primers were synthesised;
PMEF2; (SEQ ID No. 153);
SHKF-14; (SEQ ID No. 154); and
PMER2; (SEQ ID No. 155).

DNA sequencing was performed using the dideoxynucleotide chain termination method [Sanger, F. S. *et al.* (1977) Proc. Natl. Acad. Sci. USA 74:5463]. Prior to sequencing, the plasmid DNA template was isolated from the host cells by alkaline-SDS lysis [Sambrook, J. *et al*., *supra*] and the DNA purified using caesium chloride centrifugation [Sambrook, J. *et al.*, *ibid*.].

Specifically, a portion of purified plasmid DNA (1 µg) was dissolved in 16 µl of redistilled water. The solution was mixed with 2 µl of mM EDTA and 2 µl of 2 N sodium hydroxide (NaOH), then incubated at room temperature for 5 minutes. A portion (4 µl) of 10 M ammonium acetate solution and 100 µl of 100% ethanol were then added and mixed, and the mixture was placed on dry ice for 10 minutes. The DNA in the solution was then recovered by centrifugation at 15,000 rpm for 5 minutes. The pellet obtained was washed with 80 % (v/v) ethanol and dried under reduced pressure. The dried DNA was dissolved in 7 µl of redistilled water and used for as a template for sequencing.

The nucleotide sequencing reaction was performed using the 7-Deaza-Sequenase kit, Version 2.0, Kit for dCTP [Amersham]. The whole of the plasmid solution (7 µl) was added to 1 pmol of a primer and 1 µl of reaction buffer (provided in the kit). The mixture was incubated at 65 °C for 2 minutes. The plasmid DNA was allowed to anneal with the primer by gradually cooling the mixture to room temperature. The DNA labelling reaction was carried out using [α³²P]dCTP [Amersham], following the protocol provided with the kit. The reaction product was analysed by gel electrophoresis on a 5 % (w/v) polyacrylamide gel containing 8 M urea in TBE buffer [100 mM Tris, 100 mM boric acid, 1mM EDTA, pH8.3]. The gel was dried, and the DNA sequence was identified by autoradiography.

The sequence of the DNA insert of plasmid pHκKY2-58 is shown in SEQ ID No. 77. This nucleotide sequence contains an open reading frame, which encodes a polypeptide chain having the sequence defined in SEQ ID No. 78.

The sequence of the DNA insert of plasmid pHκKF2-19 is shown in SEQ ID No. 79. This nucleotide sequence contains an open reading frame, which encodes a polypeptide chain having the sequence defined in SEQ ID No. 80.

The sequence of the DNA insert of plasmid pHκRY2-10 is shown in SEQ ID No. 81. This nucleotide sequence contains an open reading frame, which encodes a polypeptide chain having the sequence defined in SEQ ID No. 82.

The sequence of the DNA insert of plasmid pHκRF2-52 is shown in SEQ ID No. 83. This nucleotide sequence contains an open reading frame, which encodes a polypeptide chain having the sequence defined in SEQ ID No.84.

### EXAMPLE 6

### Construction of expression vectors for humanised versions of CH11 H chain

### 1) Preparation of the primers

The following DNA fragments were synthesised using PCR:
DNA (SEQ ID No. 85) coding for the polypeptide chain (SEQ ID No. 86) of HµH chain, an H chain of the humanised anti-human Fas antibody CH11; and
DNA (SEQ ID No. 87) coding for the polypeptide chain (SEQ ID No. 88) of HµM chain. an H chain of the humanised anti-human Fas antibody CH11.

Twenty-two primers were synthesised for the PCR, as follows:
(VH1P; (SEQ ID No. 156);
(VHSP; (SEQ ID No. 157);
VHSN; (SEQ ID No. 158);
VH2P; (SEQ ID No. 159);
VH2N; (SEQ ID No. 160);
VH3P; (SEQ ID No. 161);
VH3N; (SEQ ID No. 162);
VH4P; (SEQ ID No. 163);
VH4N; (SEQ ID No. 164);
VHAPAPX; (SEQ ID No. 165);
VHAPAN; (SEQ ID No. 166);
VHTERM; (SEQ ID No. 167);
HUMFR2P; (SEQ ID No. 168);
HUMFR2N; (SEQ ID No. 169);
MOUFR2P; (SEQ ID No. 170);
MOUFR2; (SEQ ID No. 171);
GTOSP; (SEQ ID No. 172);
GTOSN; (SEQ ID No. 173);
TCVVAP; (SEQ ID No. 174);
TCVVN1; (SEQ ID No. 175);
ME18P; (SEQ ID No. 176); and
VH06; (SEQ ID No. 178).

### 2) Construction of plasmid pMEC22

An expression vector was constructed for a humanised CH11 chain, in a multi stage process. Initially, a vector containing the carboxyl terminus (hereinafter referred to as the "C-terminus") of the constant region of the H chain of human IgM was constructed

### MEC

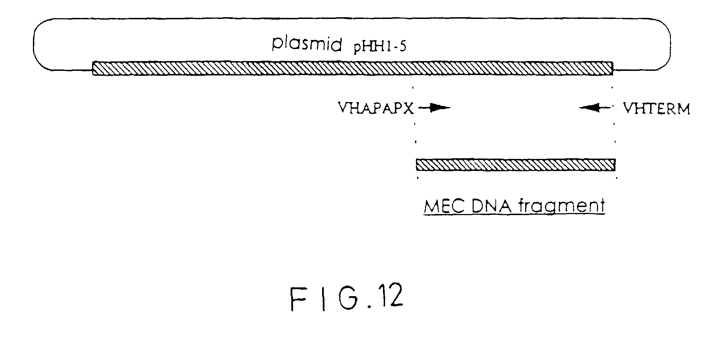

A DNA fragment was prepared encoding the C-terminal amino acid sequence of the H chain of human IgM. This fragment is hereinafter referred to as "MEC DNA fragment". The construction is outlined in Figure 12. The PCR reaction conditions were as follows:
Composition of the reaction solution:
plasmid pHH1-5 DNA, 1 µg;
oligonucleotide primer VHAPAPX, 80 pmol;
oligonucleotide primer VHTERM, 80 pmol;
dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The MEC DNA fragment amplified by PCR in this way was extracted with phenol. The DNA was then precipitated using 100% ethanol. The DNA (20 - 30 µg) was electrophoresed on a 5% (w/v) polyacrylamide gel. The gel was stained with 1 µg/ml of ethidium bromide, such that the DNA fragments were visible when viewed under UV light. The DNA fragment detected in this way was excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 50 µl of distilled water.

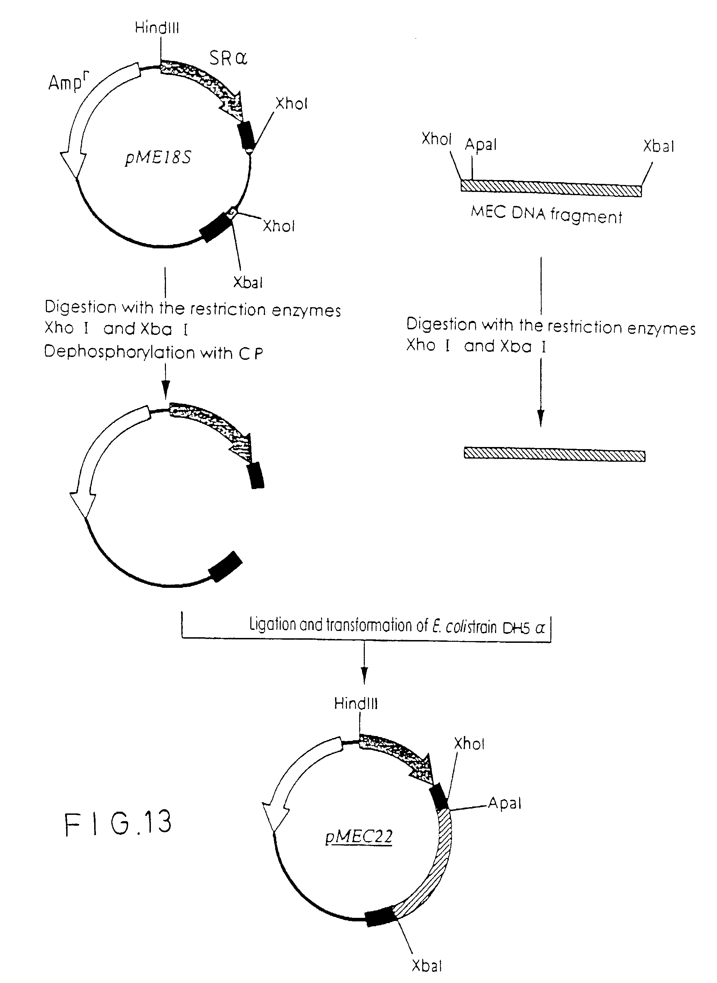

The construction of a plasmid carrying MEC DNA fragment is outlined in Figure 13.

The MEC DNA was further purified by phenol extraction followed by ethanol precipitation. The DNA (1 µg) was then digested with the restriction enzymes Xho1 and Xba1.

A portion (1 µg) of plasmid pME18S DNA [Hara, T. and Miyajima, A., *supra*] was also digested with the restriction enzymes Xho1 and Xba1. The resulting DNA was treated with CIP. A portion (100 ng) of the dephosphorylated pME18S plasmid DNA was ligated to 0.5 µg of the Xba-1, Xho1 digested MEC fragment. Ligation was carried out using a ligation kit [Takara Shuzo], and the ligation product was transformed into *E. coli* strain JM109 [Takara Shuzo].

Restriction analysis of plasmid DNA contained in transformant colonies was carried out, to identify a plasmid containing the DNA insert of interest. Plasmid pMEC22 was obtained, in which MEC DNA was inserted downstream of SRα promoter in pME18S in the correct orientation for expression of the immunoglobulin protein product.

### 3) Construction of plasmid pMEHC20

### a) First step PCR

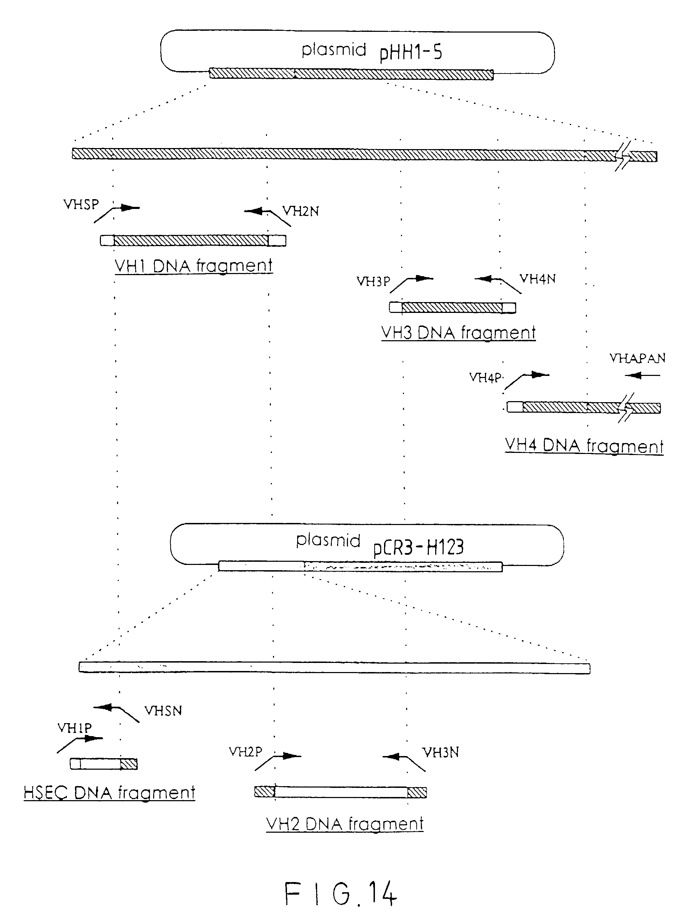

The outline of the first step PCR is shown in Figure 14.

### HSEC

A DNA fragment was prepared encoding a secretion signal sequence and the N-terminus of the FRH₁ region. This fragment is hereinafter referred to as the "HSEC DNA fragment". The following PCR reaction conditions were used:
Composition of the reaction solution:
plasmid pCR3-H123 DNA, 1 µg;
oligonucleotide primer VHSN, 80 pmol;
oligonucleotide primer VH1P, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VH1

A DNA fragment was prepared encoding the FRH₁ region and the N-terminus of the CDRH₁ region. This fragment is hereinafter referred to as "VH1 DNA fragment". The following PCR reaction conditions were used:
Composition of the reaction solution:
plasmid pHH1-5 DNA, 1 µg;
oligonucleotide primer VHSP, 80 pmol;
oligonucleotide primer VH2N, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VH2

A DNA fragment was prepared encoding the CDRH₁ region, the C-terminus of the FRH₂ region and the N-terminus of the CDRH₂ region. This fragment is hereinafter referred to as "VH2 DNA fragment". The following PCR reaction conditions were used:
Composition of the reaction solution:
plasmid pCR3-H123 DNA, 1 µg;
oligonucleotide primer VH2P, 80 pmol;
oligonucleotide primer VH3N, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VH3

A DNA fragment was prepared encoding the N-terminus of the CDRH₂ region, the FRH₃ region and the CDRH₃ region. This fragment is hereinafter referred to as the "VH3 DNA fragment". The following PCR reaction conditions were used:
Composition of the reaction solution:
plasmid pHH1-5 DNA, 1 µg;
oligonucleotide primer VH3P, 80 pmol;
oligonucleotide primer VH4N, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VH4

A DNA fragment was prepared encoding the CDR-3 region, the FR-4 region and the N-terminus of the constant region of the H chain. This fragment is hereinafter referred to as the "VH4 DNA fragment". The following PCR reaction conditions were used:
Composition of the reaction solution:
plasmid pHH1-5 DNA, 1 µg;
oligonucleotide primer VH4P, 80 pmol;
oligonucleotide primer VHAPAN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HSEC, VH1, VH2, VH3 and VH4 DNA fragments amplified by PCR in this way were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### b) Second step PCR

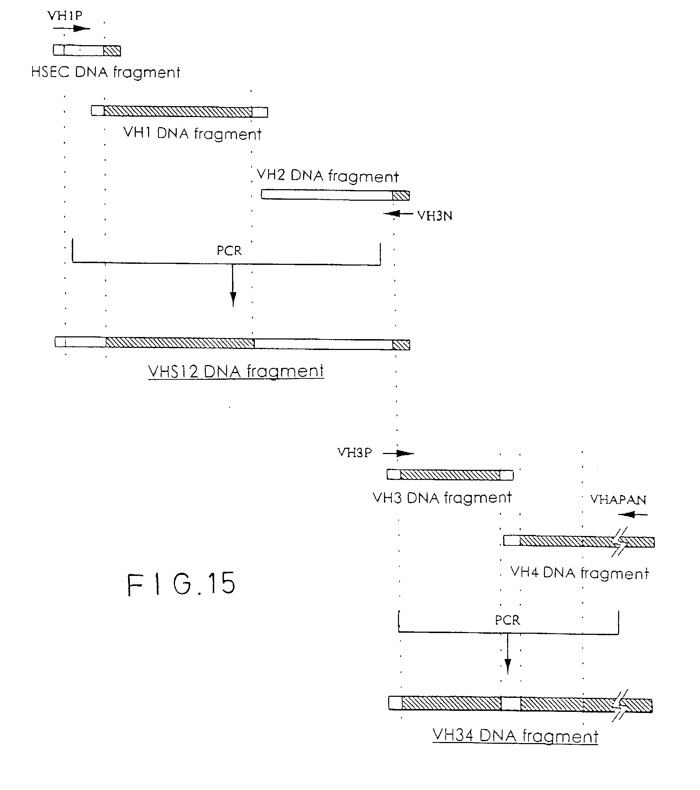

The second step PCR is outlined in Figure 15.

### VHS12

A fusion of the HSEC, VH1 and VH2 DNA fragments, described above, was prepared using PCR. This fragment is hereinafter referred to as "VHS12 DNA fragment". The following PCR reaction conditions were used:
Composition of the reaction solution:
HSEC DNA solution prepared in the first step PCR, 10 µl;
VH1 DNA solution prepared in the first step PCR, 10 µl;
VH2 DNA solution prepared in the first step PCR, 10 µl;
oligonucleotide primer VH1P, 80 pmol;
oligonucleotide primer VH3N, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### VH34

A fusion of the VH3 DNA fragment and the VH4 DNA fragment, described above, was prepared using PCR. This fragment is hereinafter referred to as the "VH34 DNA fragment". The following PCR reaction conditions were used:
Composition of the reaction solution:
VH3 DNA solution prepared in the first step PCR, 10 µl;
VH4 DNA solution prepared in the first step PCR, 10 µl;
oligonucleotide primer VH3P, 80 pmol;
oligonucleotide primer VHAPAN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The resulting VHS 12 and VH34 DNA fragments were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### c) Third step PCR

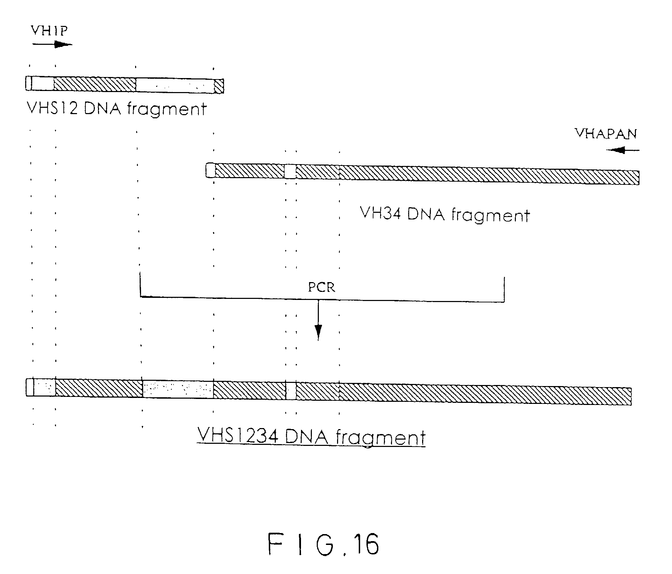

The third step PCR is outlined in Figure 16.

### VHS1234

A fusion of the VHS12 DNA fragment and the VH34 DNA fragment, described above, was prepared using PCR. This fragment is hereinafter referred to as the "VHS1234 DNA fragment". The following PCR reaction conditions were used:
Composition of the reaction solution:
VHS12 DNA solution prepared in the second step PCR, 10 µl;
VH34 DNA solution prepared in the second step PCR, 10 µl;
oligonucleotide primer VH1P, 80 pmol;
oligonucleotide primer VHAPAN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The resulting VHS1234 DNA fragment was extracted with phenol. The DNA was then precipitated using ethanol. The DNA (20 -30 µg) was electrophoresed on a 5% (w/v) polyacrylamide gel. The gel was stained with 1 µg/ml of ethidium bromide, such that the DNA fragments were visible when viewed under UV light. The DNA fragment detected in this way was excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 50 µl of distilled water.

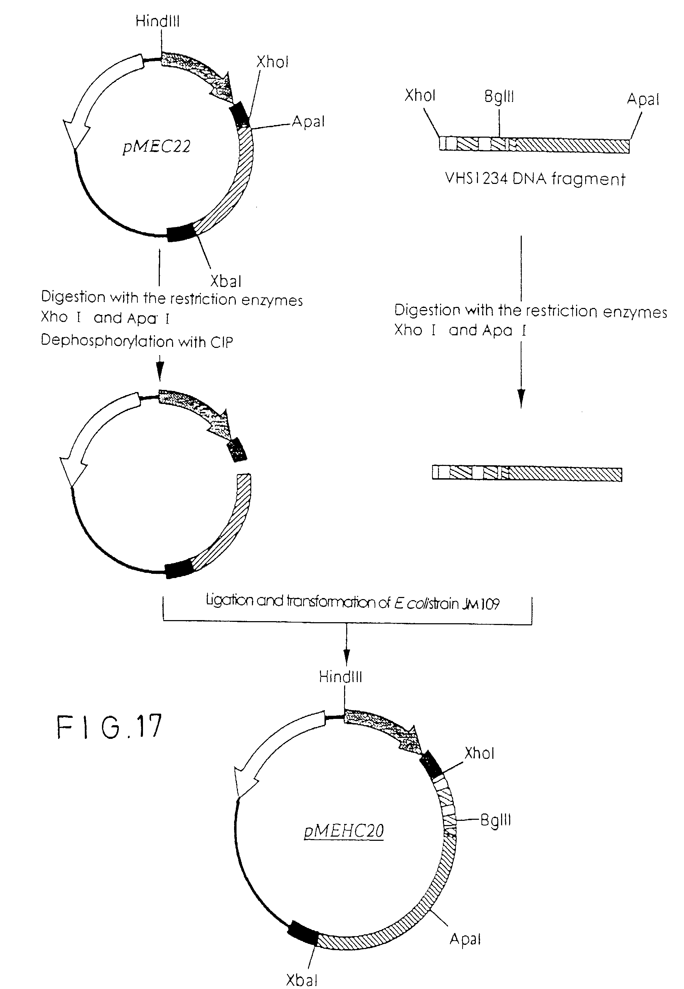

The construction of a plasmid carrying VHS1234 DNA is outlined an Figure 17.

The VHS1234 DNA obtained in this way was further purified by phenol extraction followed by ethanol precipitation. The DNA was then digested with the restriction enzymes Xho1 and Apa1.

A portion (1 µg) of plasmid pMEC22 DNA was also digested with the restriction enzymes Xho1 and Apa1, and then dephosphorylated with CIP. A portion of the dephosphorylated pMEC22 plasmid DNA (100 ng) was ligated to 0.5 µg, of the Xho1-Apa1 digested VHS1234 DNA fragment. Ligation was carried out using a ligation kit [Takara Shuzo] and the product of the ligation transformed into *E. coli* strain JM109 [Takara Shuzo].

Restriction analysis of plasmid DNA contained in transformant colonies was carried out, to identify plasmids containing the DNA insert of interest. Plasmid pMEHC20 was obtained, containing the VHS1234 DNA fragment. This fragment was inserted downstream of the SRα promoter in pMHC22, in the correct orientation for expression of the immunoglobulin protein product.

### 4) Construction of plasmid pHFR3 and plasmid pHFR4

### a) First step PCR

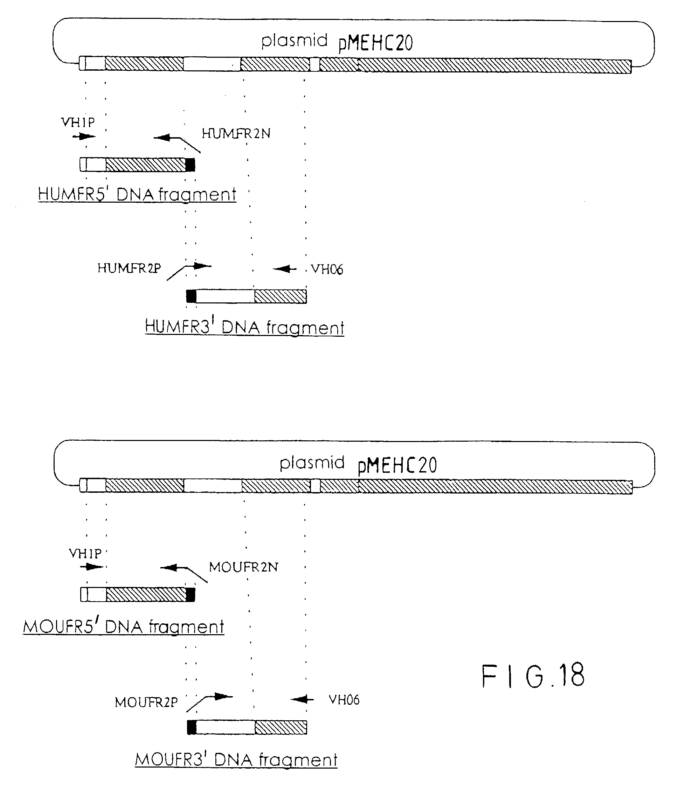

The outline of the first step PCR is shown in Figure 18.

### HUMFR5'

A DNA fragment was prepared encoding a secretion signal sequence, the FRH₁ region, the CDRH₁ region, and the FRH₂ region (in which the amino acid residues of positions 38 to 44 had been replaced by arginine, glutamine, alanine, proline, glycine, glutamine and glycine residues). The fragment is hereinafter referred to as the "the HUMFR5' DNA fragment". The PCR reaction conditions were as follows:
Composition of the reaction solution:
plasmid pMEHC20 DNA, 1 µg;
oligonucleotide primer VH16, 80 pmol;
oligonucleotide primer HUMFR2N, 80 pmol;
25 mM dNTPs cocktail, 20 µl;
10x Pfu buffer, 20 µl;
Pfu DNA polymerase [Stratagene], 10 units.

Specifically, the reaction solution was initially, heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### HUMFR3'

A DNA fragment was prepared encoding the FRH₂ region (in which the amino acid residues of positions 38 to 44 had been replaced by arginine, glutamine, alanine, proline, glycine, glutamine and glycine residues), the CDRH₂ region, the FRH₃ region, the CDRH₃ region, the FRH₄ region and the N-terminus of the constant region of the H chain. This fragment is hereinafter referred to as the "HUMFR3' DNA fragment". The PCR reaction conditions were as follows:
Composition of the reaction solution:
plasmid pMEHC20 DNA, 1 µg;
oligonucleotide primer VH06, 80 pmol;
oligonucleotide primer HUMFR2P, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### MOUFR5'

A DNA fragment was prepared encoding a secretion signal sequence, the FRH₁ region, the CDRH₁ region, and the FRH₂ region (in which the amino acid residues of positions 38 to 44 had been replaced by lysine, glutamine, alanine, histidine, glycine, lysine and serine residues). This fragment is hereinafter referred to as the "MOUFR5' DNA fragment". The PCR reaction conditions were as follows:
Composition of the reaction solution:
plasmid pMEHC20 DNA, 1 µg;
oligonucleotide primer VH1P, 80 pmol;
oligonucleotide primer MOUFR2N, 80 pmol;
25 mM dNTPs cocktail, 20 µl;
10x Pfu buffer, 20 µl;
Pfu DNA polymerase [Stratagene], 10 units.
Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### MOUFR3'

A DNA fragment was prepared encoding the FRH₂ region (in which the amino acid residues of positions 38 to 44 had been replaced by lysine, glutamine, alanine, histidine, glycine, lysine and serine residues), the CDRH₂ region, the FRH₃ region, the CDRH₃ region, the FRH₄ region, and the N-terminus of the constant region of the H chain. This fragment is hereinafter referred to as the "MOUFR3' DNA fragment". The PCR reaction conditions were as follows:
Composition of the reaction solution:
plasmid pMEHC20 DNA, 1 µg;
oligonucleotide primer VH06, 80 pmol;
oligonucleotide primer MOUFR2P, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HUMFR5', HUMFR3', MOUFR5' and MOUFR3' DNA fragments were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### b) Second step PCR

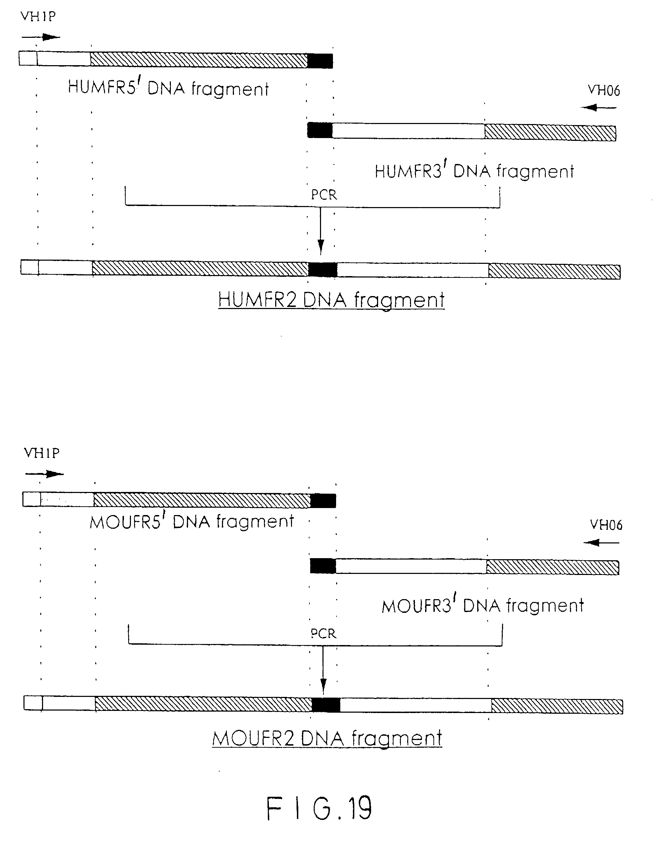

The second step PCR is outlined in Figure 19.

### HUMFR2

A fusion of the HUMFR5' and HUMFR3' DNA fragments, described above, was prepared using PCR. This fragment is hereinafter referred to as the "HUMFR2 DNA fragment". The PCR reaction conditions were as follows:
Composition of the reaction solution:
HUMFR5' DNA solution prepared in the first step PCR, 10 µl;
HUMFR3' DNA solution prepared in the first step PCR, 10 µl;
oligonucleotide primer VH1P, 80 pmol;
oligonucleotide primer VH06, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### MOUFR2

A fusion of the MOUFR5' and MOUFR3' DNA fragments described above (hereinafter referred to as the "MOUFR2 DNA fragment") was prepared using PCR under the following conditions.
Composition of the reaction solution:
MOUFR5' DNA solution prepared in the first step PCR, 10 µl;
MOUFR3' DNA solution prepared in the first step PCR, 10 µl;
oligonucleotide primer VHIP, 80 pmol;
oligonucleotide primer VH06, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HUMFR2 and MOUFR2 DNA fragments were extracted with phenol. The DNA was then precipitated using ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

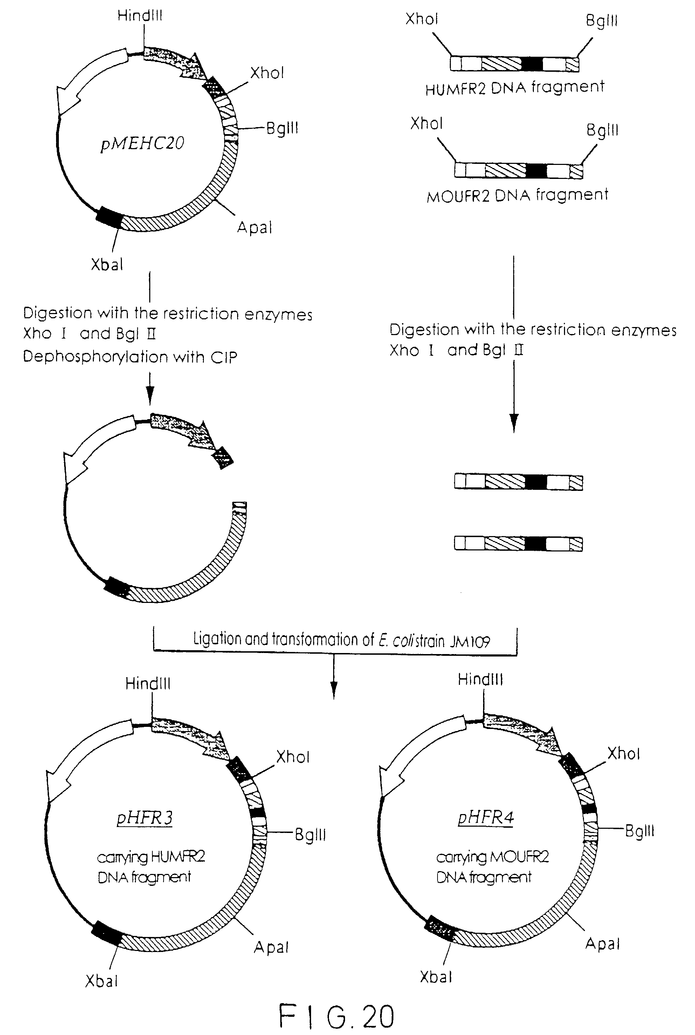

The construction of a plasmid carrying the HUMFR2 DNA fragment and the MOUFR2 DNA fragment is outlined in Figure 20.

The HUMFR2 and MOUFR2 DNA fragments obtained in this way were further purified by phenol extraction followed by ethanol precipitation. The DNA was then digested with the restriction enzymes Xho 1 and Bg1II.

A portion (1 µg) of plasmid pMEHC20 DNA was also digested with the restriction enzymes Xho1 and Bg1II, and then dephosphorylated with CIP. A portion (100 ng) of the dephosphorylated plasmid pMEHC20 DNA was ligated to 0.5µg of each of the Xho1 and Bg1II digested HUMFR2 or MOUFR2 DNA fragments. Ligation was carried out using a ligation kit [Takara Shuzo] and the product of the ligation transformed into *E. coli* strain JM109 [Takara Shuzo].

Restriction analysis of plasmid DNA contained in transformant colonies was carried out, to identify plasmids containing the DNA insert of interest. Plasmid pHFR3, containing the HUMFR2 DNA fragment and plasmid pHFR4, containing the MOUFR2 DNA fragment were obtained.

### 5) Construction of plasmid pMECW5

Plasmid pMECW5 was constructed by PCR using DNA from plasmid pMEC22 as a template for the PCR reaction.

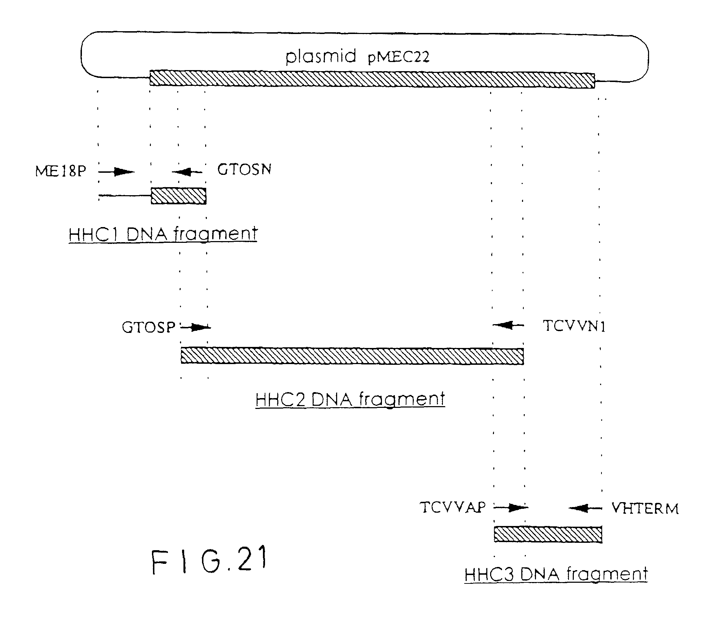

The PCR process is outlined in Figure 21.

### a) First step PCR

### HHC1

A DNA fragment was prepared representing the 5'-terminal region of the DNA insert of plasmid pMEC22. This fragment is hereinafter referred to as the "HHC DNA fragment". The PCR reaction conditions were as follows:
Composition of the reaction solution:
plasmid pMEC22 DNA, 1 µg.
oligonucleotide primer ME18P, 80 pmol;
oligonucleotide primer GTOSN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### HHC2

A DNA fragment was prepared corresponding to an internal region of the DNA insert of plasmid pMEC22. This fragment is hereinafter referred to as the "HHC2 DNA fragment" The PCR reaction conditions were as follows:
Composition of the reaction solution:
plasmid pMEC22 DNA, 1 µg;
oligonucleotide primer GTOSP, 80 pmol;
oligonucleotide primer TCVVN1, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### HHC3

A DNA fragment was prepared representing the 3'-terminal region of the DNA insert of plasmid pMEC22. This fragment is hereinafter referred to as the "HHC3 DNA fragment". The PCR reaction conditions were as follows:
Composition of the reaction solution:
plasmid pMEC22 DNA, 1 µg;
oligonucleotide primer TCVVAP, 80 pmol;
oligonucleotide primer VHTERM, 80 pmol;
25 mM dNTPs cocktail, 20 µl;
10x Pfu buffer, 20 µl;
Pfu DNA polymerase [Stratagene], 10 units.

Specifically, the reaction solution was initially, heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HHC1, HHC2 and HHC3 DNA fragments thus obtained were extracted with phenol. The DNA was then precipitated using 100% ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product in each case was dissolved in 50 µl of distilled water.

### B) Second step PCR

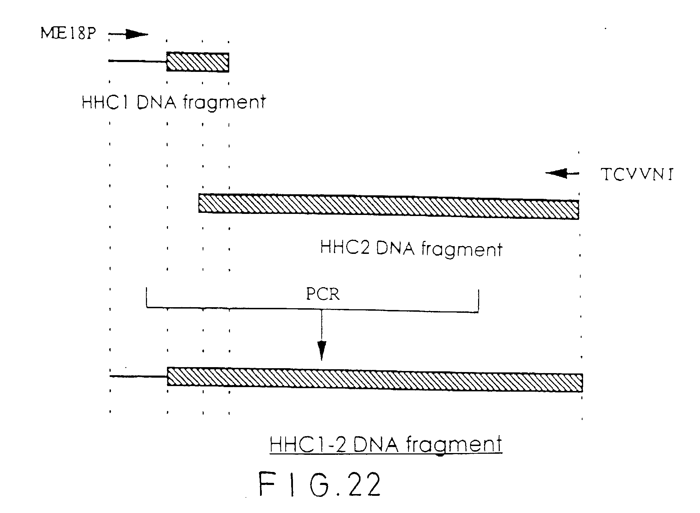

The second step PCR is outlined in Figure 22.

### HHC1-2

A fusion of the HHC1 DNA fragment and HHC2 DNA fragment was prepared using PCR. The DNA fragment is hereinafter referred to as the "HHC1-2 DNA fragment". The PCR reaction conditions were as follows:
Composition of the reaction solution:
HHC1 DNA solution prepared in the first step PCR, 10 µl;
HHC2 DNA solution prepared in the first step PCR, 10 µl;
oligonucleotide primer ME18P, 80 pmol;
oligonucleotide primer TCVVN, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HHC1-2 fragment thus obtained was extracted with phenol. The DNA was then precipitated using ethanol. The DNA (20-30 µg) was electrophoresed on a 5% (w/v) polyacrylamide gel. The gel was stained with 1 µg/ml of ethidium bromide, such that the DNA fragments were visible when viewed under UV light. The DNA fragment detected in this way was excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 50 µl of distilled water.

### c) Third step PCR

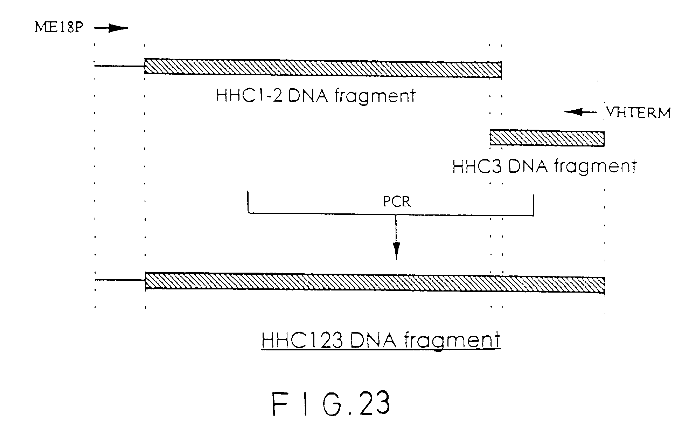

The third step PCR is outlined in Figure 23.

### HHC123

A fusion of the HHC1-2 and HHC3 DNA fragments, described above, was prepared using PCR. The fragment is hereinafter referred to as "HHC12 DNA fragment". The PCR reaction conditions were as follows:
Composition of the reaction solution:
HHC3 DNA solution prepared in the first step PCR, 10 µl;
HHC1-2 DNA solution prepared in the second step PCR, 10 µl;
oligonucleotide primer ME18P, 80 pmol;
oligonucleotide primer VHTERM, 80 pmol;
25 mM dNTPs cocktail, 20 µl;
10x Pfu buffer, 20 µl;
Pfu DNA polymerase [Stratagene], 10 units.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HHC1-2 fragment thus obtained was extracted with phenol. The DNA was then precipitated using ethanol. The DNA (20 -30 µg) was electrophoresed on a 5% (w/v) polyacrylamide gel. The gel was stained with 1 µg/ml of ethidium bromide, such that the DNA fragments were visible when viewed under UV light. The DNA fragment detected in this way was excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 50 µl of distilled water.

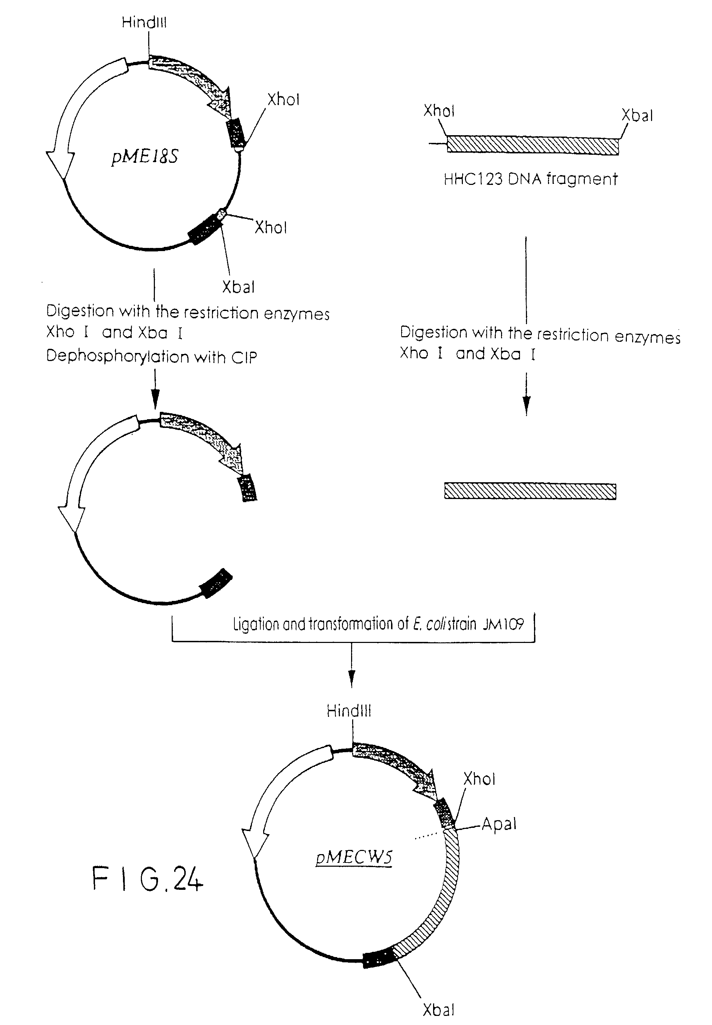

The construction of a plasmid carrying HHC123 DNA is outlined in Figure 24.

The HHC123 DNA obtained in this way was further purified by phenol extraction followed by ethanol precipitation. The DNA was then digested with the restriction enzymes Xho1 and Xba1.

A portion (1 µg) of plasmid pME18S DNA [Hara, T. and Miyajima, A., *supra*] was also digested with the restriction enzymes Xho1 and Xba1, and then dephosphorylated with CIP. A portion (100 ng) of the dephosphorylated plasmid pME18S DNA was ligated to 0.5 µg of Xho1 and Xba1 digested HMC123 DNA. Ligation was carried out using a ligation kit [Takara Shuzo] and the resulting DNA was transformed into *E*. *coli* strain JM109 [Takara Shuzo].

Restriction analysis of plasmid DNA contained in transformant colonies was carried out, to identify plasmids containing the DNA insert of interest. Plasmid pMECW5 was identified, containing the HHC123 DNA fragment.

### 6) Construction of expression plasmids pHµH5-1 and plasmid pHµM1-1 encoding humanised versions of the CH11 H chain

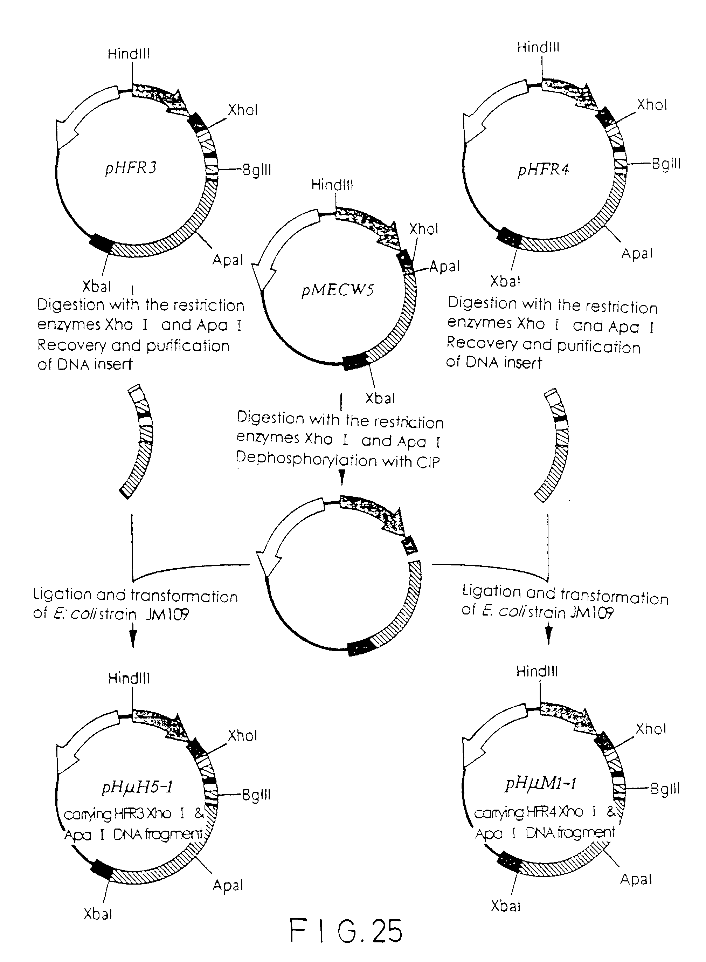

The final expression plasmids, pHµH5-1 and pHµM1-1, were constructed by combining DNA from plasmid pHFR3 DNA, plasmid pHFR4 DNA and plasmid pMECW5 DNA. The construction is outlined in Figure 25.

The HFR3 DNA fragment was prepared as follows A portion (30 µg) of plasmid pHFR3 DNA was digested simultaneously with the restriction enzymes Apa1 and Xho1. The products of the digestion were separated by 5% (w/v) polyacrylamide gel electrophoresis. The gel was stained with 1 µg/ml of ethidium bromide, such that the DNA fragments were visible when viewed under UV light. The DNA fragment of interest detected in this way, having a size of about 950 bp was excised from the gel with a razor blade and electro-eluted from the acrylamide gel using a Centriruter [Amicon] equipped with Centricon 10 [Amicon].

The HFR4 DNA fragment was prepared as follows. A portion (30 µg) of plasmid pHFR3 DNA was digested simultaneously with the restriction enzymes Apa1 and Xho1. The products of the digestion were separated by 5% (w/v) polyacrylamide gel electrophoresis. The gel was stained with 1 µg/ml of ethidium bromide, such that the DNA fragments were visible when viewed under UV light. The DNA fragment of interest detected in this way, having a size of about 950 bp was excised from the gel with a razor blade and electro-eluted from the acrylamide gel using a Centriruter [Amicon] equipped with Centricon 10 [Amicon].

A portion 1 µg of DNA of plasmid pMECW5 was digested with the restriction enzymes Xho1 and Apa1, and then dephosphorylated with CIP. A portion (100 ng) of the dephosphorylated plasmid pMECW5 DNA was ligated to 0.5 µg of each of the HFR3 DNA or HFR4 DNA fragments prepared above. The ligation was carried out using a ligation kit [Takara Shuzo] and the product of the ligation reaction was transformed into *E. coli* strain DH5α.

Restriction analysis of plasmid DNA contained in transformant colonies was carried out, to identify plasmids containing the DNA insert of interest. Plasmid pHµH5-1 was identified, containing the HFR3 DNA fragment. Plasmid pHµM1-1 was identified, containing the HFR4 DNA fragment.

### 7) Verification of nucleotide sequences

The DNA inserts of the plasmids pHµH5-1 and pHµM1-1 were sequenced. The primers used in the sequencing process were ME18P (SEQ ID No. 176) and VH06 (SEQ ID No. 178), described above, in addition to 8 newly synthesised primers. These were:
ME18RV; (SEQ ID No. 177);
VH05; (SEQ ID No. 179);
VH07; (SEQ ID No. 180);
VH08; (SEQ ID No. 181);
VH01; (SEQ ID No. 182);
VH02; (SEQ ID No. 183);
VH03; (SEQ ID No. 184); and
VH04; (SEQ ID No. 185).

DNA sequencing was performed using the dideoxynucleotide chain termination method [Sanger, F. S. *et al.*, *supra*]. Prior to sequencing, the plasmid DNA template was isolated from the host cells by alkaline-SDS lysis [Sambrook, J. *et al., supra*] and the DNA purified using caesium chloride [Sambrook, J. *et al., ibid*.].

Sequence analysis confirmed that the sequence of the DNA insert of pHµH5-1 encodes the polypeptide defined in SEQ ID No. 86. The sequence of the DNA insert of pHµM1-1 encodes the polypeptide defined in SEQ ID No. 88.

### EXAMPLE 7

### Expression of the genes coding for the subunits of humanised versions of CH11 in COS-7 cells

Humanised H chain DNA and humanised L chain DNA, constructed above, was expressed in the COS-7 cell line, a cell line derived from monkey kidney. The expression plasmids for the humanised H chains and the humanised L chains were transfected into COS-7 cells by electroporation, using the gene transfection apparatus ECM600 M (BTX).

COS-7 cells [American Type Culture Collection No. CRL-1651] were cultured in a 225 cm² culturing flask [Sumitomo Bakelite]. The cells were grown to a semi-confluent state in Dulbecco's modified Eagle minimum essential medium (hereinafter abbreviated as "DMEM"; Nissui Seiyaku) containing 10 % foetal bovine serum [CSL]. The medium was removed and the COS-7 cells were treated with 3 ml of trypsin-EDTA solution [Sigma Chemicals Co.] at 37°C for 3 minutes. The cells were harvested by centrifugation at 800 rpm for 2 minutes and then washed twice with phosphate buffer [0.02 % (w/v) potassium chloride (KCl), 0.02 % (w/v) potassium dihydrogenphoshate (KH₂PO₄), 0.8 % (w/v) sodium chloride (NaCl), 1.15 % (w\v) disodium hydrogenphosphate (Na₂HPO₄); hereinafter referred to as "PBS(-) buffer"; Nissui Seiyaku]. The washed COS-7 cells were adjusted to a density of 4x10⁶ cells, ml with PBS(-) buffer to produce a COS-7 cell suspension.

In parallel, plasmid DNA was prepared from the H chain expression plasmids and the L chain expression plasmids, using a plasmid Maxiprep kit [MaxiPrep DNA Purification Kit; Promega]. A portion (40µg) of DNA from each of a heavy chain expression plasmid and a light chain expression plasmid was mixed in a single tube, and then precipitated with 100% ethanol. The combinations of heavy and light chain DNA mixtures are defined below. The DNA was resuspended in 40 µl of PBS(-) buffer. The resulting plasmid mixture (40 µl) was mixed with 500 µl of the COS-7 cell suspension (2 x 10⁶ cells), prepared above.

The mixture was transferred to an electroporation cuvette having an electrode interval of 4 mm [BioRad], and then loaded in an electroporation apparatus. Electroporation was then used to introduce the plasmid DNA of interest into the COS-7 cells, using a pulse of 150 V, 900 µF. After electroporation, the cell-DNA mixture was resuspended in 20 ml of DMEM containing 10 % foetal bovine serum, then transferred to a 75 cm² culturing flask [Sumitomo Bakelite]. The cells were incubated in 75 % CO₂ at 37 °C for 24 hours. The culture supernatant was removed and the cells were washed with serum-free DMEM medium. A portion (20 ml) of fresh serum-free DMEM medium was added and the cells were cultured in 7.5 % CO₂ at 37 °C for 24 hours. The supernatant was then recovered

COS-7 cells were transfected with the following plasmids or plasmid combinations, using the above procedure. The supernatant was recovered in each case.
(A): pME18S
(B): pHµM1-1 and pHκKY2-58
(C): pHµM1-1 and pHκKF2-19
(D): pHµM1-1 and pHκRY2-10
(E): pHµM1-1 and pHκRF2-52
(F): pHµH5-1 and pHκKY2-58
(G): pHµH5-1 and pHκKF2-19
(H): pHµH5-1 and pHκRY2-10
(I): pHµH5-1 and pHκRF2-52

### TEST EXAMPLE 1

### Detection of the humanised anti-human Fas antibodies

The humanised anti-human Fas antibodies produced by the present invention were identified by Western blotting. This method involves the separation of proteins by SDS-polyacrylamide gel electrophoresis (hereinafter referred to as "SDS-PAGE"), followed by transfer to a nitrocellulose membrane. The transferred protein can then be identified by cross reaction with antibodies.

### 1) Separation by SDS-PAGE

A portion (1 ml) of the culture supernatant obtained in Working Example 7 was dialysed against 5 litres of pure water, using a dialysis tube with the exclusion limit of 12,000 to 14,000 daltons. The dialysis was carried out at 4 °C for 15 hours. The resulting solution was dried under vacuum using a centrifuge-concentrator [CC-101; Tomy Seiko]. A portion (10 µl) of sample buffer [2 % (w/v) SDS (electrophoresis grade; BioRad), 5 % (v/v) β-mercaptoethanol (Sigma Chemicals Co.), 10% (v/v) glycerol, 0.1 % (w/v) bromophenol blue] was added, after which the mixture was heated at 100 °C for 5 minutes to produce an electrophoresis sample. The electrophoresis sample obtained was loaded on an SDS-PAGE (4 to 20 % gradient gel; Iwaki Glass), and run at 20 mA, constant current, at room temperature for 1 hour.

### 2) Transfer and immobilisation of the proteins

Once the electrophoresis had been performed, the proteins were transferred from the gel to a nitrocellulose membrane [Transblot Transfer Membrane; BioRad] using the semi-dry blotting method [Towbin, H., *et al.*, (1979), Proc. Natl. Acad. Sci. USA, *76*, 4350]. The specific apparatus and conditions used were as follows:
- Transfer buffer:: 20 mM Tris, 150 mM glycine,
10 % (v/v) methanol;
- Blotting apparatus:: Manufactured by Iwaki Glass (TF03-050);
- Running conditions:: 4 °C, 0.2 A (constant current), 1 hour.

SDS-PAGE and Western blotting were performed in duplicate, under identical conditions, resulting in two identical nitrocellulose membranes. One membrane was analysed to detect the H chain and the other analysed to detect the L chain.

### 3) Antibody detection

After the Western transfer, the nitrocellulose membranes were immersed in an aqueous solution of 20 mM Tris-HCI buffer (pH 7.5) with 500 mM sodium chloride [NaCl] (hereinafter referred to as "TBS") containing 3 % (w/v) gelatin [Nippon BioRad]. The membranes were shaken gently at room temperature for 1 hour (the procedure is hereinafter referred to as "blocking").

Detection of the H chains of the humanised antibodies was carried out using a peroxidase-labelled anti-human IgM H chain antibody [Peroxidase-conjugated AffiniPure Goat Anti-Human IgM, Fc5µ Fragment Specific; Jackson Immuno-research Laboratory]. After blocking, the nitrocellulose membranes were removed from the blocking solution and shaken in 10 ml of buffer (TBS solution containing 1 % (w/v) gelatin) containing 5 µl of the labelled anti-human IgM H chain antibody at room temperature for 4 hours. The nitrocellulose membranes were then removed and immersed in 20 ml of TBS solution containing 2 % (v/v) Tween 20 [BioRad], then washed by gently shaking at room temperature for 20 minutes. This wash was repeated. The washed nitrocellulose membranes were then blot-dried with paper towels.

Cross reactivity between the antibody and the proteins on the membrane was detected via the peroxidase activity conjugated to the antibody.

Residual peroxidase activity on the membrane was detected using an ECL Western Blotting System [Amersham]. More specifically, the substrate in this system emits light during a chemical reaction under the catalytic action of peroxidase. The light emission may be detected using an ECL Mini Camera [Amersham] and instant film [Type 667; Polaroid]. The proteins remaining in the gel were silver-stained [Oakley *et al*., (1980), Anal. Biochem, *105*, 361 *et seq*]. The pictures taken were compared with the silver-stained gels to identify the protein bands that were specifically bound to the antibody.

Detection of the L chains of the humanised antibodies was carried out using a peroxidase-labelled anti-human IgM L chain antibody [Peroxidase-Labelled Monoclonal Antibody to Human Kappa Light Chain HP6156; Kilkeguard and Perry Laboratory]. After blocking, the nitrocellulose membranes were removed from the blocking solution and shaken in 10 ml of buffer (TBS solution containing 1 % (w/v) gelatin) containing 10 µl of the labelled anti-human IgM L chain antibody, at room temperature for 4 hours. The nitrocellulose membranes were then removed and immersed in 20 ml of TBS solution containing 0.05 % (v/v)Tween 20 and washed by gently shaking at room temperature for 20 minutes. This wash was repeated. The washed nitrocellulose membranes were then blot-dried with paper towels.

As with the detection of the humanised H chains, any proteins reacting with the antibody were detected using ECL Western Blotting System [Amersham]. The cross reaction was followed by the production of light, detected via photographic film. The pictures taken were compared with the silver-stained gels to identify the protein bands that were specifically bound to the antibody.

Use of the antibody specific to the human H chain resulted in the detection of a band of approximately 78,000 daltons in the following; samples (B), (C), (D), (E), (F), (G), (H) and (I) of Working Example 7. These samples all derive from COS-7 cells transfected with either pHµM1-1 or pHµH5-1

Use of the antibody specific to the human L chain resulted in the detection of a band of approximately 25,000 daltons in the following; samples (B), (C), (D), (E), (F), (G), (H) and (I) of Working Example 7. These samples all derive from COS-7 cells transfected with either plasmid pHκKY2-58, plasmid pHκKF2-19, plasmid pHκRY2-10 or plasmid pHκRF2-52.

### TEST EXAMPLE 2

### Determination of the binding activity of the anti-Fas antibodies to Fas antigen

The ability of the humanised anti-Fas antibodies of the present invention to bind the Fas antigen was assayed by the ELISA technique. This method involves the preparation of a soluble human Fas fusion protein, followed by an assay to detect binding of the antibody to the soluble protein.

### 1) Expression of a soluble human Fas antigen fusion protein

In order to produce a soluble human Fas antigen, an expression vector for a fusion protein was constructed, consisting of the extracellular domain of the human Fas antigen and the extracellular domain of mouse interleukin-3 receptor. This protein is hereinafter referred to as the human Fas fusion protein".

DNA encoding the human Fas fusion protein was prepared by PCR, as follows;

### a) Template DNA

Plasmid DNA from two plasmids was used in the PCR reaction, to generate a human Fas fusion protein. The first plasmid was plasmid pME18S-mFas-AIC [Nishimura, Y. *et al.*, (1995), J. Immunol., *154*, 4395], which encodes a fusion protein of the extracellular domain of mouse Fas antigen [Watanabe-Fukunaga, R., *et al.,* (1992), J. Immunol., *148*, 1274 et *seq*.] and the extracellular domain of mouse interleukin-3 receptor [Gorman. D., *et al.*, (1990), Proc. Natl. Acad. Sci. USA 87, 5459 *et seq*., Hara, T. and Miyajima, A., (1992), EMBO J, *11*, 1875]. The other plasmid, pCEV4, carries cDNA encoding the human Fas antigen [Itoh, N., *et al.*, (1991), Cell, *66*, 233].

### b) Preparation of the primers

Four nucleotide primers were prepared for PCR. The sequences prepared were:
N1; (Seq. ID No. 186);
C3N; (Seq. ID No. 187);
N3N; (Seq. ID No. 188); and
CTN2;(Seq. ID No. 189).

### c) First step PCR

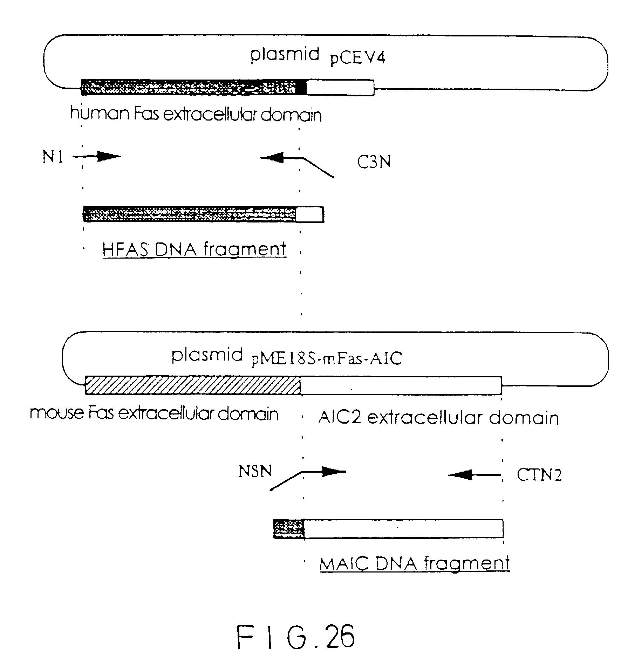

The outline of the first step PCR is shown in Figure 26.

### HFAS

A fragment of DNA was prepared encoding the extracellular domain of the human Fas antigen. This fragment is herein referred to as the "HFAS DNA fragment". The PCR process was carried out using the LA PCR Kit [Takara Shuzo], under the following conditions.
Composition of the reaction solution:
plasmid pCEV4 DNA, 20 ng;
oligonucleotide primer N1, 0.5 µg;
oligonucleotide primer C3N, 0.5 µg;
dNTP mix, 25 µl;
10 x LA PCR buffer, 25 µl;
LA Taq polymerase [Takara Shuzo], 12.5 units.

The final volume of the solution was made up to 250 µl with redistilled water. The dNTP mix, 10 x LA PCR buffer and LA Taq polymerase were provided in the kit.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

### MAIC

A DNA fragment was prepared coding for the extracellular domain of mouse interleukin-3 receptor. This fragment is hereinafter referred to as the "MAIC DNA fragment". The PCR process was carried out using the LA PCR Kit [Takara Shuzo], under the following conditions.
Composition of the reaction solution:
plasmid pME18S-mFas-AIC DNA, 20 ng;
oligonucleotide primer N3N, 0.5 µg;
oligonucleotide primer CTN2, 0.5 µg;
dNTP mix, 25 µl;
10 x LA PCR buffer, 25 µl;
LA Taq polymerase [Takara Shuzo], 12.5 units.

The final volume of the solution was made up to 250 µl with redistilled water. The dNTP mix, 10 x LA PCR buffer and LA Taq polymerase were provided in the kit.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The HFAS DNA and MAIC DNA fragments amplified after PCR were extracted with phenol. The DNA was then precipitated using 100% ethanol. The DNA fragments (20-30 µg) were electrophoresed on a 5% (w/v) polyacrylamide gel. The gel was stained with 1 µg/ml of ethidium bromide, such that the DNA fragments were visible when viewed under UV light. The DNA fragments detected in this way were excised from the gel with a razor blade and electro-eluted from the acrylamide gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 20 µl of distilled water.

### d) Second step PCR

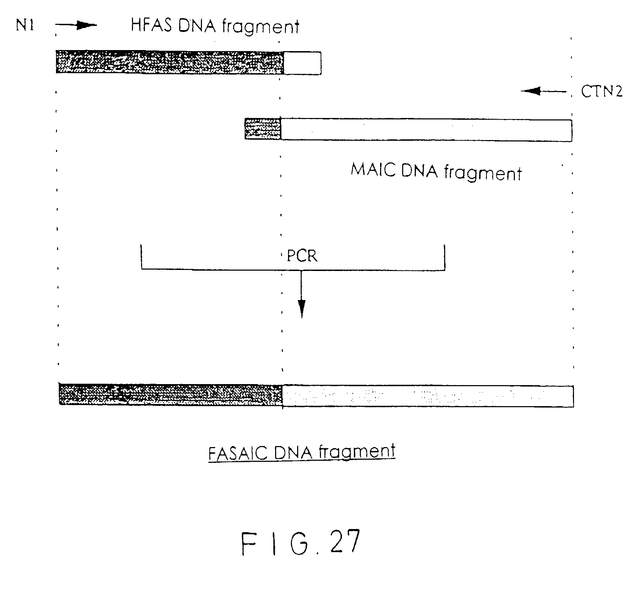

The outline of the second step PCR is shown in Figure 27.

### FASAIC

A DNA fragment was prepared encoding a human Fas fusion protein. The fragment is hereinafter referred to as the "FASAIC DNA fragment". The PCR process was carried out using the LA PCR Kit [Takara Shuzo], under the following conditions.
Composition of the reaction solution:
HFAS DNA solution prepared in the first step PCR, 20 µl;
MAIC DNA solution prepared in the first step PCR, 20 µl;
oligonucleotide primer N1, 0.5 µg;
oligonucleotide primer CTN2, 0.5 µg;
DNTP MIX, 25 µl;
10x LA PCR buffer, 25 µl;
LA Taq polymerase [Takara Shuzo], 12.5 units.

The final volume of the solution was made up to 250 µl with redistilled water. The DNTP MIX, 10 x LA PCR buffer and LA Taq polymerase were provided in the kit.

Specifically, the reaction solution was initially heated at 94 °C for 2 minutes. The sample was then heated using the following thermal cycle: 94 °C for 1 minute, 55 °C for 1 minute and 72 °C for 2 minutes. This cycle was repeated 30 times. Subsequently, the reaction solution was incubated at 72 °C for 10 minutes.

The FASAIC DNA fragment amplified by PCR in this way was extracted with phenol. The DNA was then precipitated using 100% ethanol. The DNA fragment (20-30 µg) was electrophoresed on a 1% (w/v) agarose gel. The gel was stained with 1 µg/ml of ethidium bromide, such that the DNA fragment was visible when viewed under UV light. The DNA fragment detected in this way were excised from the gel with a razor blade and electro-eluted from the agarose gel using a Centriruter [Amicon] equipped with Centricon 10 [Amicon]. The eluted DNA was concentrated by a centrifugation step at 7,500 x g for approximately 1 hour, followed by ethanol precipitation. The final DNA product was dissolved in 50 µl of distilled water.

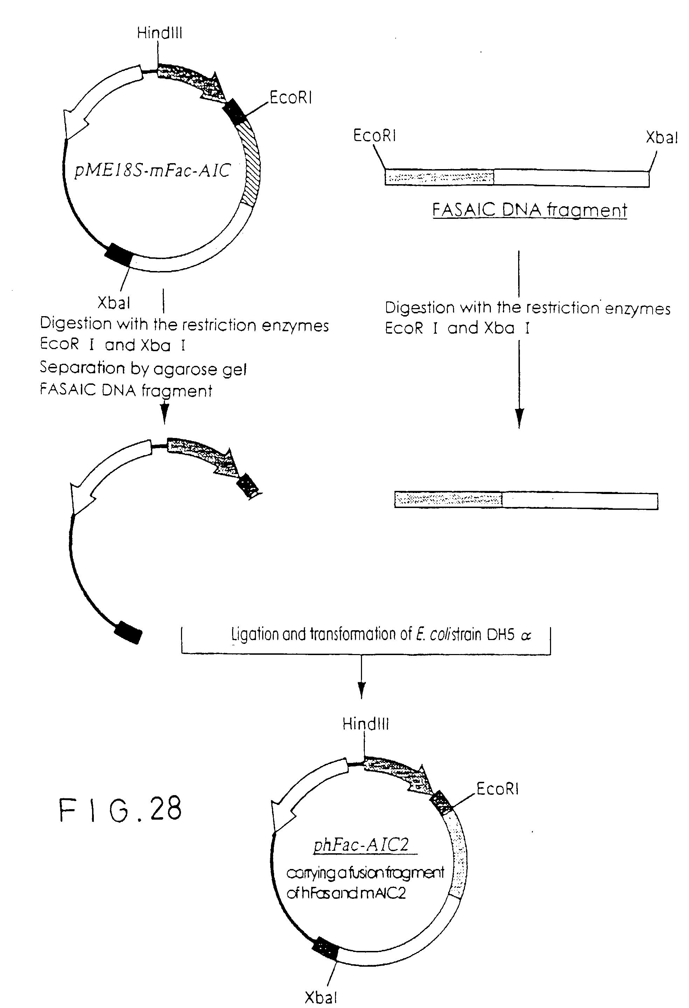

The construction of a plasmid carrying FASAIC DNA fragment is outlined in Figure 28.

The FASAIC DNA obtained in this way was further purified by phenol extraction followed by ethanol precipitation. The DNA was then digested with the restriction enzymes EcoR1 and Xba1.

A portion (2 µg) of plasmid pME18S-mFas-AIC DNA was digested with the restriction enzymes EcoR1 and Xba1. The products of the digestion were separated by electrophoresis on a 0.8 % (w/v) agarose gel. The gel was stained with 1 µg/ml of ethidium bromide, such that the DNA fragments were visible when viewed under UV light. A DNA band of approximately 3,000 bp was excised with a razor blade to recover the DNA.

A portion of the digested pME18S-mFas-AIC DNA obtained above was ligated to a portion of the EcoR1 and Xba1 digested FASAIC DNA. The ligation was carried out using a ligation kit, and the ligation product was transformed into *E. coli* strain DH5α.

Restriction analysis of plasmid DNA contained in transformant colonies was carried out, to identify plasmids containing the DNA insert of interest. Plasmid phFas-AlC2 was identified containing the FASAIC DNA fragment (encoding a human Fas fusion protein) inserted downstream of SRα promoter in the correct orientation for expression of the immunoglobulin polypeptide.

### e) Expression in COS-7 cells

The expression plasmid obtained above for the human Fas fusion protein was transfected into COS-7 cells by electroporation using the gene transfection apparatus ECM600 M (BTX).

COS-7 cells [American Type Culture Collection No. CRL-1651] were cultured in a 225 cm² culturing flask [Sumitomo Bakelite]. The cells were grown to a semi-confluent state in DMEM containing 10 % foetal bovine serum [CSL]. The medium was removed and the COS-7 cells were treated with 3 ml of trypsin-EDTA solution [Sigma Chemicals Co.] at 37°C for 3 minutes. The detached cells were harvested by centrifugation at 800 rpm for 2 minutes and then washed twice with PBS(-) buffer [Nissui Seiyaku]. The washed cells were adjusted to a density of 4x10⁶ cells/ml with PBS(-) buffer to produce a COS-7 cell suspension.

In parallel, 100 µg of phFas-AIC2 plasmid DNA was prepared using a plasmid MaxiPrep kit [MaxiPrep DNA Purification Kit; Promega]. The DNA was precipitated with 100% ethanol, and then suspended in 100 µl of PBS(-) buffer. The plasmid solution (100 µl) was mixed with 500 µl of COS-7 cells, prepared above (equivalent to 2 x 10⁶ cells). The mixture was transferred to an electroporation cuvette having an electrode interval of 4 mM distance [BioRad], and then loaded in an electroporation apparatus Electroporation was then used to introduce the plasmid DNA of interest into the COS-7 cells, using a pulse 150 V-900 µF.

After electroporation, the cell-DNA mixture was resuspended in 20 ml of DMEM containing 10 % foetal bovine serum, then transferred to a 75 cm² culturing flask [Sumitomo Bakelite] The cells were incubated in 7.5 % CO₂ at 37 °C for 24 hours. The culture supernatant was removed and the cells were washed with serum-free DMEM medium. A portion (20 ml) of fresh serum-free DMEM medium was added and the cells were cultured in 7.5 % CO₂ at 37 °C for 24 hours. The supernatant was then recovered

### 2) Assay for the binding ability to Fas antigen by ELISA

The ability of the humanised antibodies to bind the Fas antigen was assayed by the ELISA method, as follows.

The supernatant of the COS-7 cell culture (prepared in section 1, above) was mixed with 50 mM carbonate-bicarbonate buffer (pH 9 5) in the ratio of (1 : 5). A portion of the mixture (50 µl) was added to each well of a a 96-yell EIA plate (3690, bottom area 0.16 cm²; Coster) and incubated at 4 °C overnight, to allow adsorption of the human Fas fusion protein to the surface of the wells. After adsorption, each well was washed with PBS (-) buffer containing 0.05 % Tween 20 (EIA grade; BioRad, hereinafter referred to as "PBS-T").

SuperBlock Blocking Buffer [Pierce, Inc.] was made up in PBS, and 50 µl of this buffer was added to each well. The plate was incubated at room temperature for 2 hours in order to effect blocking. The wells were washed again with PBS-T.

A 50 µl sample of each of the diluted culture supernatants prepared in Working Example 7 was added to each well and incubated at 37 °C for 2 hours. The wells were then washed with PBS-T. A portion (50 µl) of peroxidase-labelled goat anti-human IgM monoclonal antibody [Jackson Immuno-research Laboratory], diluted at 1 : 10,000 in PBS, was dispensed into each well and the plate incubated at 37 °C for 2 hours. After washing with PBS-T, 50 µl of substrate solution [Peroxidase Substrate Set - ABTS; BioRad] was dispensed into each well, to initiate a colourimetric assay.

The ability of the humanised antibodies contained in the culture supernatants to bind to the human Fas antigen fusion protein was evaluated by reading the absorbance of each well at 405 nm and 492 nm with a microplate reader [Model 3550UV; BioRad]. The ratio of the absorbance at 405 nm and that at 492 nm allows the binding of IgM to the immobilised to be calculated.

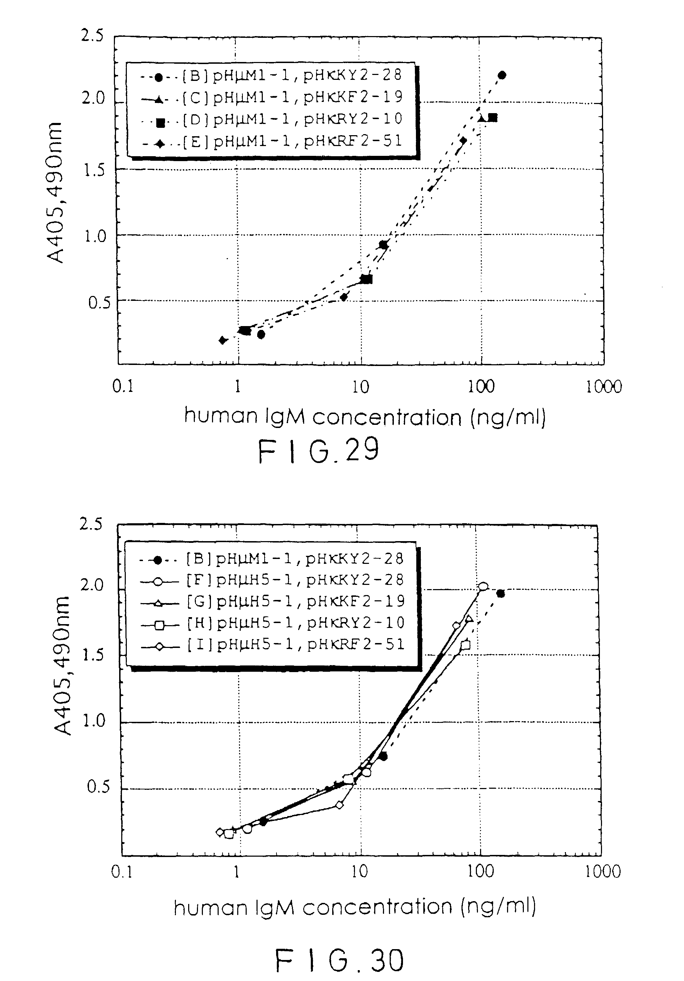

The results of the assay indicate that the humanised antibodies produced in samples (B), (C), (D), (E). (F), (G), (H) and (I) of Working Example 7 were capable of binding to the human Fas antigen fusion protein (Figures 29 and 30).

### TEST EXAMPLE 3

### Assay for apoptosis-inducing activity

The culture supernatant samples prepared in Working Example 7 above were incubated with the human lymphocyte cell line 'HPB-ALL', in order to determine the cytotoxic activity of the humanised antibodies contained in the supernatants.

HPB-ALL cells were grown in RPMI 1640 medium [Nissui Seiyaku] containing 20mM HEPES, 50 µM β-mercaptoethanol, 0.33% sodium bicarbonate [Sigma] and 10% foetal bovine serum [CSL], (hereinafter referred to as 'RPMI medium') in 5% CO₂ at 37°C. HBP-ALL cells were harvested at logarithmic phase by centrifugation, at 800 RPM for 3 minutes. The cell pellet was resuspended in RPMI medium at a density of 6 x 10⁵ cells, ml, producing a HBP-ALL cell suspension.

Each of the culture supernatants prepared in Working Example 7, along with the mouse anti-human Fas antibody CH11 were diluted to the following concentrations: 250, 100, 25, 10, 2.5, 1, 0.25 and 0.1 ng/ml. A portion (50 µl) of each dilution was mixed with 50 µl of the HBP-ALL cell suspension (at 3 x 10⁴ cells/50 µl) in each well of a 96-well culture plate. The plate was incubated in 5% CO₂ at 37°C for 2 hours. The absorbance at 450nm and 750 nm was measured, using a microplate reader Model 3550-UV [Bio-Rad Co]. The concentration of each sample was measured by the densitometric analysis of Western Blots, prepared as described in Test Example 1.

The % survival of HBP-ALL cells was calculated using the following formula:
Survival Rate (%) = (A-C)/(B-C)x 100
Where:
A = Number of cells remaining after incubation of CH11 or humanised antibody with HBP-ALL cells.
B = Number of cells remaining after culture of HBP-ALL cells alone (no CH11 or humanised antibody).
C = RPMI medium alone, without HBP-ALL cells (incubated for 20 hours, as A and B above).

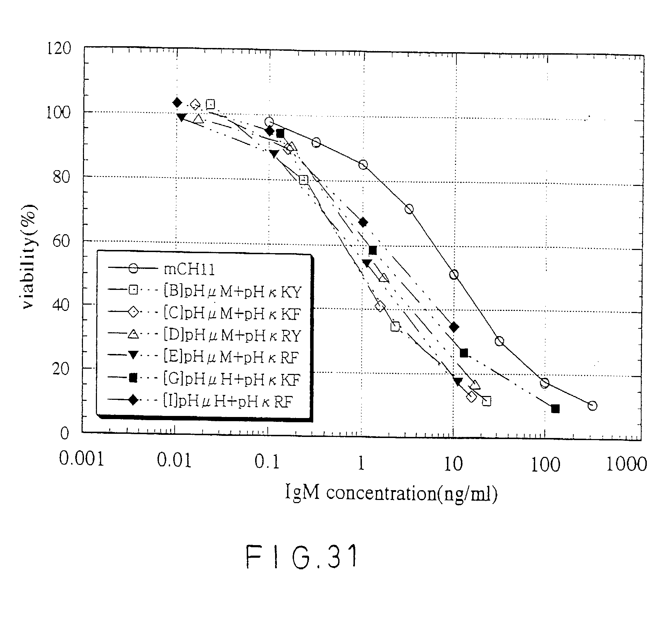

The results are presented graphically in Figure 31. The ED₅₀ value, an index of the cytotoxic activity, was calculated in each case. ED₅₀ represents the concentration of the IgM for which 50% of the cells survive.

The results are as follows:

| **Sample** | **ED**_{**50**} **(ng/ml)** |
|---|---|
| B | 1.1 |
| C | 1.0 |
| D | 1.7 |
| E | 1.5 |
| G | 2.4 |
| I | 3.4 |
| CH11 | 10.7 |

The results indicate that the recombinant anti-Fas IgM molecules that lack the J chain have 3 to 10 times higher cytotoxic activity than CH11, which possesses the J chain.

## Claims

1. A method for the production of a humanised antibody and derivatives thereof, comprising at least one light chain and one heavy chain, the method comprising the steps of:
a selecting a non-human antibody having at least one CDR;
b selecting a human antibody heavy chain;
c selecting a human antibody light chain;
d introducing at least one CDR, or fragment thereof, from the non-human antibody heavy chain into the human antibody heavy chain, to form a recombinant heavy chain; and
e introducing at least one CDR, or fragment thereof, from the non-human antibody light chain into the human antibody light chain, to form a recombinant light chain;
characterised in that the selection of each of the human antibody heavy and light chains is determined solely by sequence homology with the non-human antibody heavy and light chains, respectively.

2. A method according to claim 1, wherein the CDR regions have been removed from the human antibody chain before the introduction of the at least one CDR, or fragment thereof, from the non-human antibody chain.

3. A method according to claims 1 or 2, wherein the sequence homology is amino acid sequence homology.

4. A method according to any preceding claim, wherein the sequence homology is assessed substantially only in relation to the framework regions.

5. A method according to any preceding claim, wherein the selection of each human antibody chain is determined by sharing at least 70% amino acid identity in the framework regions with the non-human antibody chain.

6. A method according to any preceding claim, wherein all of the CDRs from each non-human antibody chain are introduced into the relevant human antibody chain.

7. A method according to any preceding claim, wherein the selected non-human antibody is the mouse CH11 antibody.

8. A method according to any preceding claim, wherein the selected human light chain is from the human antibody RPMI6410'CL.

9. A method according to any preceding claim, wherein the human heavy chain is from the human antibody 21•28'CL.

10. A method according to any preceding claim, wherein the amino acid regions derived from the human antibody comprise at least most of each of the framework regions of the antibody.

11. A method according to claim 11, wherein the amino acid regions derived from the human antibody further comprise the constant region, or a portion of the constant region.

12. A method according to any preceding claim, wherein the at least one non-human CDR is introduced into the human antibody along with at least one significant amino acid residue of at least one of the framework regions of the non-human CDR.

13. A method according to claim 12, wherein the at least one significant amino acid residue is introduced from a non-human framework region along with the CDR, if the residue meets at least one of the following criteria:
a) the amino acid in the human framework region of the acceptor is rarely found at that position in the acceptor, whereas the corresponding amino acid in the donor is commonly found at that position in the acceptor;
b) in a three-dimensional model of the immunoglobulin, the amino acid has a side-chain atom which is judged to form a bond with an antigen or a CDR of a humanised antibody, in accordance with one or more of the criteria i), ii) and iii);
i) the side chain atom is within a distance of a second atom of less than the sum of their Van der Waal's radii plus 0.5 Å,
ii) the side chain atom is polar and is less than 3.4 Å from a second polar atom, and
iii) the side chain atom is charged and is less than 3.35 Å from an oppositely charged atom;
c) the amino acid is found in a position which is involved in determining the structure of the canonical class of the CDR; and
d) the position of the amino acid is found at a putative contact surface of the heavy and light chains.

14. A method according to claim 13, wherein the positions of amino acids in criteria (b) and (d) are determined by molecular modelling.

15. A method according to claim 14, wherein the positions of amino acids are additionally determined by comparison with X-ray crystallographic data for other antibodies.

16. A method according to claim 15, wherein an amino acid from the framework region is introduced if it is predicted both to contact a CDR by molecular modelling and is frequently found experimentally to contact a CDR by X-ray crystallography.

17. An antibody produced by the method of any of claims 1 to 16.

18. An antibody according to claim 17, wherein the antibody has anti-Fas activity.

19. An antibody according to claim 18, wherein the molecule is an IgM molecule with anti-Fas activity.

20. An antibody according to any of claims 17 to 19, wherein the antibody is an IgM antibody lacking a J chain.

21. An antibody according to any of claims 17 to 20, wherein the light chain comprises the amino acid sequence as defined by SEQ ID No. 78 and the heavy chain comprises the amino acid sequence as defined by SEQ ID No. 86.

22. An antibody according to any of claims 17 to 20, wherein the light chain comprises the amino acid sequence as defined by SEQ ID No. 78 and the heavy chain comprises the amino acid sequence as defined by SEQ ID No. 88.

23. An antibody according to any of claims 17 to 20, wherein the light chain comprises the amino acid sequence as defined by SEQ ID No. 80 and the heavy chain comprises the amino acid sequence as defined by SEQ ID No. 86.

24. An antibody according to any of claims 17 to 20, wherein the light chain comprises the amino acid sequence as defined by SEQ ID No. 80 and the heavy chain comprises the amino acid sequence as defined by SEQ ID No. 88.

25. An antibody according to any of claims 17 to 20, wherein the light chain comprises the amino acid sequence as defined by SEQ ID No. 82 and the heavy chain comprises the amino acid sequence as defined by SEQ ID No. 86.

26. An antibody according to any of claims 17 to 20, wherein the light chain comprises the amino acid sequence as defined by SEQ ID No. 82 and the heavy chain comprises the amino acid sequence as defined by SEQ ID No. 88.

27. An antibody according to any of claims 17 to 20, wherein the light chain comprises the amino acid sequence as defined by SEQ ID No. 84 and the heavy chain comprises the amino acid sequence as defined by Seq ID No. 86.

28. An antibody according to any of claims 17 to 20, wherein the light chain comprises the amino acid sequence as defined by SEQ ID No. 84 and the heavy chain comprises the amino acid sequence as defined by SEQ ID No. 88.

29. RNA encoding an antibody according to any of claims 17 to 28.

30. DNA encoding an antibody according to any of claims 17 to 28.

31. DNA which hybridises with the DNA of claim 30, preferably under conditions of 60 - 70 °C and in 6 x SSC.

32. A vector comprising DNA according to claims 30 or 31.

33. A vector according to claim 33 which is an expression vector.

34. A vector selected from recombinant DNA vectors pHκKY2-58, pHκKF2-19, pHκRY2-10, pHκRF2-52, pHµH5-1 and pHµm1-1.

35. A host cell transformed with an expression vector according to any of claims 32 to 34.

36. *E. coli* pHκKY2-58 (FERM BP-5861).

37. *E. coli* pHκKF2-19 (FERM BP-5860).

38. *E*. *coli* pHκRY2-10 (FERM BP-5859).

39. *E. coli* pHκRF2-52 (FERM BP-5862).

40. *E. coli* pHµH5-1 (FERM BP-5863).

41. *E. coli* pHµm1-1 (FERM BP-5864).

42. A method for producing an immunoglobulin protein of the present invention comprising culturing a cell according to any of claims 32 to 34 under conditions which enable expression of DNA encoding the immunoglobulin H chain or L chain subunit contained in the vector, and recovering the immunoglobulin protein from the culture.

43. Use of an antibody according to any of claims 17 to 28 in the preparation of a medicament for the treatment of an autoimmune disease.

44. Use according to claim 43, wherein the autoimmune disease is rheumatism.
